# EUROPEAN PATENT SPECIFICATION

(11) **EP 4 491 695 B1**
(45) Date of publication and mention of the grant of the patent: **02.09.2026**
(21) Application number: 24187285.2
(22) Date of filing: 09.07.2024
(51) Int. Cl.: C09K 11/06, C07D 251/24, C07D 401/14, C07D 403/10, C07D 403/12, C07D 403/14, C07D 405/14, C07F 9/6558, H10K 85/60, C07D 409/12, C07F 7/08, C07D 491/048, C07D 471/14, C07D 495/04, H10K 50/11, H10K 85/40, H10K 85/30

(54) **ORTHO-SUBSTITUTED THERMALLY ACTIVATED DELAYED FLUORESCENCE MATERIAL AND ORGANIC LIGHT-EMITTING DEVICE COMPRISING SAME**
ORTHO-SUBSTITUIERTES THERMISCH AKTIVIERTES VERZÖGERTES FLUORESZENZMATERIAL UND ORGANISCHE LICHTEMITTIERENDE VORRICHTUNG DAMIT
MATÉRIAU À FLUORESCENCE RETARDÉE ACTIVÉ THERMIQUEMENT ORTHO-SUBSTITUÉ ET DISPOSITIF ÉLECTROLUMINESCENT ORGANIQUE LE COMPRENANT

(30) Priority: 10.07.2023 US 202318349892
(43) Date of publication of application: 15.01.2025
(60) Divisional application: 26190601.0 / 4 796 546
(73) Proprietor: Samsung Display Co., Ltd., Gyeonggi-do 17113 (KR)
(72) Inventor: Ahn, Heechoon, 17113 Yongin-si, Gyeonggi-do (KR); Gong, Myoungseon, 06337 Gangnam-gu, Seoul (KR); Lee, Chilwon, 16889 Suji-gu, Yongin-si (KR); Cha, Jaeryung, 31117 Dongnam-gu, Cheonan-si (KR); Park, Youngjin, 17113 Yongin-si, Gyeonggi-do (KR); Um, Hyunah, 17113 Yongin-si, Gyeonggi-do (KR); Lee, Hyoyoung, 17113 Yongin-si, Gyeonggi-do (KR)
(74) Representative: Dr. Weitzel & Partner

(56) References cited:
- WO-A1-2023/090288
- WO-A1-2023/277645
- US-A1- 2022 181 561

## Description

The present invention relates to compounds for an organic light emitting diode and more specifically to thermally activated delayed fluorescence materials as the compounds for the organic light emitting diode.

### Background Art

OLED (organic light emitting device) is a self-emitting device, and has advantages of fast response time, excellent or suitable brightness, driving voltage and response rate characteristics, and implementing multi-color, as well as wide viewing angle and excellent or suitable contrast.

General organic light emitting diode may include an anode, a cathode, and an organic layer interposed between the anode and the cathode. The organic layer may include a hole injection layer, a hole transport layer, a light emitting layer, an electron transport layer, and an electron injection layer and/or the like. When a voltage is applied between the anode and the cathode, holes injected from the anode are moved to the light emitting layer via the hole transport layer, and electrons injected from the cathode moves to the light emitting layer via the electron transport layer. Carriers, such as the holes and electrons, recombine in the light emitting layer to generate excitons, and the excitons transition to a ground state to emit light.

The excitons generated when the organic light emitting diode is driven are stochastically in a singlet state of 25% and a triplet state of 75%. In the case of a fluorescent light emitting material, only excitons in the singlet state which is 25% of total excitons participate in luminescence, and the internal quantum efficiency remains at maximum 25%. In order to improve these properties, iridium or platinum complexes facilitating emitting from the triplet excitons can be utilized, and thereby allowing excellent or suitable quantum efficiency characteristics. However, these materials are expensive, and their applications are limited due to the instability of the blue light emitting material.

In order to solve this problem, recently, a thermally activated delayed fluorescent organic material are being developed. The thermally activated delayed fluorescent organic material has an energy difference of 0.3 eV or less between the singlet state and the triplet state of excitons. In this case, the up-conversion from the triplet state into the singlet state is allowed by heat corresponding to room temperature or device driving temperature, and the theoretical quantum efficiency of nearly 100% can be achieved.

US 2022/181561 A1 is directed to a high performance OLED which includes an anode; a cathode; and an emissive layer, disposed between the anode and the cathode, wherein the emissive layer includes a first phosphorescent emitter and a first host; the first phosphorescent emitter is a metal complex; the first host is partially or fully deuterated. For example, a general formula of the first host is disclosed as follows 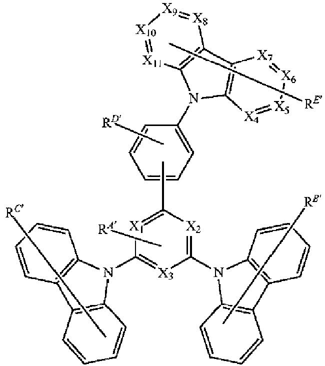 wherein X₁ to X₁₁ and R^{A'} to R^{E'} are defined as disclosed in US 2022/181561 A1.

WO 2023/277645 A1 describes an organic molecule for the application in optoelectronic devices, the organic molecule has a first chemical moiety comprising a structure of Formula I and one or two second chemical moieties, comprising a structure of Formula II, wherein the first chemical moiety is linked to the second chemical moiety via a single bond; W is the binding site of a single bond linking the first chemical moiety to the second chemical moiety, Q is W or is R¹, # represents the binding site of the first chemical moiety to the second chemical moiety: 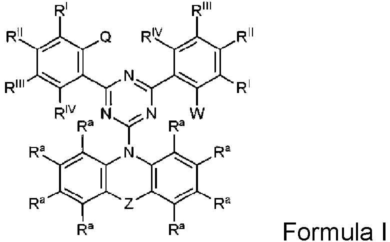 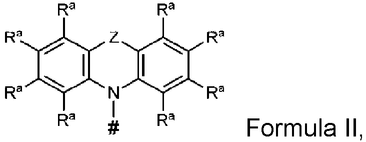 wherein R^{I} to R^{IV}, R^{a}, Z are defined as disclosed in WO 2023/277645 A1.

WO 2023/090288 A1 describes an organic light-emitting element in which a compound represented by the following general formula is used: 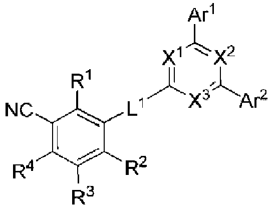 wherein X¹ to X³, R¹ to R⁴, Ar¹, Ar², and L¹ are defined as disclosed in WO 2023/090288 A1.

### Technical Problem

However, it is known that the actual quantum efficiency of currently developed thermally activated delayed fluorescent organic materials is very different from the theoretical quantum efficiency and still needs to be improved.

### Technical Solution

It is an object of the present invention to provide a thermally activated delayed fluorescent material and an organic light emitting diode including the same, which can improve quantum efficiency as the transition from a triplet state to a single state can be more efficiently performed.

In some example embodiments, a thermally activated delayed fluorescent (TADF) material is provided. The TADF material has a form in which an electron donating group and an electron withdrawing group are connected to benzene and the electron withdrawing group is position in an ortho position to the electron donating group.

The subject matter of the present invention is defined in independent claim 1 referring to a light-emitting material, in independent claim 10 defining a light-emitting device and in independent claim 15 referring to an electronic apparatus or equipment. Preferred embodiments are defined in the sub-claims.

The electronic apparatus may further comprise a thin-film transistor electrically connected to the light-emitting device; and a color filter, a color conversion layer, a touch screen layer, a polarizing layer, or any combination thereof.

The electronic equipment may comprise the light-emitting device according to the present invention, and may be at least one of a flat panel display, a curved display, a computer monitor, a medical monitor, a television, a billboard, an indoor or outdoor light and/or light for signal, a head-up display, a fully or partially transparent display, a flexible display, a rollable display, a foldable display, a stretchable display, a laser printer, a telephone, a portable phone, a tablet personal computer, a phablet, a personal digital assistant (PDA), a wearable device, a laptop computer, a digital camera, a camcorder, a viewfinder, a micro display, a three-dimensional (3D) display, a virtual reality or augmented reality display, a vehicle, a video wall with multiple displays tiled together, a theater or stadium screen, a phototherapy device, or a signboard.

### Advantageous Effects

According to the present invention, as the compound introduced with the electron donating group and the electron withdrawing group at the ortho positions of the benzene ring can have reduced energy difference between the singlet and triplet excited states, up-conversion of the triplet excited states to the singlet excited states through reverse intersystem crossing by heat at room temperature or device operating temperature can easily occurs, which may result in delayed fluorescence.

### Description of Drawings

Example embodiments of the present invention will become more apparent by describing in more detail example embodiments of the present invention with reference to the accompanying drawings, in which:
FIG 1 is a cross-sectional view illustrating an organic light emitting diode according to an example embodiment of the present invention;
FIG 2 shows distribution of molecular orbital of Compound 1 prepared according to Synthesis Example 1, Comparative Compound 2, and Comparative Compound 3;
FIG. 3 is a schematic cross-sectional view of a light-emitting device according to an embodiment;
FIGS. 4 and 5 are cross-sectional views of an electronic device according to an embodiment;
FIG. 6 is a schematic perspective view of an electronic apparatus including a light-emitting device according to an embodiment;
FIG. 7 is a schematic diagram illustrating an exterior of a vehicle as an electronic apparatus including a light-emitting device according to an embodiment; and
FIGS. 8A, 8B, and 8C are schematic diagrams illustrating an interior of a vehicle according to an embodiment.

### Modes of the Invention

Hereinafter, the present invention will be described in further detail with reference to several aspects and one or more suitable example embodiments. Hereinafter, example embodiments of the present invention will be described in more detail with reference to the accompanying drawings. The embodiments to be described may be modified in several different forms, and the scope of the present invention is not limited to the embodiments. Rather, the embodiments are provided to further faithfully and fully explain this invention and to fully convey the scope of the present invention to those skilled in the art. Like reference numerals in the drawings denote like elements.

As utilized herein, the term "alkyl group" refers to an aliphatic hydrocarbon group, unless otherwise defined. The alkyl group may be a saturated alkyl group which does not contain any double or triple bonds. Or the alkyl group may be an unsaturated alkyl group including at least one double bond or triple bond. The alkyl group, whether saturated or unsaturated, may be branched, straight chain or cyclic. The alkyl group may be a C1 to C30 alkyl group. More specifically, the alkyl group may be a C1 to C10 alkyl group or a C1 to C6 alkyl group. For example, the C1 to C4 alkyl groups may be selected from the group consisting of methyl, ethyl, propyl, isopropyl, n-butyl, iso-butyl, sec-butyl and t-butyl.

As utilized herein, the term "aryl group" refers to a monocyclic aromatic compound or a polycyclic aromatic compound composed of fused aromatic rings unless otherwise defined, and includes a heteroaryl group.

As utilized herein, "heteroaryl group", unless otherwise defined, is a monocyclic aromatic compound or a polycyclic aromatic compound composed of fused aromatic rings, which contains at least one heteroatom selected from the group consisting of N, O, S, Se, and P in at least one ring.

As utilized herein, the term "halogen group" refers to a group 7 element, for example, F, Cl, Br, or I, unless otherwise defined. As an example, the halogen group may be F.

When "Cx-Cy" is described in the present specification, the number of carbon atoms corresponding to all the integers between x and y is also to be interpreted as described.

### Thermally Activated Delayed Fluorescent (TADF) material

The following Chemical Formula 2 represents the compound according to the present invention.

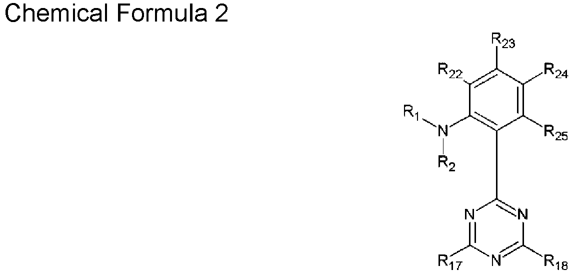

In Chemical Formula 2, R₁ and R₂ are, independently of each other, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, or a substituted or unsubstituted C3-C30 heteroaryl group; R₁ and R₂ are optionally combined together with the nitrogen to which they are attached to form a substituted or unsubstituted heterocyclyl or a substituted or unsubstituted heteroaryl group.

R₂₂ to R₂₅ are, independently of each other, hydrogen, deuterium, a halogen group, or a substituted or unsubstituted C4-C6 aryl group. For example, in Chemical Formula 2, R₂₂ to R₂₅ may be, independently of each other, hydrogen or deuterium.

In Chemical Formula 2, at least one selected from R₁, R₂, R₁₇ and R₁₈ comprises a deuterium, or at least one selected from R₂₂ to R₂₅ is a deuterium.

In Chemical Formula 2, R₁ and R₂ may be combined together with the nitrogen to which they are attached, to form a substituted or unsubstituted heterocyclyl or a substituted or unsubstituted heteroaryl group represented by any one of Structural Formulas A1 to A6 and A9 to A19: 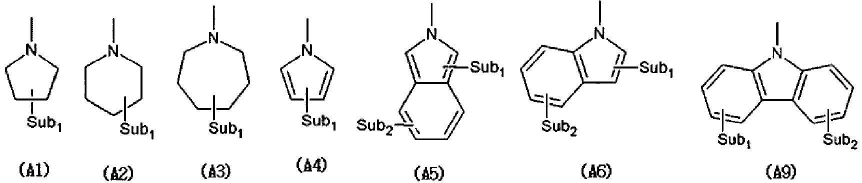 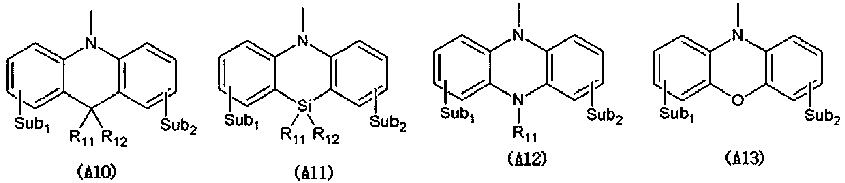 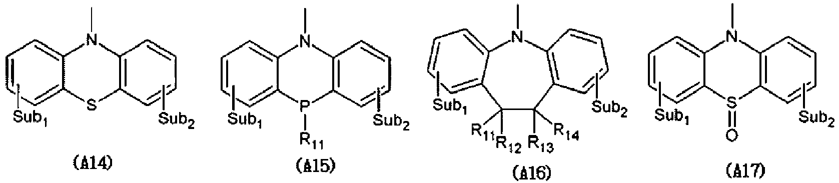 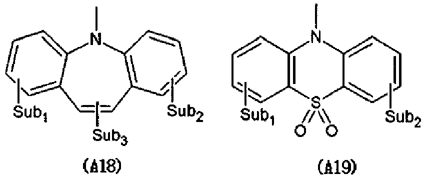
wherein in Structural Formulas A1 to A6 and A9 to A19,
R₁₁ to R₁₄ may be, independently of each other, hydrogen, deuterium, a substituted or unsubstituted C1-C2 alkyl group, or a substituted or unsubstituted C6-C30 aryl group,

Sub₁ and Sub₂ may be, independently of each other, hydrogen, deuterium, a halogen group, a cyano group, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C3-C30 heteroaryl group, a substituted or unsubstituted C1-C9 alkyloxy group, a substituted or unsubstituted -NR_{c}R_{d}, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylsilyl group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group. For example, in Chemical Formula 2, Sub₁ and Sub₂ may be, independently of each other, hydrogen, deuterium, a halogen group, or a cyano group. Also, Sub₁ and Sub₂ may exist, independently of each other, in multiple numbers. For example, Sub₁ and Sub₂ may exist, independently of each other, in the number of 2, 3, 4, or 5. R_{c} and R_{d} may be, independently of each other, selected from the group consisting of a substituted or unsubstituted C1-C9 alkyl group, a substituted or unsubstituted C5-C30 cycloalkyl group, a substituted or unsubstituted C6-C30 aryl group, a substituted or unsubstituted C6-C30 aralkyl group, or a substituted or unsubstituted C3-C30 heteroaryl group. R_{c} and R_{d} may optionally be joined together with the nitrogen to which they are attached to form a substituted or unsubstituted heterocyclyl or a substituted or unsubstituted heteroaryl. Also, Sub₁ and Sub₂ may be, regardless of each other, optionally fused to a main body to which they are bonded to form a substituted or unsubstituted cyclic or a substituted or unsubstituted aryl. In particular, when at least one of Sub₁ and Sub₂ is a substituted or unsubstituted C5-C30 arylsilyl, a substituted or unsubstituted C5-C30 arylthio, a substituted or unsubstituted C5-C30 aryloxy, a substituted or unsubstituted C5-C30 arylamine group, or a substituted or unsubstituted C5-C30 aralkyl group, the aryl group contained therein may be fused to the main body to form a substituted or unsubstituted heterocyclyl or a substituted or unsubstituted heteroaryl.
In Chemical Formula 2 R₁ and R₂ are combined together with the nitrogen to which they are attached to form a substituted or unsubstituted heterocyclyl or a substituted or unsubstituted heteroaryl group represented by any one of Structural Formulas A1 to A6 or A9 to A45: 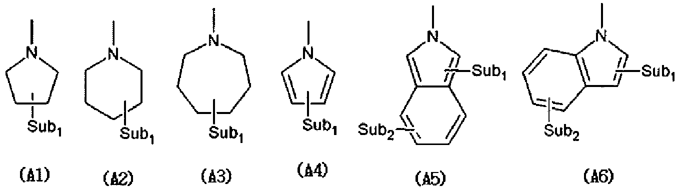 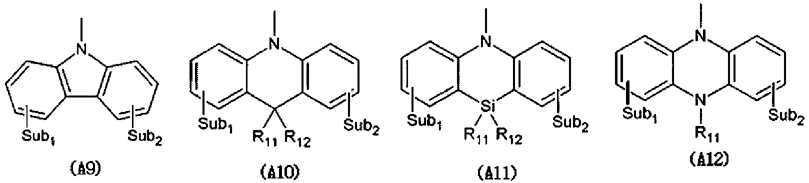 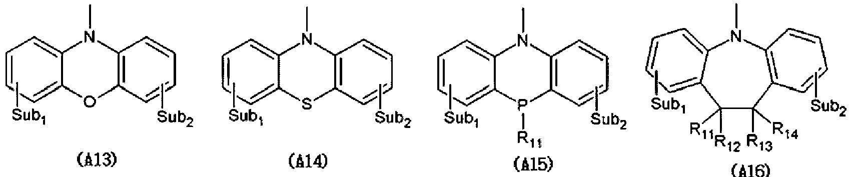 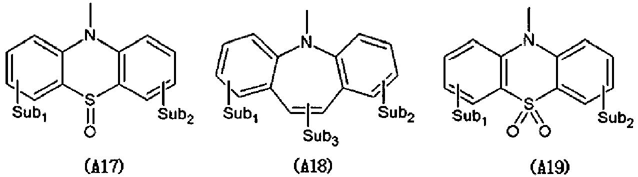 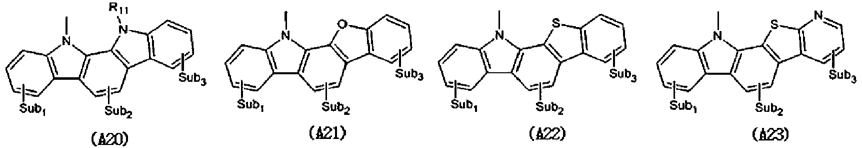 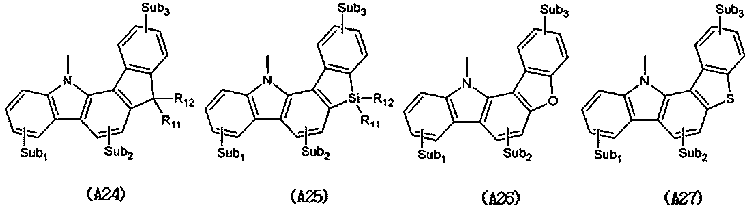 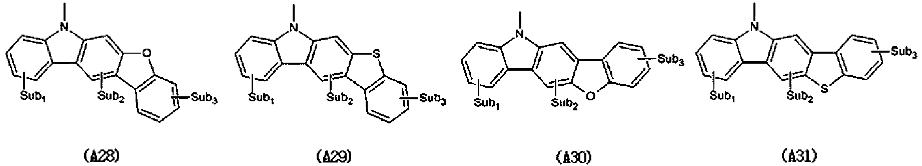 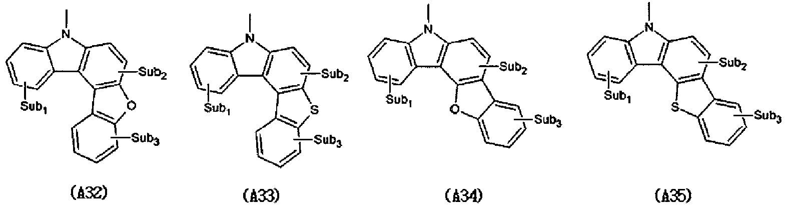 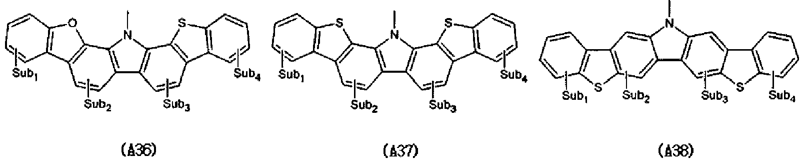 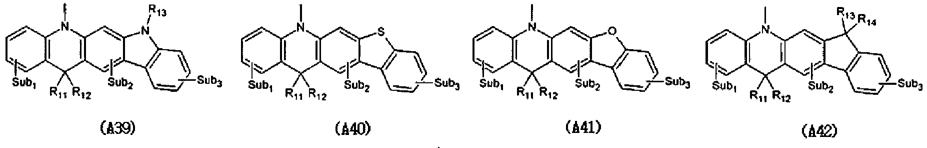 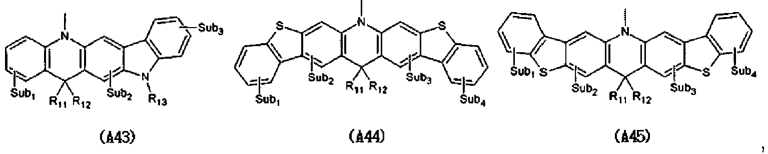 or, in Chemical Formula 2, R₁ and R₂ are selected to be represented by any one of Structural Formulas A7 or A8: 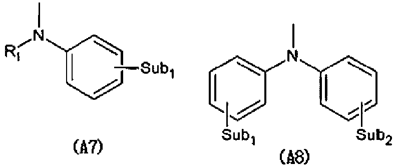
wherein in Structural Formulas A1 to A45,
R₁₁ to R₁₄ may be, independently of each other, hydrogen, deuterium, a substituted or unsubstituted C1-C2 alkyl group, or a substituted or unsubstituted C6-C30 aryl group, and
Sub₁ to Sub₄ may be, independently of each other, hydrogen, deuterium, a halogen group, a cyano group, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, a substituted or unsubstituted C3-C30 heteroaryl group, a substituted or unsubstituted C1-C9 alkyloxy group, a substituted or unsubstituted C6-C30 aryloxy group, a substituted or unsubstituted silyl group, a substituted or unsubstituted phosphine group, a substituted or unsubstituted phosphine oxide group, a substituted or unsubstituted thiol group, a substituted or unsubstituted sulfoxide group, or a substituted or unsubstituted sulfone group. For example, in Chemical Formula 2, Sub₁ to Sub₄ may be, independently of each other, hydrogen, deuterium, a halogen group, or a cyano group. Also, Sub₁ to Sub₄ may exist, independently of each other, in multiple numbers. For example, Sub₁ to Sub₄ may exist, independently of each other, in the number of 2, 3, or 4.

In Chemical Formula 2 the triazine group substituted with R₁₇ and R₁₈ is represented by any one of the Structural Formulas B1 to B13, B50 and B51.

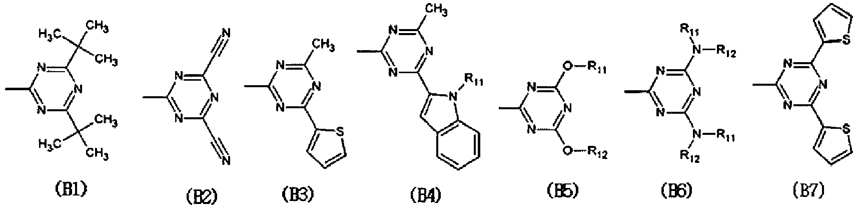

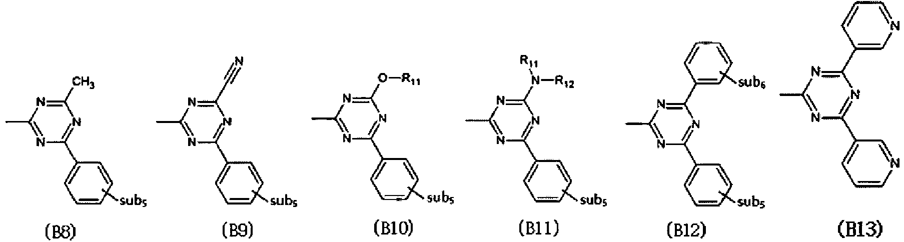

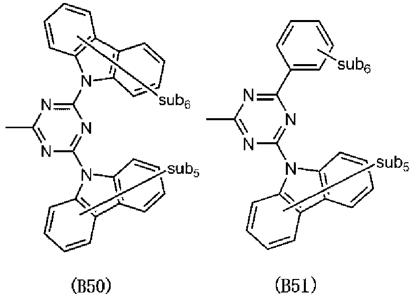

In Structural Formulas B1 to B13, B50 and B51,
R₁₁ and R₁₂ are, independently of each other, hydrogen, deuterium, or a substituted or unsubstituted C1-C2 alkyl group,
Sub₅ and Sub₆ are, independently of each other, hydrogen, deuterium, a halogen group, a cyano group, a substituted or unsubstituted C1-C9 alkyl group, a substituted or unsubstituted C6-C30 aryl group, a substituted or unsubstituted C3-C30 heteroaryl group, a substituted or unsubstituted C1-C9 alkyloxy group, a substituted or unsubstituted -NRₑR_{f}, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group. Also, Sub₅ and Sub₆ may exist, independently of each other, in multiple numbers. For example, Sub₅ and Sub₆ may exist, independently of each other, in the number of 2, 3, 4, 5, 6, 7, or 8. Also, Sub₅ and Sub₆ are, regardless of each other, optionally fused to a main body to which they are bonded to form a substituted or unsubstituted cyclic or a substituted or unsubstituted aryl. In particular, when Sub₅ or Sub₆ is a substituted or unsubstituted C5-C30 arylthio, a substituted or unsubstituted C5-C30 aryloxy, a substituted or unsubstituted C5-C30 arylamine group, or a substituted or unsubstituted C5-C30 aralkyl group, the aryl group contained therein may be fused to the main body to form a substituted or unsubstituted heterocyclyl or a substituted or unsubstituted heteroaryl.

Rₑ and R_{f} are each independently selected from the group consisting of a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, or a substituted or unsubstituted C3-C30 heteroaryl group. Rₑ and R_{f} may optionally be joined together with the nitrogen to which they are attached to form a substituted or unsubstituted heterocyclyl or a substituted or unsubstituted heteroaryl.

In Structural Formula B50, Sub₅ is a cyano group, whereby Sub₅ may also exist, independently of each other, in multiple numbers, so that other Sub₅ may be as defined above,
wherein, in Structural Formula B51, R₂₂ to R₂₅ are, independently of each other, deuterium, a halogen group, or a substituted or unsubstituted C4-C6 aryl group, and wherein at least one selected from R₁, R₂ comprises a deuterium, or at least one selected from R₂₂ to R₂₅ is a deuterium.

The electron withdrawing group in the above Chemical Formula 2 may be the same as the following Structural Formulas B8 to B12, B50 and B51. 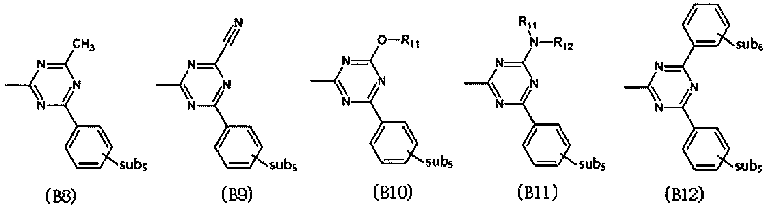 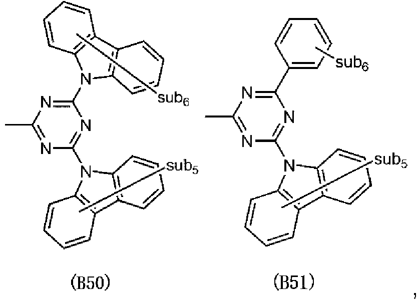
wherein in Structural Formulas B8 to B12, B50 and B51,
   R₁₁ and R₁₂ may be, independently of each other, hydrogen, deuterium, or a substituted or unsubstituted C1-C2 alkyl group,
Sub₅ and Sub₆ are, independently of each other, hydrogen, deuterium, a halogen group, a cyano group, a substituted or unsubstituted C1-C9 alkyl group, a substituted or unsubstituted C6-C30 aryl group, a substituted or unsubstituted C3-C30 heteroaryl group, a substituted or unsubstituted C1-C9 alkyloxy group, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group; and Sub₅ and Sub₆ are, independently of each other, optionally fused to a main body to which they are bonded to form a substituted or unsubstituted cyclic or a substituted or unsubstituted aryl, and
   wherein, in Structural Formula B50, Sub₅ is a cyano group, whereby Sub₅ may also exist, independently of each other, in multiple numbers, so that other Sub₅ may be as defined above.

The triazine group substituted with R₁₇ and R₁₈ in the above Chemical Formula 2 may be the same as the following Structural Formulas B12, and B50 to B54. 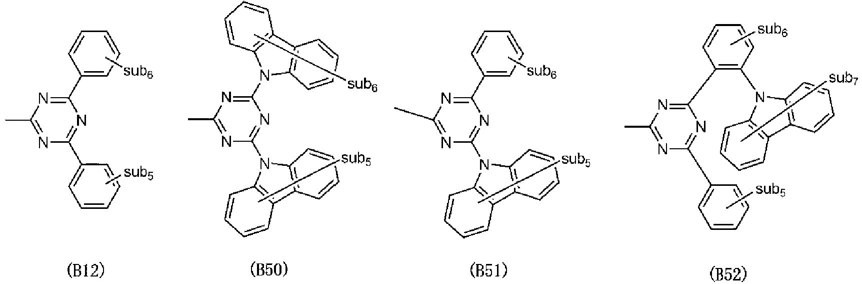 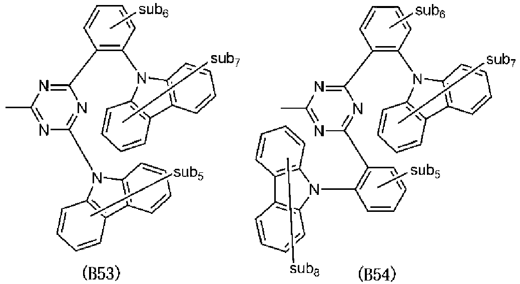 in Structural Formulas B12, and B50 to B54,
Sub₅ to Sub₈ may be, independently of each other, hydrogen, deuterium, a halogen group, a cyano group, a substituted or unsubstituted C6-C30 aryl group, or a substituted or unsubstituted C3-C30 heteroaryl group and
wherein, in Structural Formula B50, Sub₅ is a cyano group, whereby Sub₅ may also exist, independently of each other, in multiple numbers, so that other Sub₅ may be as defined above. For example, Sub₇ and Sub₈ may be, independently of each other, hydrogen, deuterium, a halogen group, or a cyano group.

The thermally activated delayed fluorescent material can implement red, green, and blue luminescent colors by appropriate or suitable introduction of the electron donating group and the electron withdrawing group and reduce the difference between the singlet energy and the triplet energy to 0.3 eV or less.

Specific compounds of such thermally activated delayed fluorescent materials are represented by the following Compounds 100, 101, 104 to 106, 108, 109, 112, 113 and 120 to 122, but are not limited thereto.

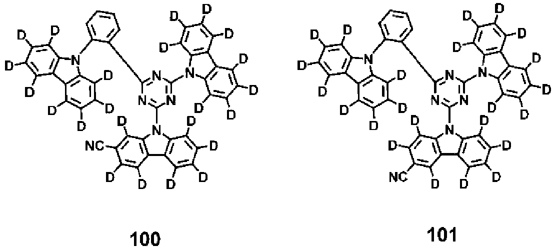

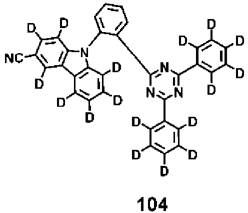

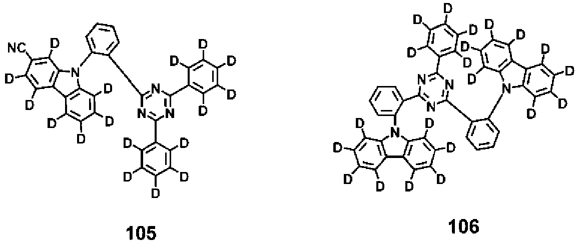

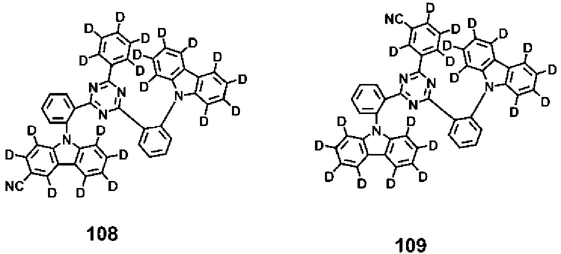

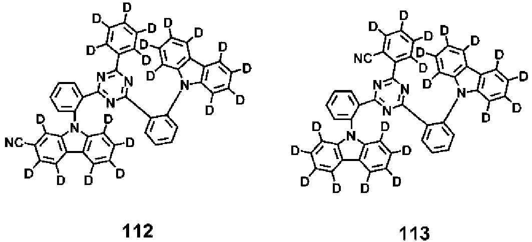

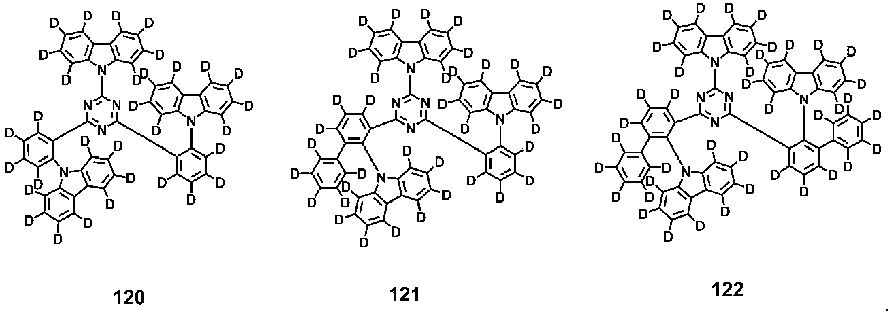

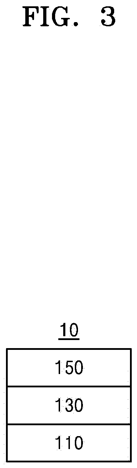

### Description of FIG. 3

FIG. 3 is a schematic cross-sectional view of a light-emitting device 10 according to an embodiment. The light-emitting device 10 may include a first electrode 110, an interlayer 130, and a second electrode 150.

Hereinafter, a structure of the light-emitting device 10 according to an embodiment and a method of manufacturing the light-emitting device 10 will be described with reference to FIG. 3.

### First electrode 110

In FIG. 3, a substrate may be further included under the first electrode 110 and/or on the second electrode 150. In an embodiment, the substrate may be a glass substrate and/or a plastic substrate. In an embodiment, the substrate may be a flexible substrate. For example, the flexible substrate may include plastic(s) with excellent or suitable heat resistance and durability, such as polyimide, polyethylene terephthalate (PET), polycarbonate, polyethylene naphthalate, polyarylate (PAR), polyetherimide, or any combination thereof.

The first electrode 110 may be formed by applying a material for forming the first electrode 110 onto the substrate by utilizing a deposition or sputtering method. When the first electrode 110 is an anode, a material for forming the first electrode 110 may be a high work function material to facilitate injection of holes.

The first electrode 110 may be a reflective electrode, a semi-transmissive electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. When the first electrode 110 is a semi-transmissive electrode or a reflective electrode, a material for forming the first electrode 110 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 110 may have a structure including (e.g., consisting of) a single layer or a structure including multiple layers. For example, the first electrode 110 may have a three-layered structure of ITO/Ag/ITO.

### Interlayer 130

The interlayer 130 may be disposed on the first electrode 110. The interlayer 130 may include an emission layer.

The interlayer 130 may further include a hole transport region between the first electrode 110 and the emission layer and an electron transport region between the emission layer and the second electrode 150.

The interlayer 130 may further include, in addition to one or more suitable organic materials, a metal-containing compound such as an organometallic compound, an inorganic material such as quantum dots, and/or the like.

The interlayer 130 may include two or more emitting units stacked between the first electrode 110 and the second electrode 150, and at least one charge generation layer located between the two or more emitting units. When the interlayer 130 includes the two or more emitting units and the at least one charge generation layer as described herein, the light-emitting device 10 may be a tandem light-emitting device.

### Hole transport region in interlayer 130

The hole transport region may have a structure including (e.g., consisting of) a layer including (e.g., consisting of) a single material, a structure including (e.g., consisting of) a layer including different materials, or a structure including multiple layers including different materials.

The hole transport region may include a hole injection layer, a hole transport layer, an emission auxiliary layer, an electron blocking layer, or any combination thereof.

In embodiments, the hole transport region may have a multi-layered structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein the layers of each structure may be stacked from the first electrode 110 in its respective stated order, but the structure of the hole transport region is not limited thereto.

The hole transport region may include a compound represented by Formula 201, a compound represented by Formula 202, or any combination thereof:

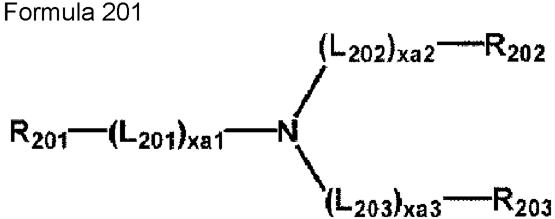

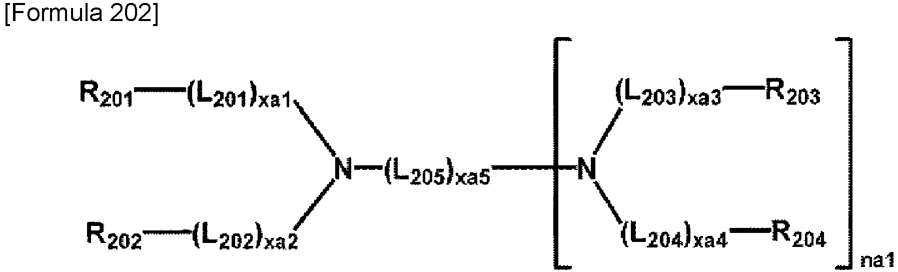

In Formulae 201 and 202,
L₂₀₁ to L₂₀₄ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ, or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ to form a C₈-C₆₀ polycyclic group (for example, a carbazole group and/or the like) unsubstituted or substituted with at least one R₁₀ₐ (for example, see Compound HT16),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ to form a C₈-C₆₀ polycyclic group unsubstituted or substituted with at least one R₁₀ₐ, and
na1 may be an integer from 1 to 4.

In embodiments, the compound represented by Formula 201 and the compound represented by Formula 202 may each independently include at least one of groups represented by Formulae CY201 to CY217:

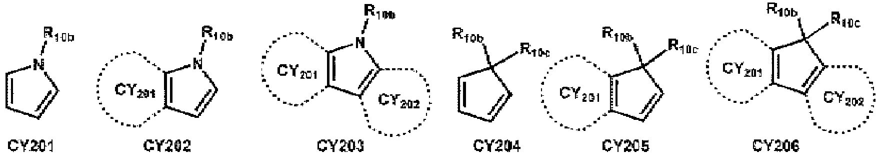

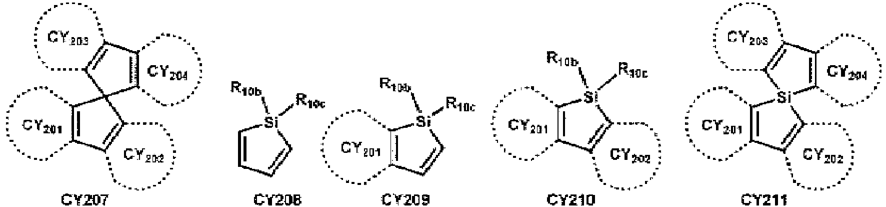

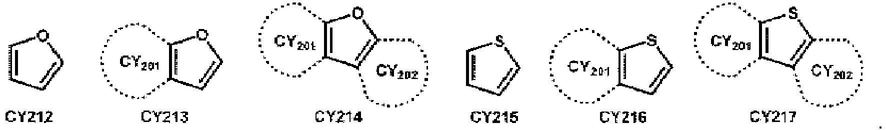

In Formulae CY201 to CY217, R_{10b} and R_{10c} may each independently be the same as described with respect to R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ as described herein.

In an embodiment, in Formulae CY201 to CY217, ring CY₂₀₁ to ring CY₂₀₄ may each independently be a benzene group, a naphthalene group, a phenanthrene group, or an anthracene group.

In embodiments, the compound represented by Formula 201 and the compound represented by Formula 202 may each independently include at least one of groups represented by Formulae CY201 to CY203.

In embodiments, the compound represented by Formula 201 may include at least one of the groups represented by Formulae CY201 to CY203 and at least one of groups represented by Formulae CY204 to CY217.

In embodiments, in Formula 201, xa1 may be 1, R₂₀₁ may be a group represented by one of Formulae CY201 to CY203, xa2 may be 0, and R₂₀₂ may be a group represented by one of Formulae CY204 to CY207.

In embodiments, the compound represented by Formula 201 and the compound represented by Formula 202 may each not include a group represented by one of Formulae CY201 to CY203.

In embodiments, the compound represented by Formula 201 and the compound represented by Formula 202 may each not include a group represented by one of Formulae CY201 to CY203, and may each independently include at least one of groups represented by Formulae CY204 to CY217.

In embodiments, the compound represented by Formula 201 and the compound represented by Formula 202 may each not include a group represented by one of Formulae CY201 to CY217.

In an embodiment, the hole transport region may include at least one selected from Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), and/or any combination thereof:

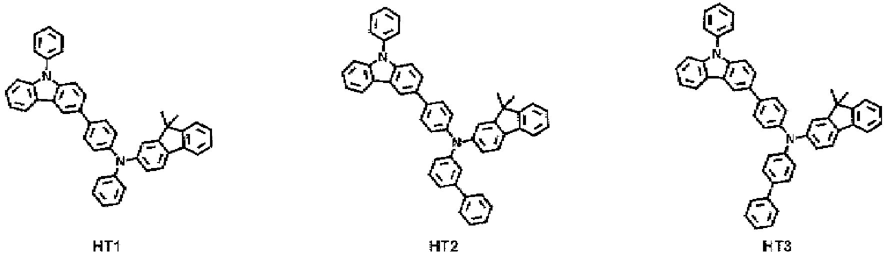

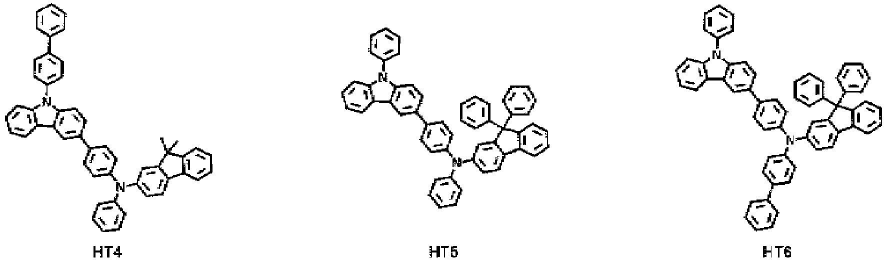

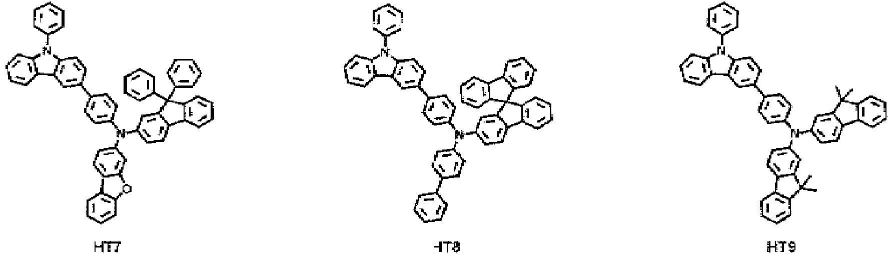

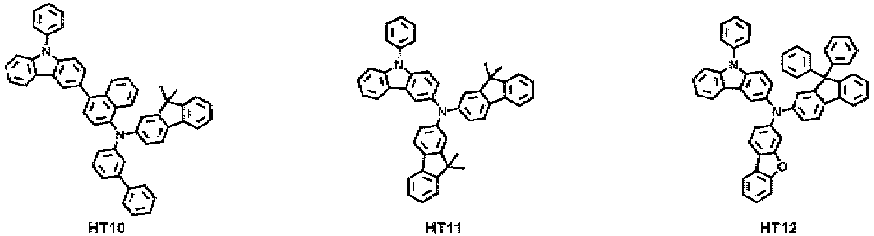

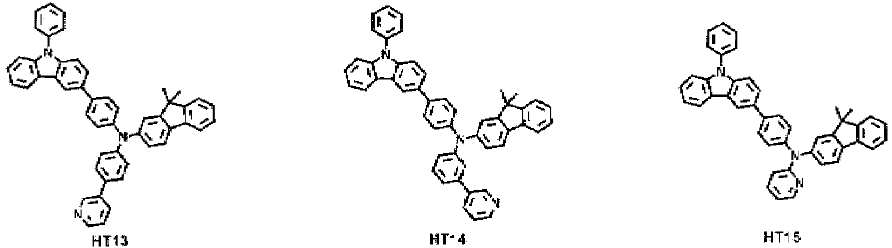

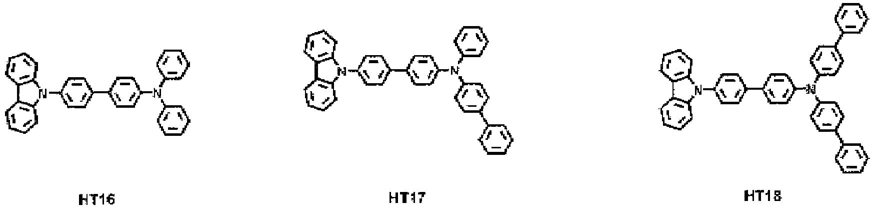

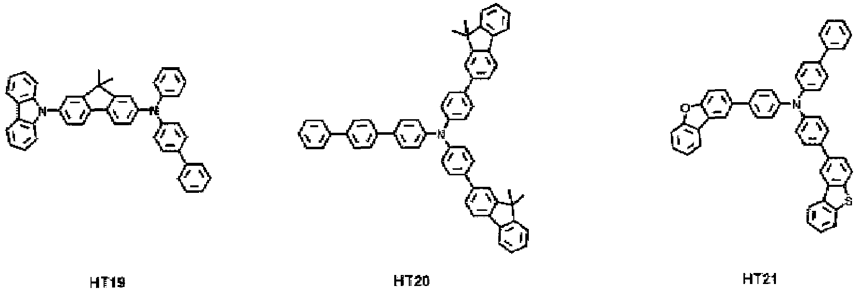

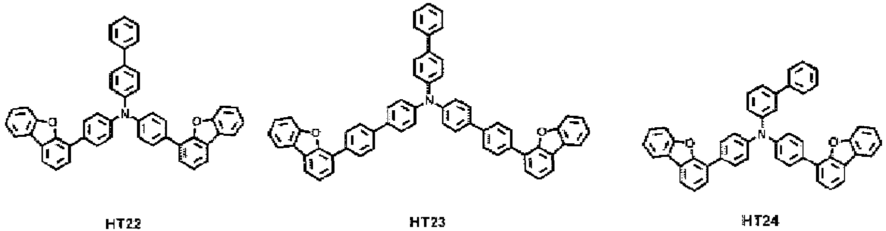

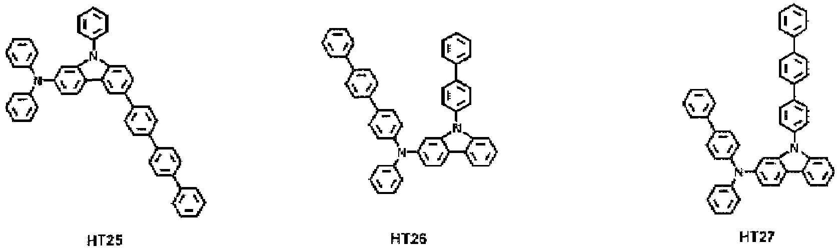

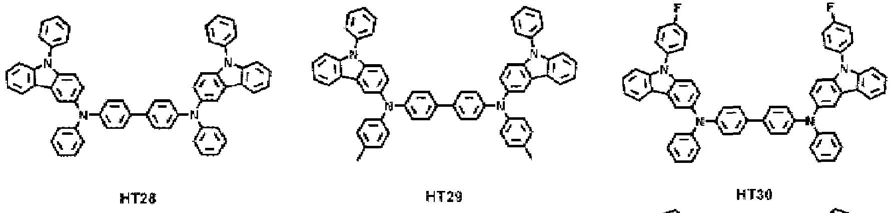

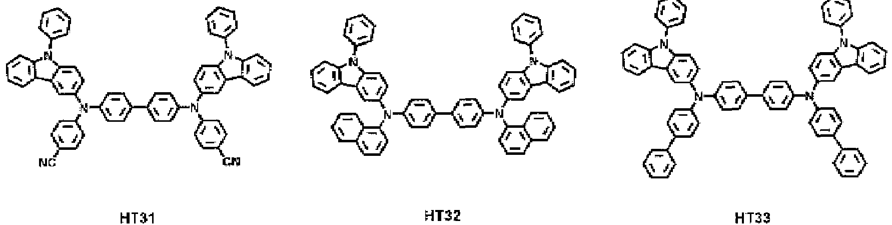

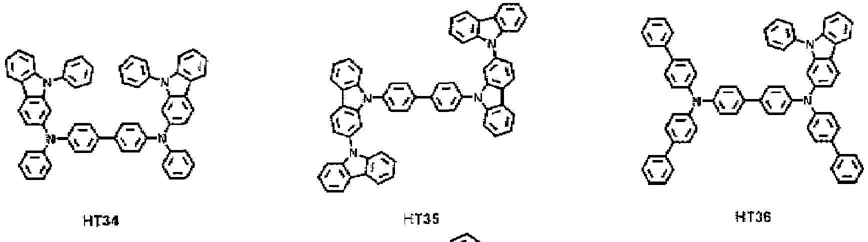

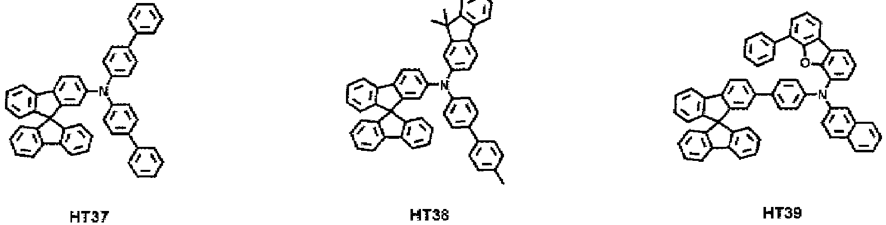

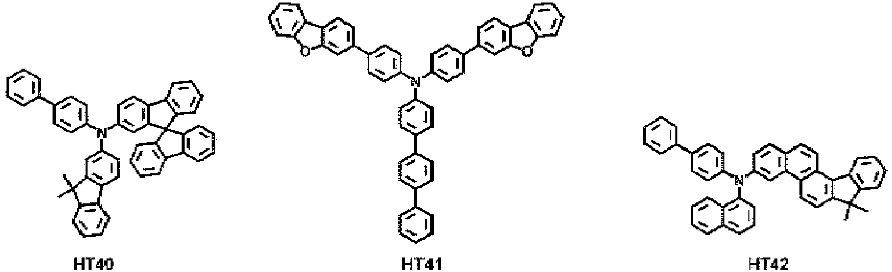

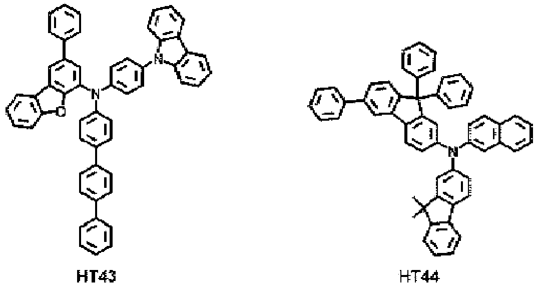

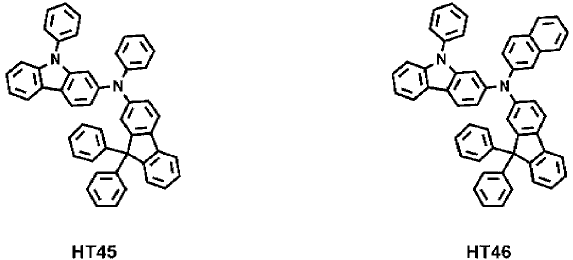

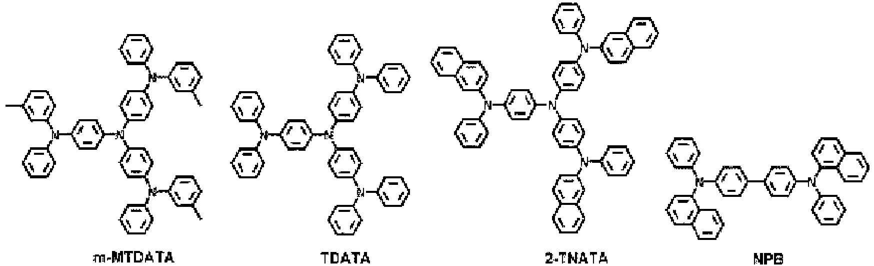

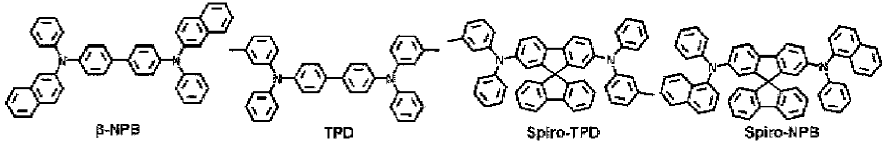

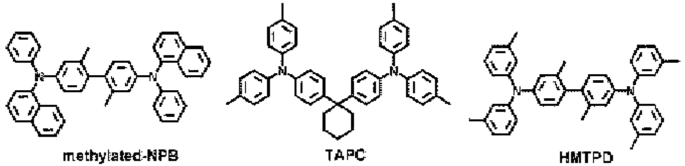

A thickness of the hole transport region may be in a range of about 5 nm (50 Å) to about 1,000 nm (10,000 Å). For example, the thickness of the hole transport region may be in a range of about 10 nm (100 Å) to about 400 nm (4,000 Å). When the hole transport region includes a hole injection layer, a hole transport layer, or any combination thereof, a thickness of the hole injection layer may be in a range of about 10 nm (100 Å) to about 900 nm (9,000 Å), and a thickness of the hole transport layer may be in a range of about 5 nm (50 Å) to about 200 nm (2,000 Å). For example, the thickness of the hole injection layer may be in a range of about 10 nm (100 Å) to about 100 nm (1,000 Å). For example, the thickness of the hole transport layer may be in a range of about 10 nm (100 Å) to about 150 nm (1,500 Å). When the thicknesses of the hole transport region, the hole injection layer, and the hole transport layer are within these ranges, satisfactory hole transporting characteristics may be obtained without a substantial increase in driving voltage.

The emission auxiliary layer may compensate for an optical resonance distance according to a wavelength of light emitted from the emission layer, so that light emitting efficiency may be improved. The electron blocking layer may prevent or reduce electron leakage from the emission layer to the hole transport region. Materials that may be included in the hole transport region may be included in the emission auxiliary layer and the electron blocking layer.

### p-dopant

The hole transport region may further include, in addition to these materials, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, a lowest unoccupied molecular orbital (LUMO) energy level of the p-dopant may be equal to or less than about -3.5 eV.

In embodiments, the p-dopant may include a quinone derivative, a cyano group-containing compound, a compound including element EL1 and element EL2, or any combination thereof.

Examples of the quinone derivative may include TCNQ, F4-TCNQ, etc.

Examples of the cyano group-containing compound may include HAT-CN and a compound represented by Formula 221:

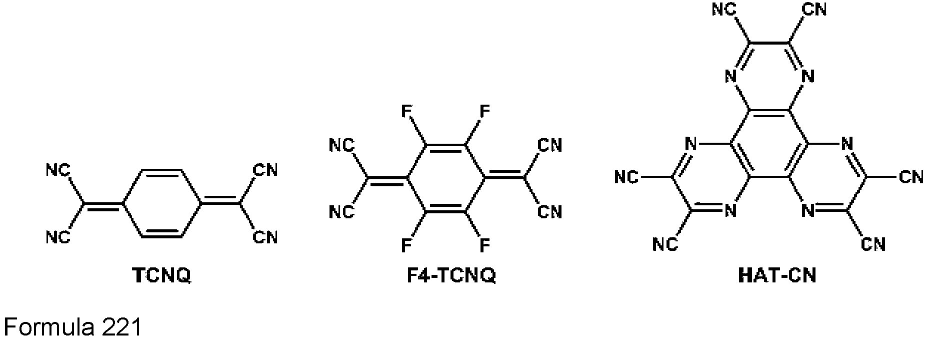

In Formula 221, 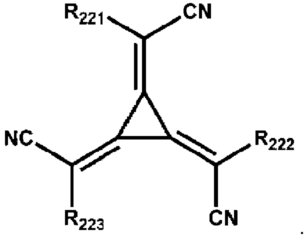
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with: a cyano group; -F; -Cl; -Br; -I; a C₁-C₂₀ alkyl group substituted with a cyano group, -F, -Cl, -Br, -I, or any combination thereof; or any combination thereof.

In the compound including element EL1 and element EL2, element EL1 may be a metal, a metalloid, or any combination thereof, and element EL2 may be a non-metal, a metalloid, or any combination thereof.

Examples of the metal may include: an alkali metal (for example, lithium (Li), sodium (Na), potassium (K), rubidium (Rb), cesium (Cs), etc.); an alkaline earth metal (for example, beryllium (Be), magnesium (Mg), calcium (Ca), strontium (Sr), barium (Ba), etc.); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); a post-transition metal (for example, zinc (Zn), indium (In), tin (Sn), etc.); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of the metalloid may include silicon (Si), antimony (Sb), and tellurium (Te).

Examples of the non-metal may include oxygen (O) and a halogen (for example, F, Cl, Br, I, etc.).

Examples of the compound including element EL1 and element EL2 may include a metal oxide, a metal halide (for example, a metal fluoride, a metal chloride, a metal bromide, or a metal iodide), a metalloid halide (for example, a metalloid fluoride, a metalloid chloride, a metalloid bromide, or a metalloid iodide), a metal telluride, or any combination thereof.

Examples of the metal oxide may include tungsten oxide (for example, WO, W₂O₃, WO₂, WO₃, W₂O₅, etc.), vanadium oxide (for example, VO, V₂O₃, VO₂, V₂O₅, etc.), molybdenum oxide (MoO, Mo₂O₃, MoO₂, MoO₃, Mo₂O₅, etc.), and rhenium oxide (for example, ReO₃, etc.).

Examples of the metal halide may include an alkali metal halide, an alkaline earth metal halide, a transition metal halide, a post-transition metal halide, and a lanthanide metal halide.

Examples of the alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and CsI

Examples of the alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, and BaI₂.

Examples of the transition metal halide may include a titanium halide (for example, TiF₄, TiCl₄, TiBr₄, TiI₄, etc.), a zirconium halide (for example, ZrF₄, ZrCl₄, ZrBr₄, ZrI₄, etc.), a hafnium halide (for example, HfF₄, HfCl₄, HfBr₄, HfI₄, etc.), a vanadium halide (for example, VF₃, VCl₃, VBr₃, VI₃, etc.), a niobium halide (for example, NbF₃, NbCl₃, NbBr₃, NbI₃, etc.), a tantalum halide (for example, TaF₃, TaCl₃, TaBr₃, TaI₃, etc.), a chromium halide (for example, CrF₃, CrCl₃, CrBr₃, CrI₃, etc.), a molybdenum halide (for example, MoF₃, MoCl₃, MoBr₃, MoI₃, etc.), a tungsten halide (for example, WF₃, WCl₃, WBr₃, WI₃, etc.), a manganese halide (for example, MnF₂, MnCl₂, MnBr₂, MnI₂, etc.), a technetium halide (for example, TcF₂, TcCl₂, TcBr₂, TcI₂, etc.), a rhenium halide (for example, ReF₂, ReCl₂, ReBr₂, ReI₂, etc.), an iron halide (for example, FeF₂, FeCl₂, FeBr₂, FeI₂, etc.), a ruthenium halide (for example, RuF₂, RuCl₂, RuBr₂, RuI₂, etc.), an osmium halide (for example, OsF₂, OsCl₂, OsBr₂, OsI₂, etc.), a cobalt halide (for example, CoF₂, CoCl₂, CoBr₂, CoI₂, etc.), a rhodium halide (for example, RhF₂, RhCl₂, RhBr₂, RhI₂, etc.), an iridium halide (for example, IrF₂, IrCl₂, IrBr₂, IrI₂, etc.), a nickel halide (for example, NiF₂, NiCl₂, NiBr₂, NiI₂, etc.), a palladium halide (for example, PdF₂, PdCl₂, PdBr₂, PdI₂, etc.), a platinum halide (for example, PtF₂, PtCl₂, PtBr₂, PtI₂, etc.), a copper halide (for example, CuF, CuCl, CuBr, Cul, etc.), a silver halide (for example, AgF, AgCl, AgBr, AgI, etc.), and a gold halide (for example, AuF, AuCl, AuBr, Aul, etc.).

Examples of the post-transition metal halide may include a zinc halide (for example, ZnF₂, ZnCl₂, ZnBr₂, ZnI₂, etc.), an indium halide (for example, InI₃, etc.), and a tin halide (for example, SnI₂, etc.).

Examples of the lanthanide metal halide may include YbF, YbF₂, YbF₃, SmF₃, YbCl, YbCl₂, YbCl₃ SmCl₃, YbBr, YbBr₂, YbBr₃, SmBr₃, Ybl, YbI₂, YbI₃, SmI₃, and/or the like.

Examples of the metalloid halide may include an antimony halide (for example, SbCl₅, etc.).

Examples of the metal telluride may include an alkali metal telluride (for example, Li₂Te, Na₂Te, K₂Te, Rb₂Te, Cs₂Te, etc.), an alkaline earth metal telluride (for example, BeTe, MgTe, CaTe, SrTe, BaTe, etc.), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), a post-transition metal telluride (for example, ZnTe, etc.), and a lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### Emission layer in interlayer 130

When the light-emitting device 10 is a full-color light-emitting device, the emission layer may be patterned into a red emission layer, a green emission layer, and/or a blue emission layer, according to a subpixel. In embodiments, the emission layer may have a stacked structure of two or more layers of a red emission layer, a green emission layer, and a blue emission layer, in which the two or more layers may contact each other or may be separated from each other to emit white light. In embodiments, the emission layer may include two or more materials of a red light-emitting material, a green light-emitting material, and a blue light-emitting material, in which the two or more materials may be mixed with each other in a single layer to emit white light.

The emission layer may include a host and a dopant. The dopant may include a phosphorescent dopant, a fluorescent dopant, or any combination thereof.

An amount of the dopant in the emission layer may be in a range of about 0.01 parts by weight to about 15 parts by weight, based on 100 parts by weight of the host.

In embodiments, the emission layer may include a quantum dot.

In embodiments, the emission layer may include a delayed fluorescence material. The delayed fluorescence material may serve as a host or as a dopant in the emission layer.

A thickness of the emission layer may be in a range of about 10 nm (100 Å) to about 100 nm (1,000 Å). For example, the thickness of the emission layer may be in a range of about 20 nm (200 Å) to about 60 nm (600 Å). When the thickness of the emission layer is within these ranges, excellent or suitable light-emission characteristics may be obtained without a substantial increase in driving voltage.

### Host

In embodiments, the host may include a compound represented by Formula 301:

Formula 301 [Ar₃₀₁]_{xb11}-[(L₃₀₁)_{xb1}-R₃₀₁]_{xb21}

In Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or -P(=O)(Q₃₀₁)(Q₃₀₂),
xb21 may be an integer from 1 to 5, and
Q₃₀₁ to Q₃₀₃ may each independently be the same as described herein with respect to Q₁₁.

In an embodiment, in Formula 301, when xb11 is 2 or more, two or more of Ar₃₀₁ may be linked to each other via a single bond.

In embodiments, the host may include a compound represented by Formula 301-1, a compound represented by Formula 301-2, or any combination thereof:

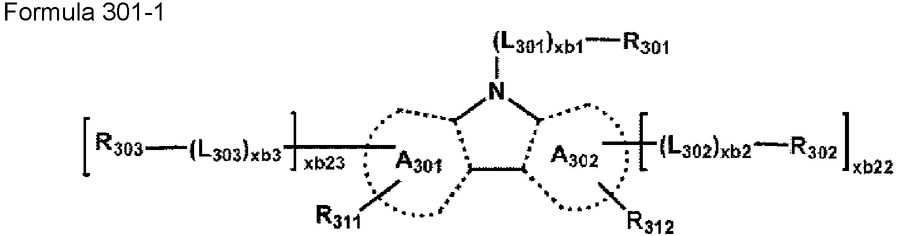

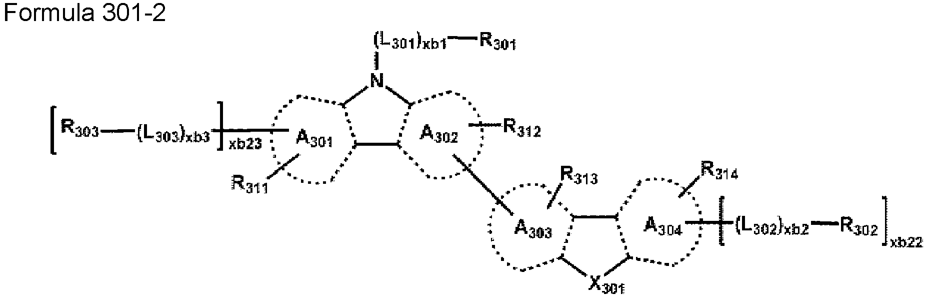

In Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be the same as described herein,
L₃₀₂ to L₃₀₄ may each independently be the same as described herein with respect to with L₃₀₁,
xb2 to xb4 may each independently be the same as described herein with respect to xb1, and
R₃₀₂ to R₃₀₅ and R₃₁₁ to R₃₁₄ may each independently be the same as described herein with respect to R₃₀₁.

In embodiments, the host may include an alkali earth metal complex, a post-transition metal complex, or any combination thereof. For example, the host may include a Be complex (for example, Compound H55), an Mg complex, a Zn complex, or any combination thereof.

In embodiments, the host may include at least one selected from Compounds H1 to H128, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and/or any combination thereof:

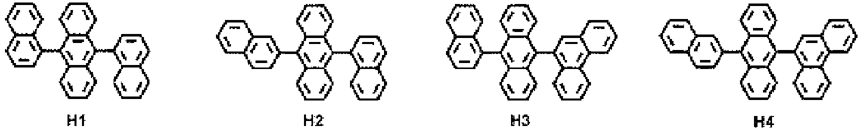

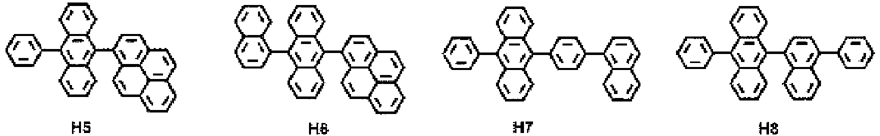

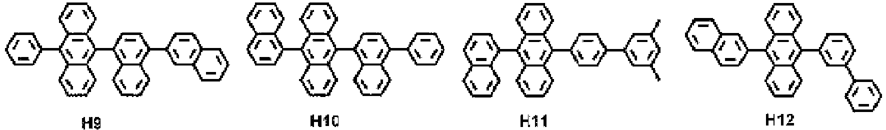

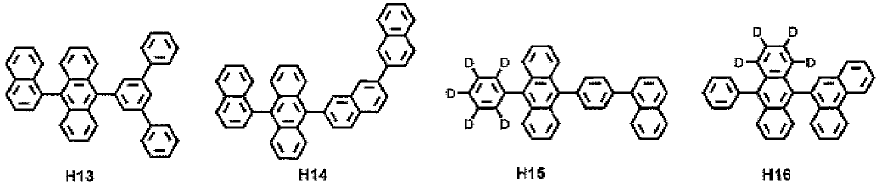

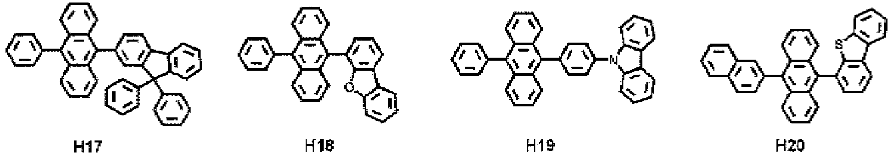

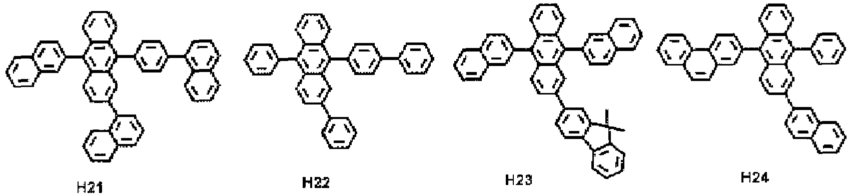

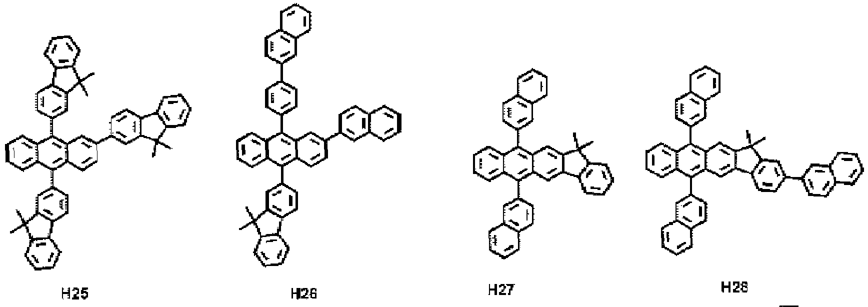

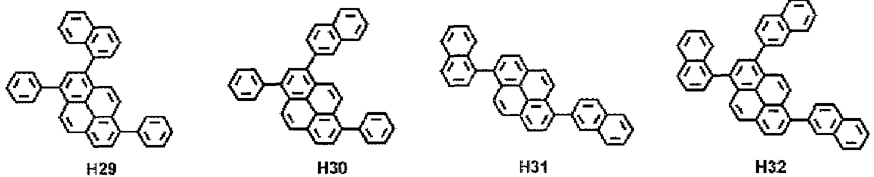

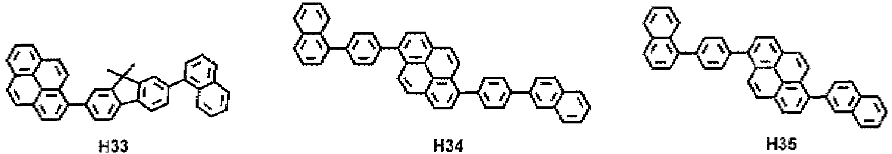

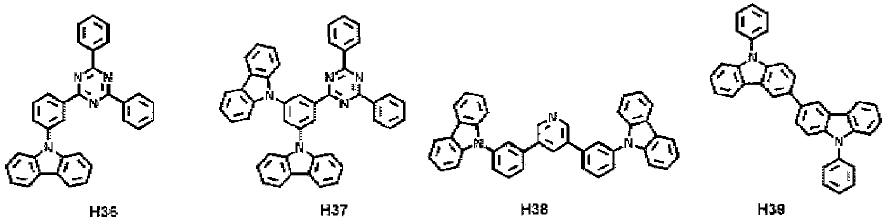

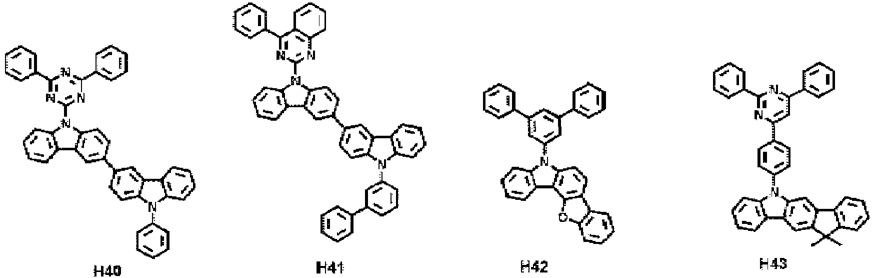

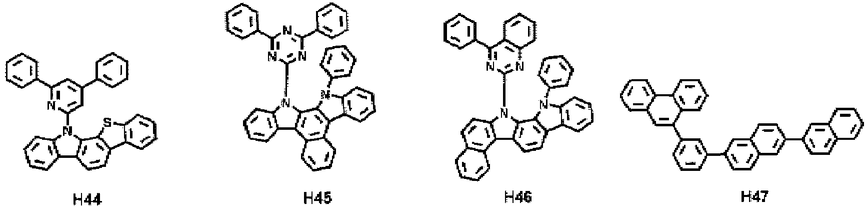

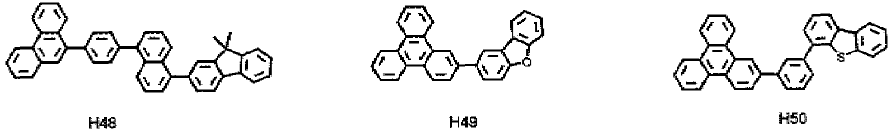

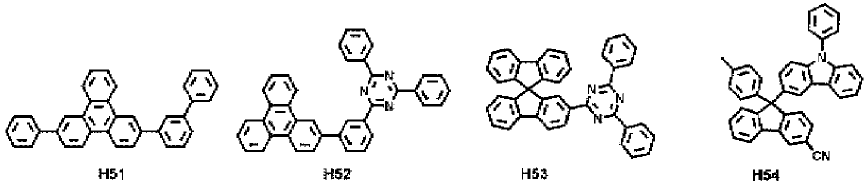

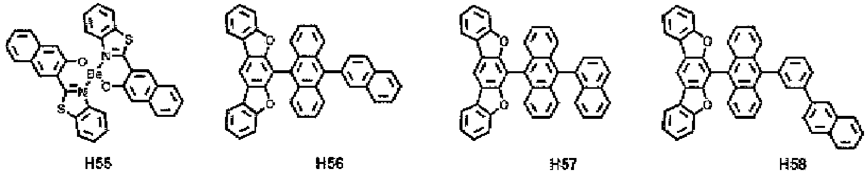

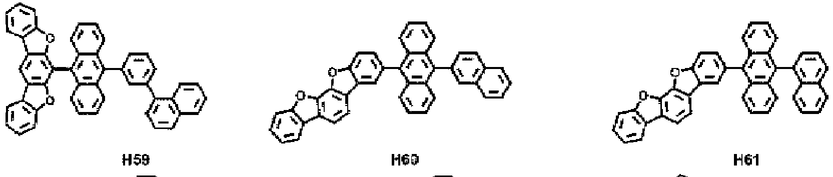

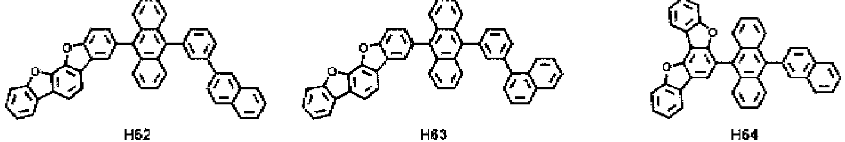

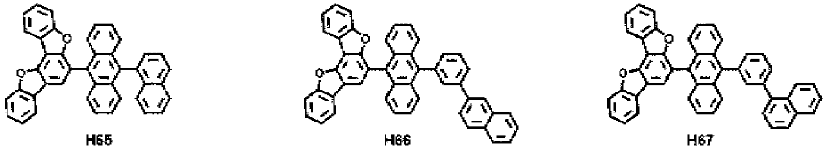

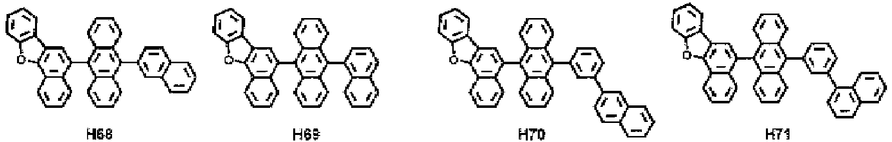

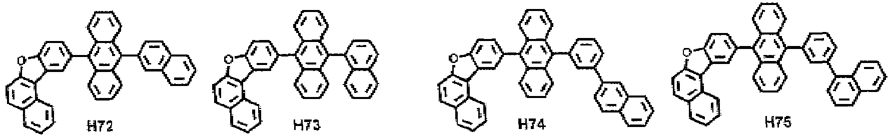

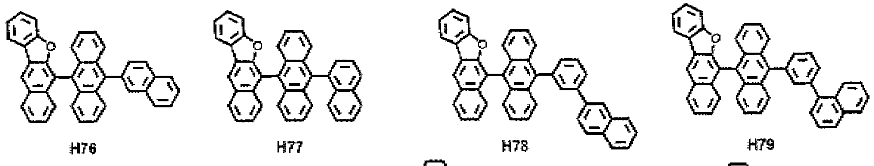

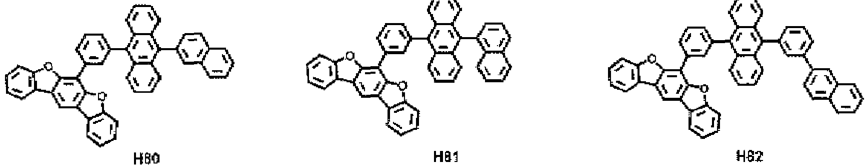

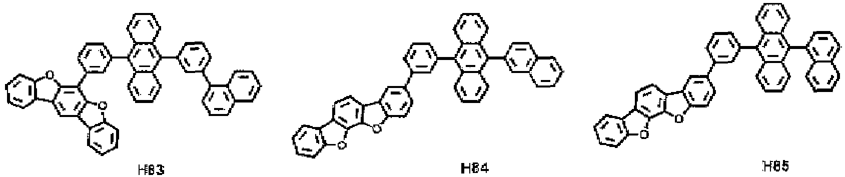

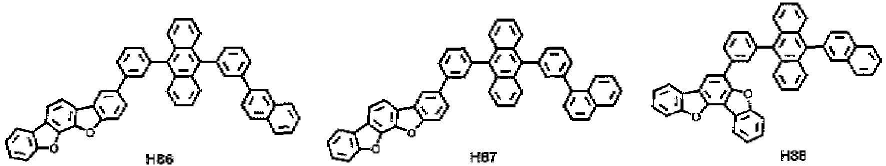

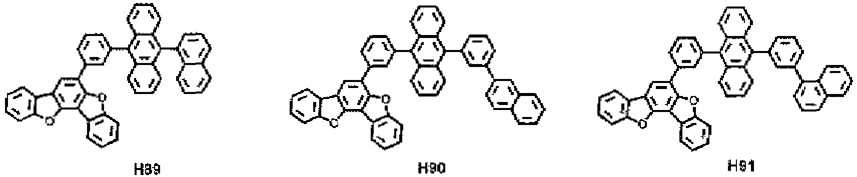

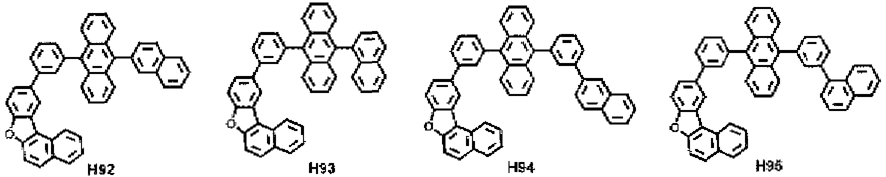

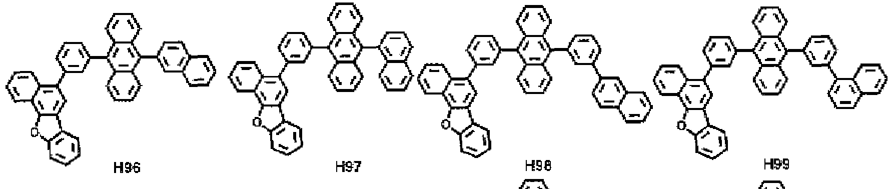

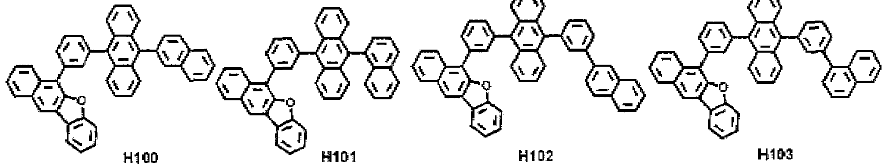

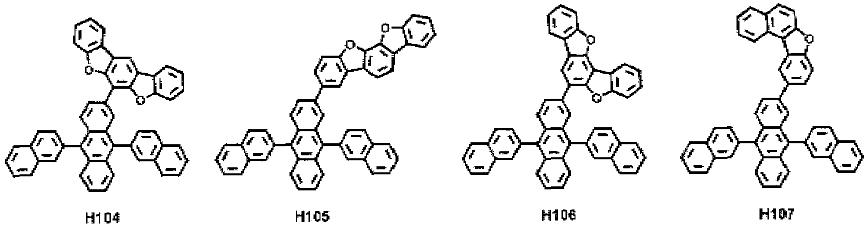

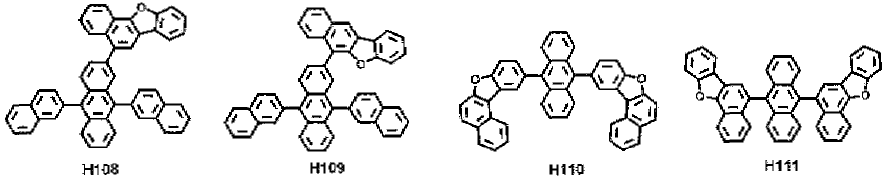

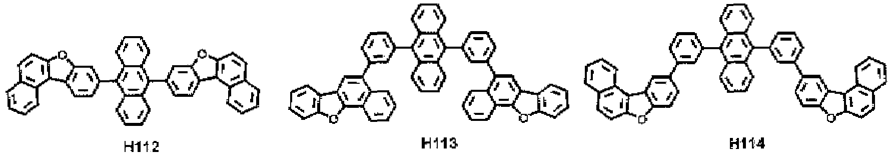

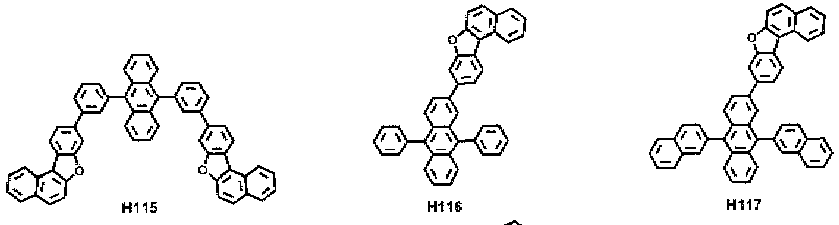

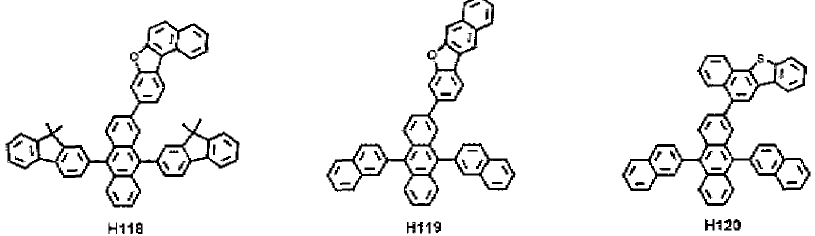

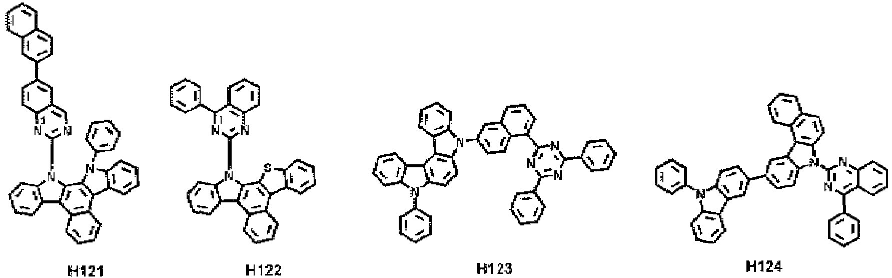

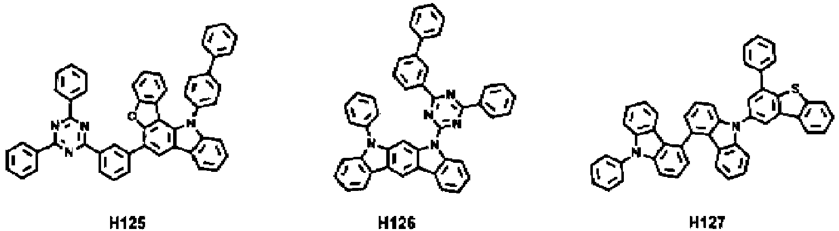

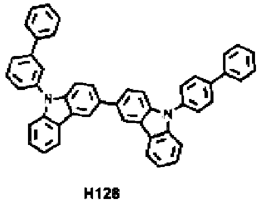

### Phosphorescent dopant

In embodiments, the phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In embodiments, the phosphorescent dopant may include an organometallic compound represented by Formula 401:

Formula 401 M(L₄₀₁)_{xc1}(L₄₀₂)_{xc2}

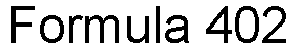

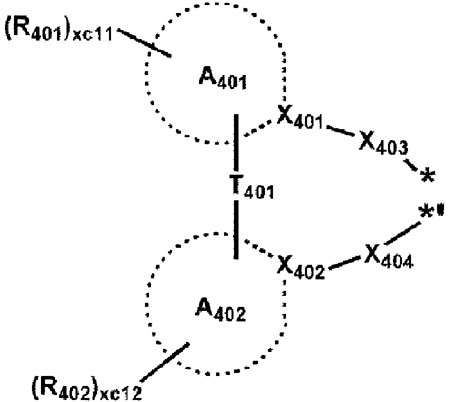

In Formulae 401 and 402,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 may be 1, 2, or 3, wherein when xc1 is two or more, two or more of L₄₀₁ may be identical to or different from each other,
L₄₀₂ may be an organic ligand, and xc2 may be 0, 1, 2, 3, or 4, wherein when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordination bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each independently be the same as described herein with respect to Q₁₁,
R₄₀₁ and R₄₀₂ may each independently be hydrogen, deuterium, -F, -Cl, -Br, - I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
Q₄₀₁ to Q₄₀₃ may each independently be the same as described herein with respect to Q₁₁,
xc11 and xc12 may each independently be an integer from 0 to 10, and
* and *' in Formula 402 each indicate a binding site to M in Formula 401.

In an embodiment, in Formula 402, X₄₀₁ may be nitrogen and X₄₀₂ may be carbon, or X₄₀₁ and X₄₀₂ may each be nitrogen.

In embodiments, in Formula 401, when xc1 is 2 or more, two ring A₄₀₁(s) in two or more of L₄₀₁ may optionally be linked to each other via T₄₀₂, which is a linking group, and two ring A₄₀₂(s) may optionally be linked to each other via T₄₀₃, which is a linking group (see Compounds PD1 to PD4 and PD7). T₄₀₂ and T₄₀₃ may each independently be the same as described herein with respect to T₄₀₁.

In Formula 401, L₄₀₂ may be an organic ligand. In an embodiment, L₄₀₂ may include a halogen group, a diketone group (for example, an acetylacetonate group), a carboxylic acid group (for example, a picolinate group), -C(=O), an isonitrile group, a - CN group, a phosphorus group (for example, a phosphine group, a phosphite group, etc.), or any combination thereof.

The phosphorescent dopant may include, for example, at least one selected from Compounds PD1 to PD39, and/or any combination thereof:

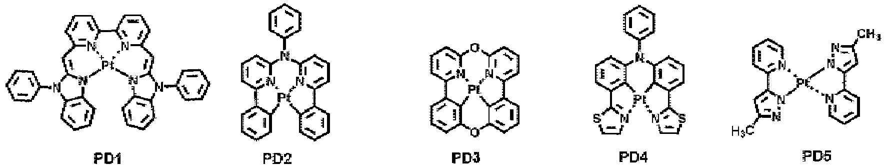

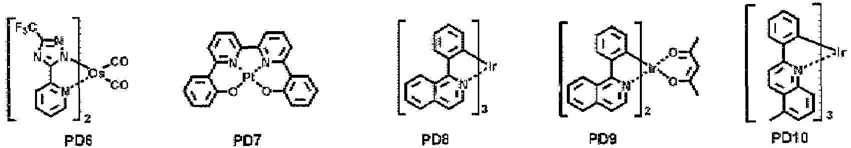

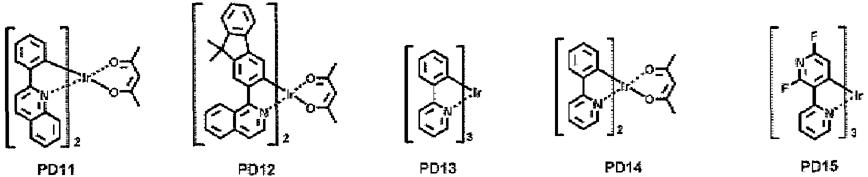

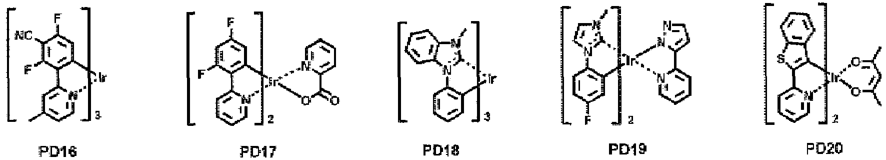

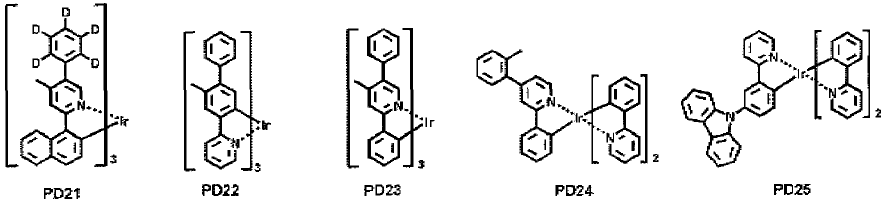

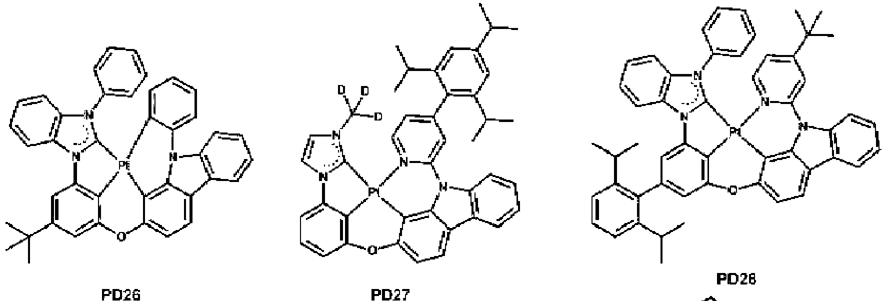

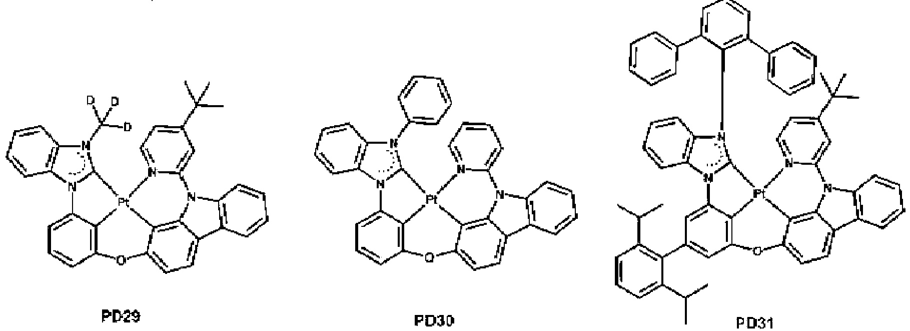

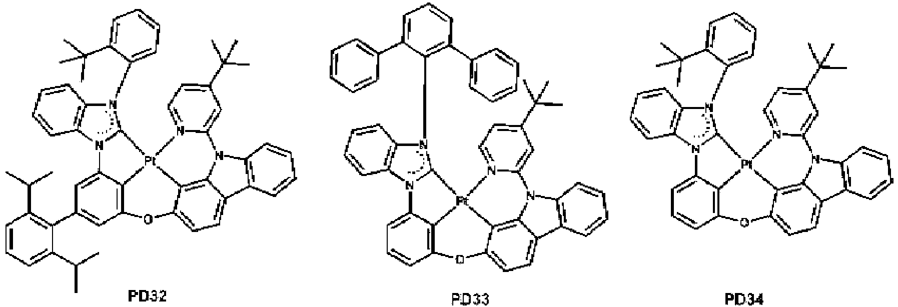

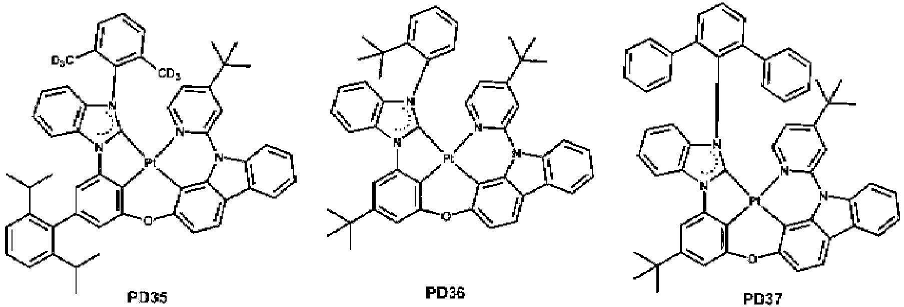

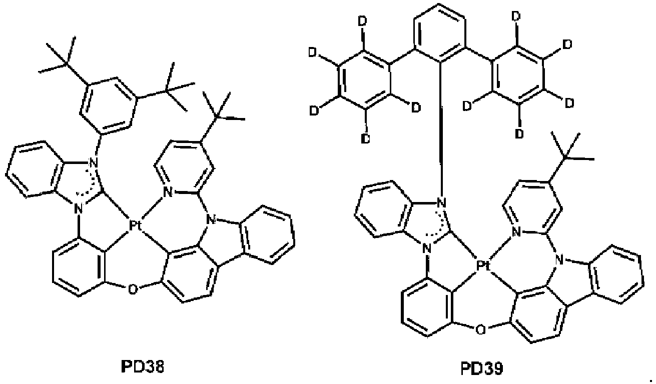

### Fluorescent dopant

The fluorescent dopant may include an amine group-containing compound, a styryl group-containing compound, or any combination thereof.

In an embodiment, the fluorescent dopant may include a compound represented by Formula 501:

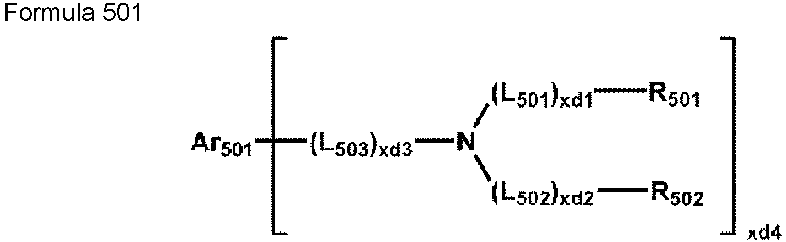

In Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In an embodiment, in Formula 501, Ar₅₀₁ may be a condensed cyclic group (for example, an anthracene group, a chrysene group, or a pyrene group) in which three or more monocyclic groups are condensed together.

In embodiments, in Formula 501, xd4 may be 2.

In an embodiment, the fluorescent dopant may include at least one selected from Compounds FD1 to FD37, DPVBi, DPAVBi, and/or any combination thereof:

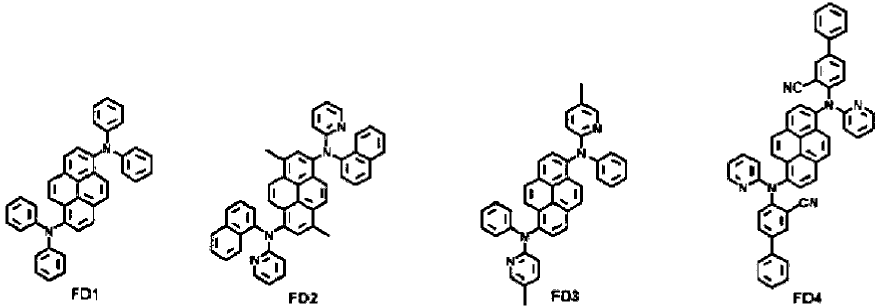

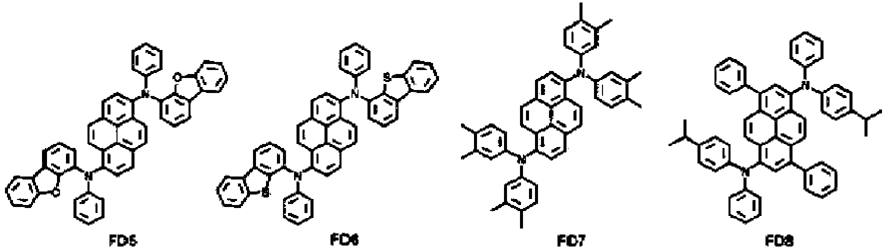

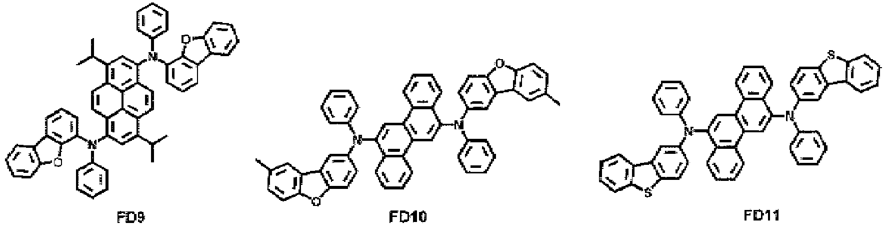

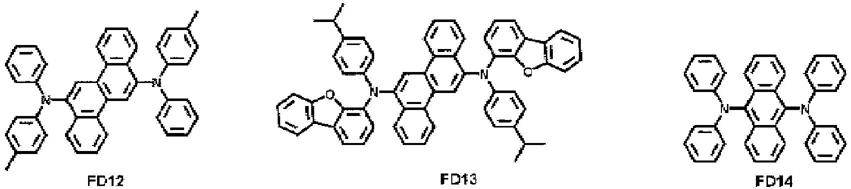

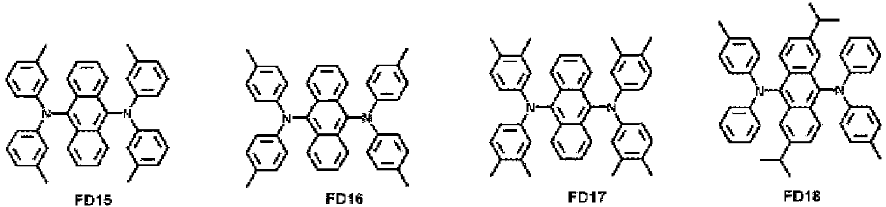

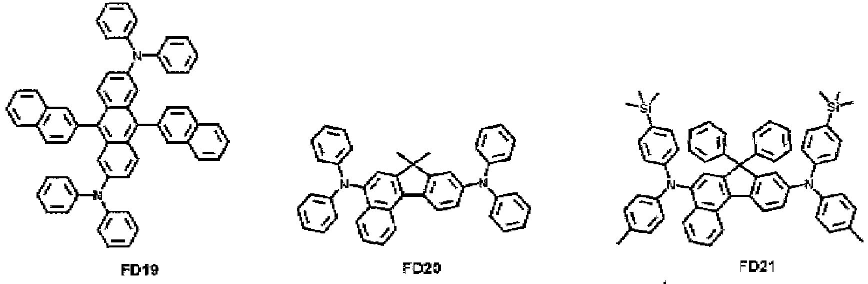

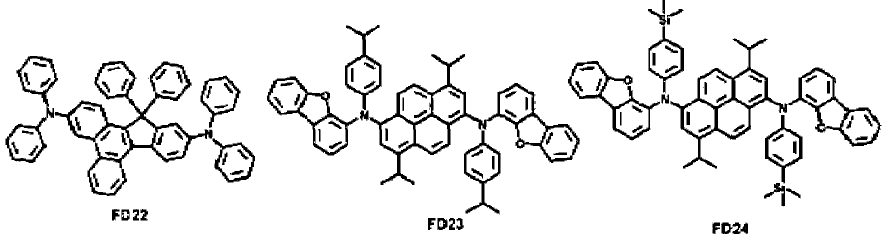

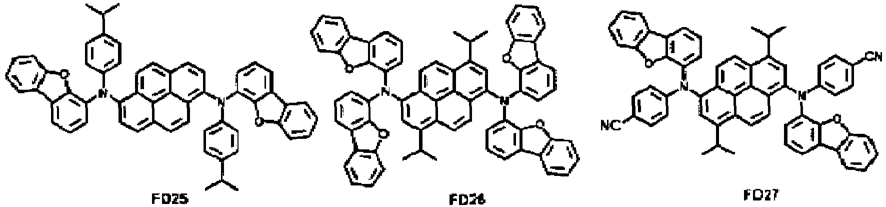

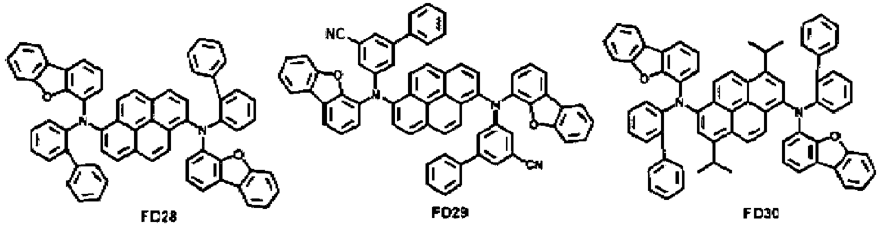

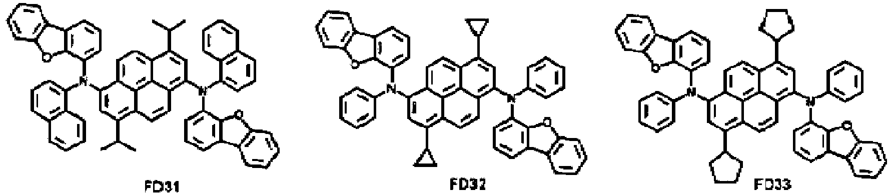

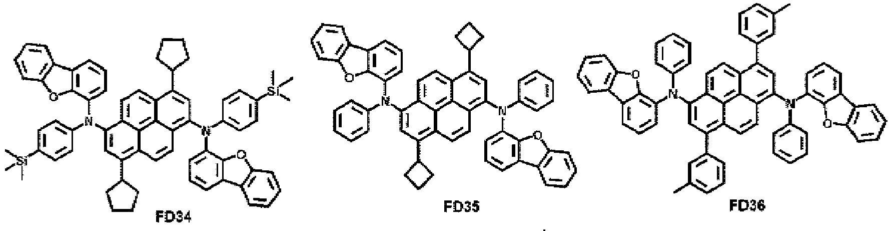

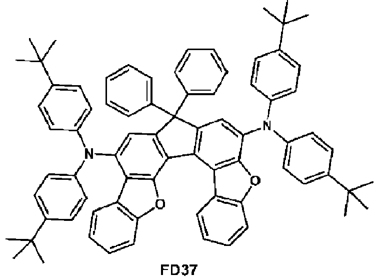

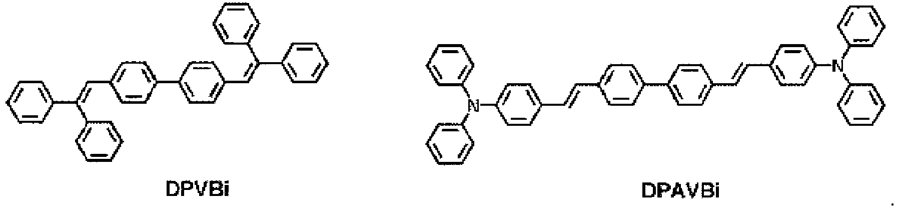

### Delayed fluorescence material

The emission layer may include a delayed fluorescence material.

In the invention, the delayed fluorescence material may be selected from compounds capable of emitting delayed fluorescent light based on a delayed fluorescence emission mechanism.

The delayed fluorescence material included in the emission layer may serve as a host or as a dopant, depending on the types (kinds) of other materials included in the emission layer.

In an embodiment, a difference between a triplet energy level (eV) of the delayed fluorescence material and a singlet energy level (eV) of the delayed fluorescence material may be in a range of about 0 eV to about 0.5 eV. When the difference between the triplet energy level (eV) of the delayed fluorescence material and the singlet energy level (eV) of the delayed fluorescence material satisfies the above-described range, up-conversion from the triplet state to the singlet state of the delayed fluorescence materials may effectively occur, and thus, the luminescence efficiency of the light-emitting device 10 may be improved.

In an embodiment, the delayed fluorescence material may include: a material including at least one electron donor (for example, a π electron-rich C₃-C₆₀ cyclic group and/or the like, such as a carbazole group) and at least one electron acceptor (for example, a sulfoxide group, a cyano group, a π electron-deficient nitrogen-containing C₁-C₆₀ cyclic group, and/or the like); a material including a C₈-C₆₀ polycyclic group in which at least two cyclic groups are condensed to each other while sharing boron (B); and/or the like.

Examples of the delayed fluorescence material may include at least one of Compounds DF1 to DF14:

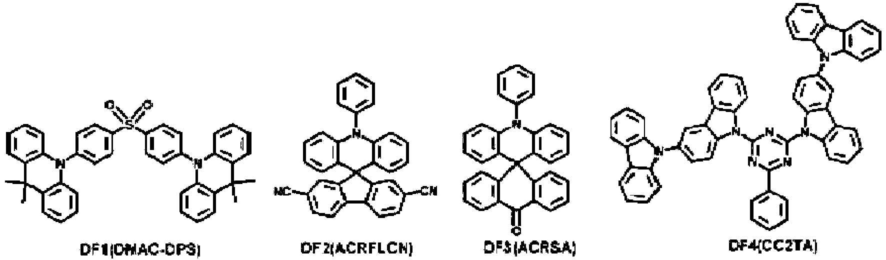

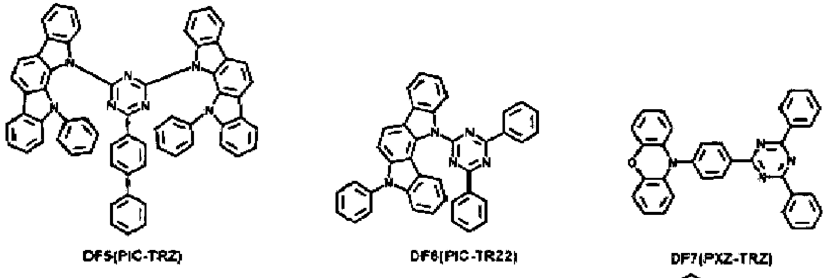

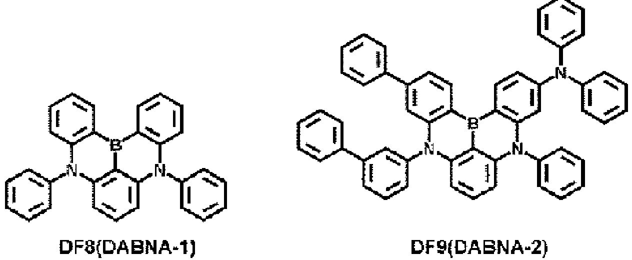

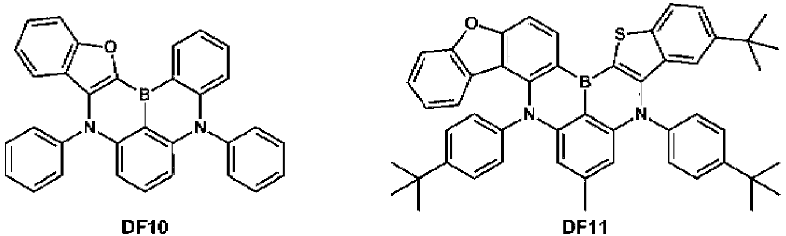

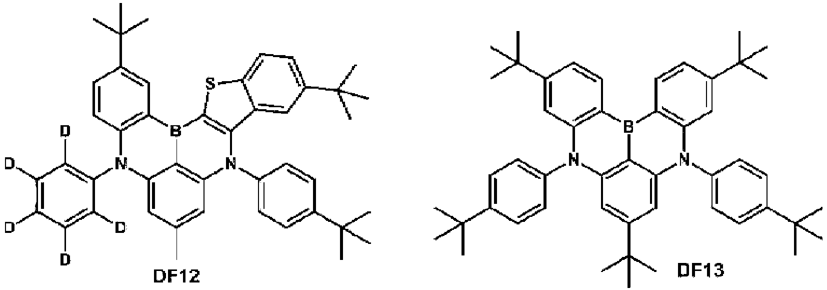

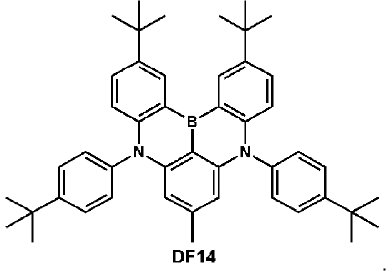

### Quantum dot

The emission layer may include a quantum dot.

In the specification, a quantum dot may be a crystal of a semiconductor compound, and may include any material capable of emitting light of one or more suitable emission wavelengths according to a size of the crystal.

A diameter of the quantum dot may be, for example, in a range of about 1 nm to about 10 nm.

The quantum dot may be synthesized by a wet chemical process, a metal organic chemical vapor deposition process, a molecular beam epitaxy process, or any process similar thereto.

The wet chemical process is a method including mixing a precursor material with an organic solvent and growing a quantum dot particle crystal. When the crystal grows, the organic solvent naturally acts as a dispersant coordinated on the surface of the quantum dot crystal and controls the growth of the crystal so that the growth of quantum dot particles can be controlled or selected through a process which costs less, and may be more readily performed than vapor deposition methods, such as metal organic chemical vapor deposition (MOCVD) or molecular beam epitaxy (MBE),

The quantum dot may include a Group II-VI semiconductor compound, a Group III-V semiconductor compound, a Group III-VI semiconductor compound, a Group I-III-VI semiconductor compound, a Group IV-VI semiconductor compound, a Group IV element or compound, or any combination thereof.

Examples of the Group II-VI semiconductor compound may include: a binary compound, such as CdS, CdSe, CdTe, ZnS, ZnSe, ZnTe, ZnO, HgS, HgSe, HgTe, MgSe, or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, or MgZnS; a quaternary compound, such as CdZnSeS, CdZnSeTe, CdZnSTe, CdHgSeS, CdHgSeTe, CdHgSTe, HgZnSeS, HgZnSeTe, or HgZnSTe; or any combination thereof.

Examples of the Group III-V semiconductor compound may include: a binary compound, such as GaN, GaP, GaAs, GaSb, AIN, AlP, AlAs, AlSb, InN, InP, InAs, or InSb; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, or InPSb; a quaternary compound, such as GaAlNP, GaAINAs, GaAINSb, GaAlPAs, GaAlPSb, GaInNP, GaInNAs, GaInNSb, GaInPAs, GaInPSb, InAlNP, InAINAs, InAlNSb, InAlPAs, or InAlPSb; or any combination thereof. In an embodiment, the Group III-V semiconductor compound may further include a Group II element. Examples of the Group III-V semiconductor compound further including a Group II element may include InZnP, InGaZnP, InAlZnP, etc.

Examples of the Group III-VI semiconductor compound may include: a binary compound, such as GaS, GaSe, Ga₂Se₃, GaTe, InS, InSe, In₂S₃, In₂Se₃, or InTe; a ternary compound, such as InGaS₃, or InGaSe₃; and any combination thereof.

Examples of the Group I-III-VI semiconductor compound may include: a ternary compound, such as AgInS, AgInS₂, CuInS, CuInS₂, CuGaO₂, AgGaO₂, or AgAlO₂; or any combination thereof.

Examples of the Group IV-VI semiconductor compound may include: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, or SnPbTe; a quaternary compound, such as SnPbSSe, SnPbSeTe, or SnPbSTe; or any combination thereof.

Examples of the Group IV element or compound may include: a single element material, such as Si or Ge; a binary compound, such as SiC or SiGe; or any combination thereof.

Each element included in a multi-element compound such as a binary compound, a ternary compound, or a quaternary compound may be present in a particle at a substantially uniform concentration or at a non-substantially uniform concentration.

In embodiments, the quantum dot may have a single structure in which the concentration of each element in the quantum dot is substantially uniform, or the quantum dot may have a core-shell structure. In an embodiment, in case that the quantum dot has a core-shell structure, a material included in the core and a material included in the shell may be different from each other.

The shell of the quantum dot may serve as a protective layer that prevents chemical degeneration of the core to maintain semiconductor characteristics, and/or may serve as a charging layer that imparts electrophoretic characteristics to the quantum dot. The shell may be a single layer or a multi-layer. An interface between the core and the shell may have a concentration gradient in which the concentration of a material that is present in the shell decreases toward the core.

Examples of the shell of the quantum dot may include a metal oxide, a metalloid oxide, a non-metal oxide, a semiconductor compound, or any combination thereof. Examples of the metal oxide, the metalloid oxide, or the non-metal oxide may include: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO; a ternary compound, such as MgAl₂O₄, CoFe₂O₄, NiFe₂O₄, or CoMn₂O₄; or any combination thereof.

Examples of the semiconductor compound may include, as described herein, a Group II-VI semiconductor compound, a Group III-V semiconductor compound, a Group III-VI semiconductor compound, a Group I-III-VI semiconductor compound, a Group IV-VI semiconductor compound, or any combination thereof. Examples of the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or any combination thereof.

A full width at half maximum (FWHM) of an emission wavelength spectrum of the quantum dot may be equal to or less than about 45 nm. For example, a FWHM of an emission wavelength spectrum of the quantum dot may be equal to or less than about 40 nm. For example, a FWHM of an emission wavelength spectrum of the quantum dot may be equal to or less than about 30 nm. Within these ranges, color purity or color reproducibility may be increased. Light emitted through a quantum dot may be emitted in all directions, so that a wide viewing angle may be improved.

In embodiments, the quantum dot may be in the form of a spherical particle, a pyramidal particle, a multi-arm particle, a cubic nanoparticle, a nanotube particle, a nanowire particle, a nanofiber particle, or a nanoplate particle.

Because the energy band gap may be adjusted by controlling the size of the quantum dot, light having one or more suitable wavelength bands may be obtained from a quantum dot emission layer. Accordingly, by utilizing quantum dots of different sizes, a light-emitting device that emits light of one or more suitable wavelengths may be implemented. In embodiments, the size of the quantum dot may be selected to emit red, green, and/or blue light. In an embodiment, the size of the quantum dot may be configured to emit white light by combination of light of one or more suitable colors.

### Electron transport region in interlayer 130

The electron transport region may have a structure including (e.g., consisting of) a layer including (e.g., consisting of) a single material, a structure consisting of a layer including different materials, or a structure including multiple layers including different materials.

The electron transport region may include a buffer layer, a hole blocking layer, an electron control layer, an electron transport layer, an electron injection layer, or any combination thereof.

In embodiments, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein the layers of each structure may be stacked from an emission layer in its respective stated order, but the structure of the electron transport region is not limited thereto.

In an embodiment, the electron transport region (for example, a buffer layer, a hole blocking layer, an electron control layer, or an electron transport layer in the electron transport region) may include a metal-free compound including at least one π electron-deficient nitrogen-containing C₁-C₆₀ cyclic group.

In embodiments, the electron transport region may include a compound represented by Formula 601:

Formula 601 [Ar₆₀₁]ₓₑ₁₁-[(L₆₀₁)ₓₑ₁-R₆₀₁]ₓₑ₂₁

In Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, -Si(O₆₀₁)(O₆₀₂)(O₆₀₃), -C(=O)(Q₆₀₁), -S(=O)₂(Q₆₀₁), or -P(=O)(O₆₀₁)(O₆₀₂),
Q₆₀₁ to Q₆₀₃ may each independently be the same as described herein with respect to Q₁₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group unsubstituted or substituted with at least one R₁₀ₐ.

In an embodiment, in Formula 601, when xe11 is 2 or more, two or more of Ar₆₀₁ may be linked to each other via a single bond.

In embodiments, in Formula 601, Ar₆₀₁ may be an anthracene group unsubstituted or substituted with at least one R₁₀ₐ

In embodiments, the electron transport region may include a compound represented by Formula 601-1:

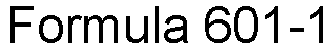

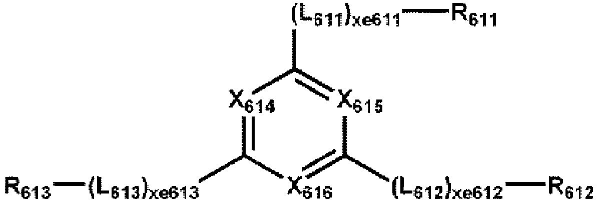

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may each be N,
L₆₁₁ to L₆₁₃ may each independently be the same as described herein with respect to L₆₀₁,
xe611 to xe613 may each independently be the same as described herein with respect to xe1,
R₆₁₁ to R₆₁₃ may each independently be the same as described herein with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

In embodiments, in Formulae 601 and 601-1, xe1 and xe611 to xe613 may each independently be 0, 1, or 2.

In embodiments, the electron transport region may include at least one selected from Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, and/or any combination thereof:

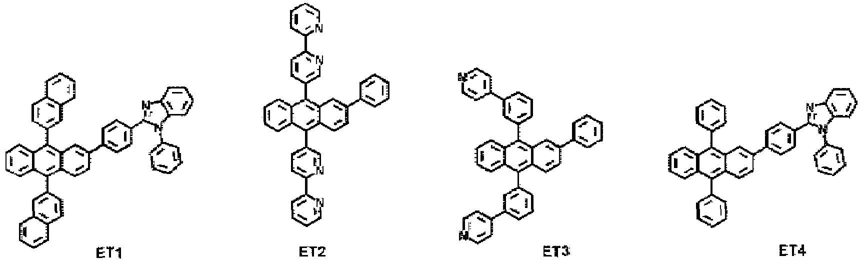

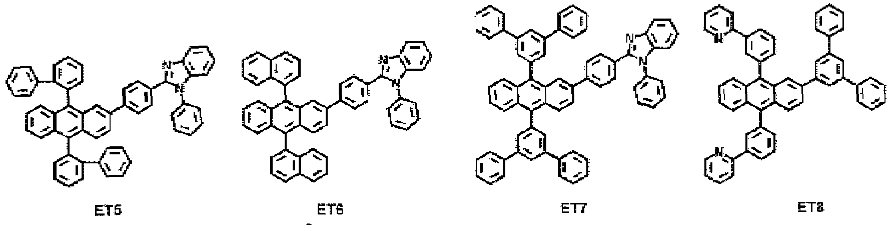

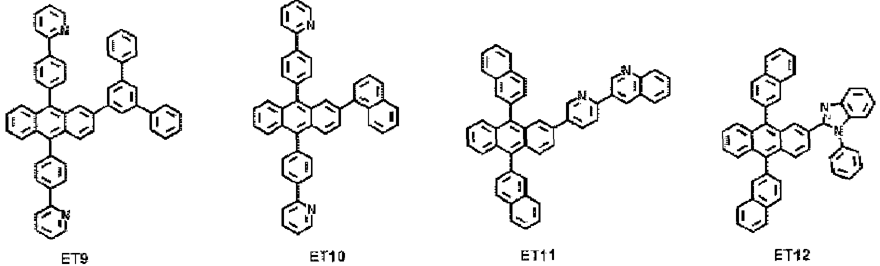

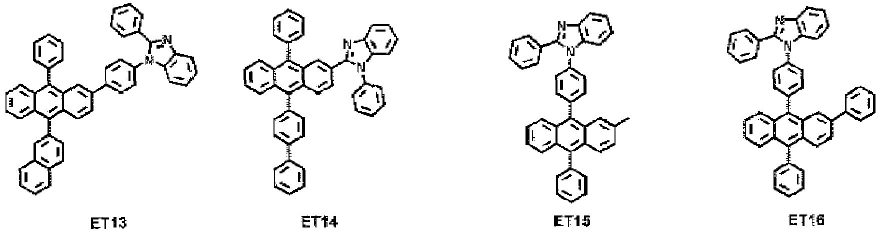

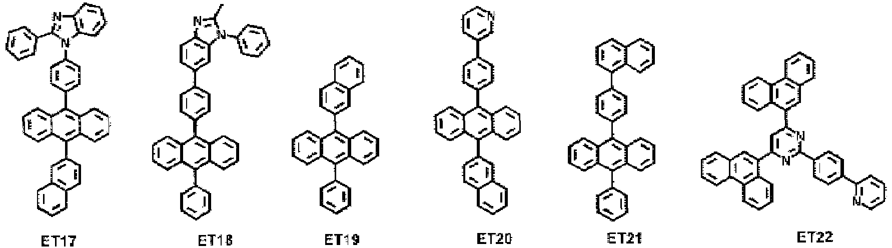

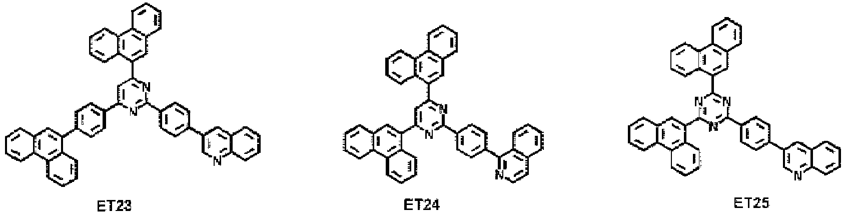

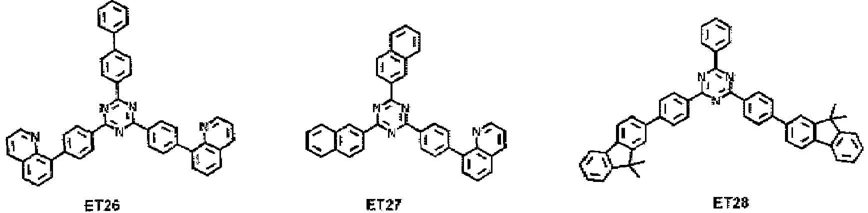

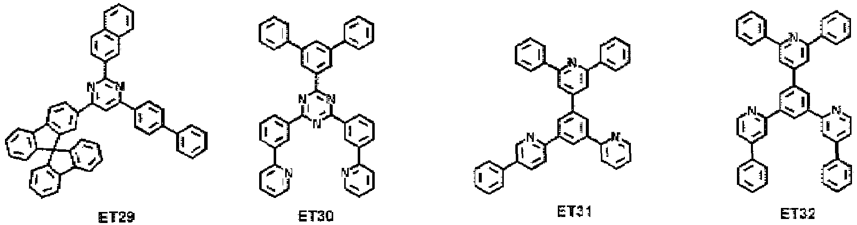

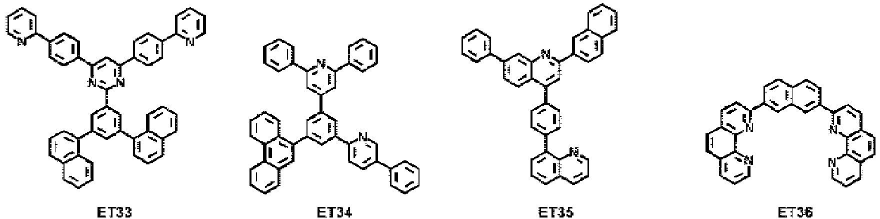

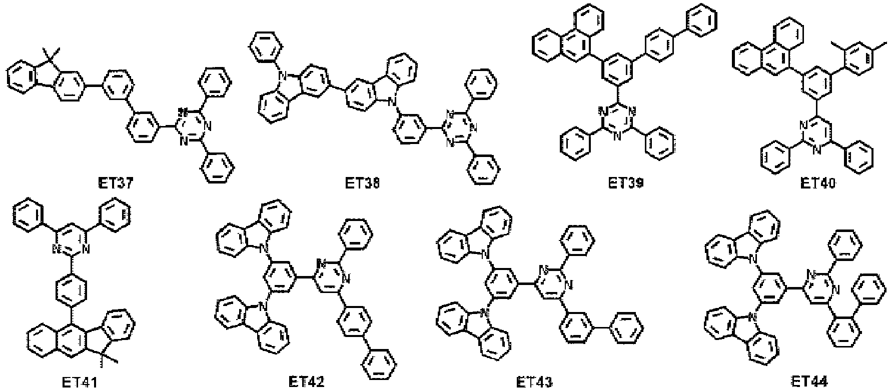

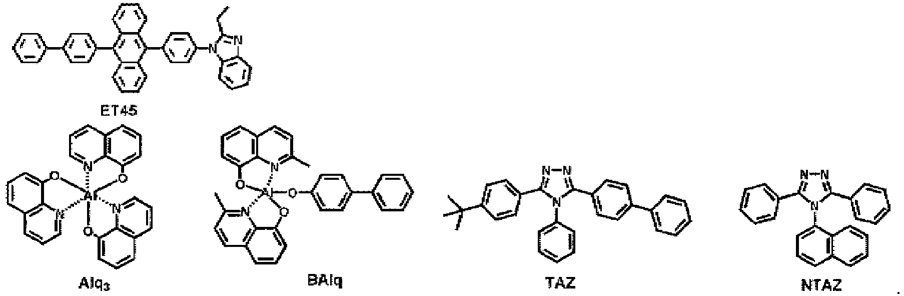

A thickness of the electron transport region may be in a range of about 10 nm (100 Å) to about 500 nm (5,000 Å). For example, the thickness of the electron transport region may be in a range of about 16 nm (160 Å) to about 400 nm (4,000 Å). When the electron transport region includes a buffer layer, a hole blocking layer, an electron control layer, an electron transport layer, or any combination thereof, a thickness of the buffer layer, the hole blocking layer, or the electron control layer may each independently be in a range of about 2 nm (20 Å) to about 100 nm (1,000 Å), and a thickness of the electron transport layer may be in a range of about 10 nm (100 Å) to about 100 nm (1,000 Å), for example. For example, the thickness of the buffer layer, the hole blocking layer, or the electron control layer may each independently be in a range of about 3 nm (30 Å) to about 30 nm (300 Å). For example, the thickness of the electron transport layer may be in a range of about 15 nm (150 Å) to about 50 nm (500 Å). When the thickness of the buffer layer, the hole blocking layer, the electron control layer, the electron transport layer, and/or the electron transport region are within these ranges, satisfactory electron transporting characteristics may be obtained without a substantial increase in driving voltage.

The electron transport region (for example, an electron transport layer in the electron transport region) may further include, in addition to the materials described above, a metal-containing material.

The metal-containing material may include an alkali metal complex, an alkaline earth metal complex, or any combination thereof. A metal ion of an alkali metal complex may be a Li ion, a Na ion, a K ion, a Rb ion, or a Cs ion, and a metal ion of an alkaline earth metal complex may be a Be ion, a Mg ion, a Ca ion, a Sr ion, or a Ba ion.

A ligand coordinated with the metal ion of the alkali metal complex or with the metal ion of the alkaline earth-metal complex may each independently include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a lithium (Li) complex. The Li complex may include, for example, Compound ET-D1 (LiQ) or Compound ET-D2:

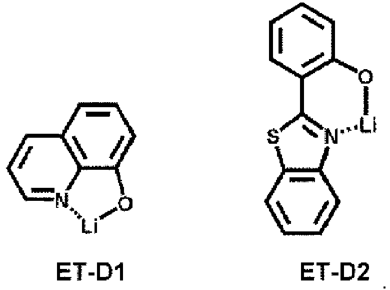

The electron transport region may include an electron injection layer that facilitates the injection of electrons from the second electrode 150. The electron injection layer may directly contact the second electrode 150.

The electron injection layer may have a structure consisting of a layer consisting of a single material, a structure consisting of a layer including different materials, or a structure including multiple layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may be oxides, halides (for example, fluorides, chlorides, bromides, or iodides), or tellurides of the alkali metal, the alkaline earth metal, and the rare earth metal, or any combination thereof.

The alkali metal-containing compound may include: alkali metal oxides, such as Li₂O, Cs₂O, or K₂O; alkali metal halides, such as LiF, NaF, CsF, KF, Lil, Nal, CsI, or Kl; or any combination thereof. The alkaline earth metal-containing compound may include an alkaline earth metal oxide, such as BaO, SrO, CaO, BaₓSr₁₋ₓO (wherein x is a real number satisfying the condition of 0<x<1), BaₓCa₁₋ₓO (wherein x is a real number satisfying the condition of 0<x<1), and/or the like. The rare earth metal-containing compound may include YbF₃, ScF₃, Sc₂O₃, Y₂O₃, Ce₂O₃, GdF₃, TbF₃, YbI₃, SCI₃, TbI₃, or any combination thereof. In embodiments, the rare earth metal-containing compound may include a lanthanide metal telluride. Examples of the lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include one of an alkali metal ion, an alkaline earth metal ion, and a rare earth metal ion, and a ligand bonded to the metal ion (for example, a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenyl benzimidazole, a hydroxyphenylbenzothiazole, a bipyridine, a phenanthroline, a cyclopentadiene, or any combination thereof).

The electron injection layer may include (e.g., consist of) an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In embodiments, the electron injection layer may further include an organic material (for example, a compound represented by Formula 601).

In embodiments, the electron injection layer may include (e.g., consist of) an alkali metal-containing compound (for example, an alkali metal halide); or the electron injection layer may include (e.g., consist of) an alkali metal-containing compound (for example, an alkali metal halide), and an alkali metal, an alkaline earth metal, a rare earth metal, or any combination thereof. For example, the electron injection layer may be a KI:Yb co-deposited layer, an Rbl:Yb co-deposited layer, a LiF:Yb co-deposited layer, and/or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 0.1 nm (1 Å) to about 10 nm (100 Å). For example, the thickness of the electron injection layer may be in a range of about 0.3 nm (3 Å) to about 9 nm (90 Å). When the thickness of the electron injection layer is within the ranges described above, satisfactory electron injection characteristics may be obtained without a substantial increase in driving voltage.

### Second electrode 150

The second electrode 150 may be disposed on the interlayer 130. The second electrode 150 may be a cathode, which is an electron injection electrode. A material for forming the second electrode 150 may be a material with a low work function, such as a metal, an alloy, an electrically conductive compound, or any combination thereof.

The second electrode 150 may include lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layered structure or a multilayered structure.

### Capping layer

The light-emitting device 10 may include a first capping layer outside the first electrode 110, and/or a second capping layer outside the second electrode 150. For example, the light-emitting device 10 may have a structure in which the first capping layer, the first electrode 110, the interlayer 130, and the second electrode 150 are stacked in this stated order, a structure in which the first electrode 110, the interlayer 130, the second electrode 150, and the second capping layer are stacked in this stated order, or a structure in which the first capping layer, the first electrode 110, the interlayer 130, the second electrode 150, and the second capping layer are stacked in this stated order.

Light generated by an emission layer in the interlayer 130 of the light-emitting device 10 may be extracted to the outside through the first electrode 110, which may be a semi-transmissive electrode or a transmissive electrode, and through the first capping layer. Light generated by an emission layer in the interlayer 130 of the light-emitting device 10 may be extracted to the outside through the second electrode 150, which may be a semi-transmissive electrode or a transmissive electrode, and through the second capping layer.

The first capping layer and the second capping layer may each increase external emission efficiency according to the principle of constructive interference. Accordingly, the light extraction efficiency of the light-emitting device 10 is increased, so that the luminescence efficiency of the light-emitting device 10 may be improved.

The first capping layer and the second capping layer may each include a material having a refractive index equal to or greater than about 1.6 (with respect to a wavelength of about 589 nm).

The first capping layer and the second capping layer may each independently be an organic capping layer including an organic material, an inorganic capping layer including an inorganic material, or an organic-inorganic composite capping layer including an organic material and an inorganic material.

At least one selected from the first capping layer and the second capping layer may each independently include carbocyclic compounds, heterocyclic compounds, amine group-containing compounds, porphine derivatives, phthalocyanine derivatives, a naphthalocyanine derivatives, alkali metal complexes, alkaline earth metal complexes, or any combination thereof. In an embodiment, the carbocyclic compound, the heterocyclic compound, and the amine group-containing compound may be optionally substituted with a substituent including O, N, S, Se, Si, F, Cl, Br, I, or any combination thereof.

In embodiments, at least one selected from the first capping layer and the second capping layer may each independently include an amine group-containing compound.

For example, at least one selected from the first capping layer and the second capping layer may each independently include a compound represented by Formula 201, a compound represented by Formula 202, or any combination thereof.

In embodiments, at least one of the first capping layer and the second capping layer may each independently include at least one selected from Compounds HT28 to HT33, at least one selected from Compounds CP1 to CP6, β-NPB, and/or any combination thereof:

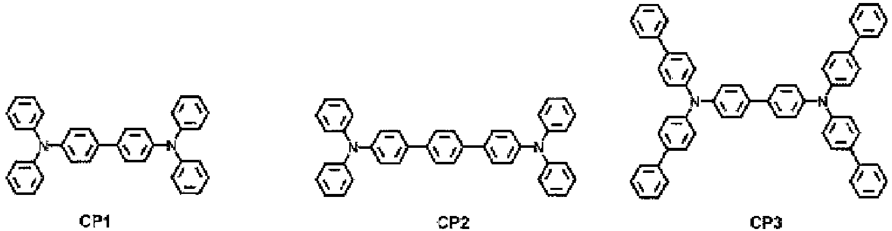

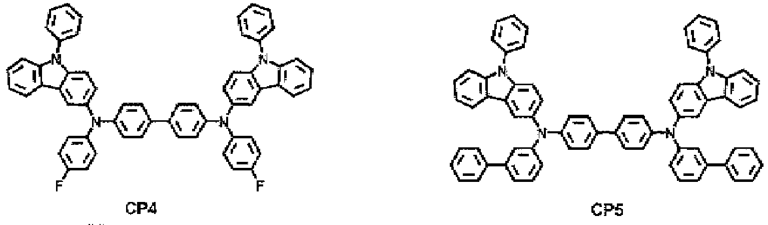

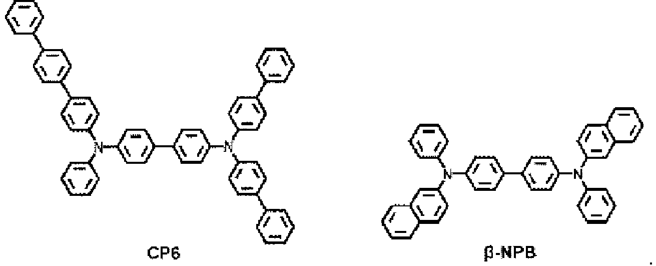

### Film

The amine-containing compound represented by Formula 1 may be included in one or more suitable films. Accordingly, another embodiment provides a film that includes the amine-containing compound represented by Formula 1. The film may be, for example, an optical member (or a light control refers to) (for example, a color filter, a color conversion member, a capping layer, a light extraction efficiency enhancement layer, a selective light absorbing layer, a polarizing layer, a quantum dot-containing layer, or like), a light-blocking member (for example, a light reflective layer, a light absorbing layer, and/or the like), or a protective member (for example, an insulating layer, a dielectric layer, and/or the like).

### Electronic apparatus

The light-emitting device may be included in one or more suitable electronic apparatuses. In an embodiment, an electronic apparatus including the light-emitting device may be a light-emitting apparatus, an authentication apparatus, and/or the like.

The electronic apparatus (for example, a light-emitting apparatus) may further include, in addition to the light-emitting device, a color filter, a color conversion layer, or a color filter and a color conversion layer. The color filter and/or the color conversion layer may be located in at least one direction in which light emitted from the light-emitting device travels. In an embodiment, the light emitted from the light-emitting device may be blue light or white light. The light-emitting device may be the same as described herein. In embodiments, the color conversion layer may include a quantum dot. The quantum dot may be, for example, a quantum dot as described herein.

The electronic apparatus may include a first substrate. The first substrate may include subpixels, the color filter may include color filter areas respectively corresponding to the subpixels, and the color conversion layer may include color conversion areas respectively corresponding to the subpixels.

A pixel-defining film may be located between the subpixels to define each subpixel.

The color filter may further include color filter areas and light-shielding patterns located between the color filter areas, and the color conversion layer may further include color conversion areas and light-shielding patterns located between the color conversion areas.

The color filter areas (or the color conversion areas) may include a first area emitting first color light, a second area emitting second color light, and a third area emitting third color light. The first color light, the second color light, and/or the third color light may have different maximum emission wavelengths from one another. For example, the first color light may be red light, the second color light may be green light, and the third color light may be blue light. In an embodiment, the color filter areas (or the color conversion areas) may include quantum dots. For example, the first area may include a red quantum dot, the second area may include a green quantum dot, and the third area may not include (e.g., may exclude) a quantum dot. The quantum dot may be the same as described herein. The first area, the second area, and/or the third area may each include a scatterer.

In embodiments, the light-emitting device may be to emit first light, the first area may be to absorb the first light to emit first-first color light, the second area may be to absorb the first light to emit second-first color light, and the third area may be to absorb the first light to emit third-first color light. The first-first color light, the second-first color light, and the third-first color light may have different maximum emission wavelengths from one another. For example, the first light may be blue light, the first-first color light may be red light, the second-first color light may be green light, and the third-first color light may be blue light.

The electronic apparatus may further include a thin-film transistor, in addition to the light-emitting device as described herein. The thin-film transistor may include a source electrode, a drain electrode, and an active layer, wherein one of the source electrode and the drain electrode may be electrically connected to one of the first electrode and the second electrode of the light-emitting device.

The thin-film transistor may further include a gate electrode, a gate insulating film, and/or the like.

The active layer may include crystalline silicon, amorphous silicon, an organic semiconductor, an oxide semiconductor, and/or the like.

The electronic apparatus may further include a sealing portion for sealing the light-emitting device. The sealing portion may be located between the color filter and/or the color conversion layer, and the light-emitting device. The sealing portion may allow light from the light-emitting device to be extracted to the outside, and may concurrently (e.g., simultaneously) prevent or reduce ambient air and moisture from penetrating into the light-emitting device. The sealing portion may be a sealing substrate including a transparent glass substrate or a plastic substrate. The sealing portion may be a thin-film encapsulation layer including an organic layer and/or an inorganic layer. When the sealing portion is a thin-film encapsulation layer, the electronic apparatus may be flexible.

Various functional layers may be further included on the sealing portion, in addition to the color filter and/or the color conversion layer, according to the utilize of the electronic apparatus. Examples of functional layers may include a touch screen layer, a polarizing layer, and/or the like. The touch screen layer may be a pressure-sensitive touch screen layer, a capacitive touch screen layer, or an infrared touch screen layer. The authentication apparatus may be, for example, a biometric authentication apparatus that authenticates an individual by utilizing biometric information of a living body (for example, fingertips, pupils, etc.).

The authentication apparatus may further include, in addition to the light-emitting device as described above, a biometric information collector.

The electronic apparatus may be applied to one or more suitable displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, one or more suitable measuring instruments, meters (for example, meters for a vehicle, an aircraft, and a vessel), projectors, and/or the like.

### Electronic device

The light-emitting device may be included in one or more suitable electronic devices.

In embodiments, the electronic device including the light-emitting device may be a flat panel display, a curved display, a computer monitor, a medical monitor, a television (TV), a billboard, an indoor light, an outdoor light, a signal light, a head-up display, a fully transparent display, a partially transparent display, a flexible display, a rollable display, a foldable display, a stretchable display, a laser printer, a telephone, a portable phone, a tablet personal computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

Because the light-emitting device has excellent or suitable luminescence efficiency and long lifespan, etc., the electronic device including the light-emitting device may have characteristics of high luminance, high resolution, and low power consumption.

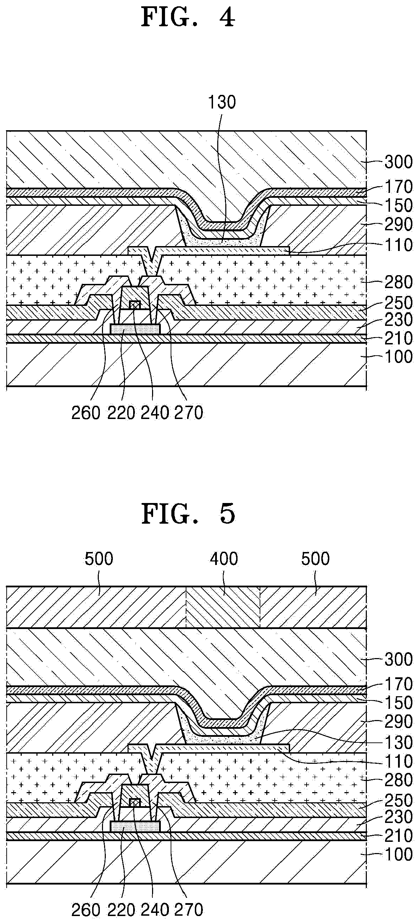

### Description of FIGS. 4 and 5

FIG. 4 is a schematic cross-sectional view of an electronic apparatus according to an embodiment.

The electronic apparatus (e.g., a light-emitting apparatus) of FIG. 4 may include a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be located on the substrate 100. The buffer layer 210 may prevent or reduce penetration of impurities through the substrate 100. The buffer layer 210 may provide a flat surface on the substrate 100.

A TFT may be located on the buffer layer 210. The TFT may include an active layer 220, a gate electrode 240, a source electrode 260, and a drain electrode 270.

The active layer 220 may include an inorganic semiconductor such as silicon or polysilicon, an organic semiconductor, or an oxide semiconductor. The active layer 220 may include a source region, a drain region, and a channel region.

A gate insulating film 230 for insulating the active layer 220 from the gate electrode 240 may be located on the active layer 220, and the gate electrode 240 may be located on the gate insulating film 230.

An interlayer insulating film 250 may be located on the gate electrode 240. The interlayer insulating film 250 may be located between the gate electrode 240 and the source electrode 260 to insulate the gate electrode 240 from the source electrode 260 and between the gate electrode 240 and the drain electrode 270 to insulate the gate electrode 240 from the drain electrode 270.

The source electrode 260 and the drain electrode 270 may be located on the interlayer insulating film 250. The interlayer insulating film 250 and the gate insulating film 230 may be formed to expose the source region and the drain region of the active layer 220, and the source electrode 260 and the drain electrode 270 may respectively contact the exposed portions of the source region and the drain region of the active layer 220.

The TFT is electrically connected to a light-emitting device to drive the light-emitting device, and is covered and protected by a passivation layer 280. The passivation layer 280 may include an inorganic insulating film, an organic insulating film, or any combination thereof. A light-emitting device is provided on the passivation layer 280. The light-emitting device may include a first electrode 110, an interlayer 130, and a second electrode 150.

The first electrode 110 may be located on the passivation layer 280. The passivation layer 280 may not completely cover the drain electrode 270. The passivation layer 280 may expose a portion of the drain electrode 270. The first electrode 110 may be electrically connected to the exposed portion of the drain electrode 270.

A pixel defining layer 290 including an insulating material may be located on the first electrode 110. The pixel defining layer 290 may expose a portion of the first electrode 110. The interlayer 130 may be formed on the exposed portion. The pixel defining layer 290 may be a polyimide or polyacrylic organic film. Although not shown in FIG. 4, at least some layers of the interlayer 130 may extend beyond the upper portion of the pixel defining layer 290 to be provided in the form of a common layer.

The second electrode 150 may be located on the interlayer 130, and a capping layer 170 may be further included on the second electrode 150. The capping layer 170 may be formed to cover the second electrode 150.

The encapsulation portion 300 may be located on the capping layer 170. The encapsulation portion 300 may be located on a light-emitting device to protect the light-emitting device from moisture and/or oxygen. The encapsulation portion 300 may include: an inorganic film including silicon nitride (SiNx), silicon oxide (SiOx), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, and/or the like), an epoxy-based resin (for example, aliphatic glycidyl ether (AGE), and/or the like), or any combination thereof; or any combination of the inorganic films and the organic films.

FIG. 5 is a schematic cross-sectional view of an electronic apparatus according to another embodiment.

The electronic apparatus (e.g., a light-emitting apparatus) of FIG. 5 may differ from the electronic apparatus of FIG. 4, at least in that a light-shielding pattern 500 and a functional region 400 are further included on the encapsulation portion 300. The functional region 400 may be a color filter area, a color conversion area, or a combination of the color filter area and the color conversion area. In embodiments, the light-emitting device included in the electronic apparatus of FIG. 5 may be a tandem light-emitting device.

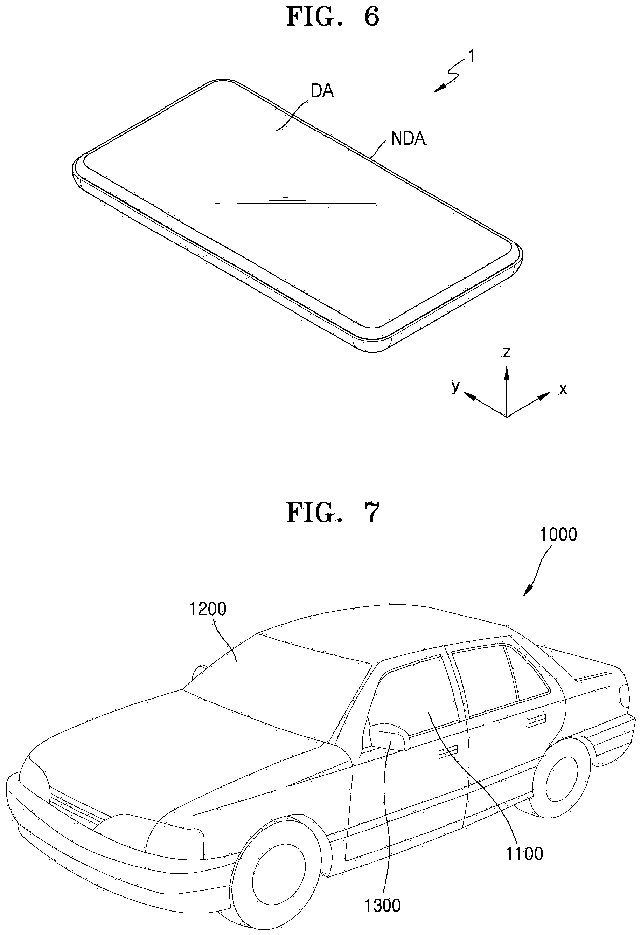

### Description of FIG. 6

FIG. 6 is a schematic perspective view of an electronic device 1 including a light-emitting device according to an embodiment.

The electronic device 1, which may be a device displaying a video or a still image, may be not only a portable electronic device (or a portion thereof) such as a mobile phone, a smartphone, a tablet personal computer (PC), a mobile telecommunication terminal, an electronic digital assistant, an electronic book, a portable multimedia player (PMP) or a navigation device, an ultra mobile PC (UMPC), but also one or more suitable products such as a television, a laptop computer, a monitor, an advertisement sign, or an internet of things (IOT).

The electronic device 1 may be a smart watch, a watch phone, or a wearable device such as a glasses-type or kind display or a head mounted display (HMD), or a portion thereof. However, embodiments are not limited thereto.

For example, the electronic device 1 may be a dashboard of a vehicle, a center information display (CID) arranged on a center fascia or dashboard of a vehicle, a room mirror display that replaces the side mirror of a vehicle, a display arranged for entertainment in the back seat of a vehicle or arranged on the back of the front seat, a head-up display (HUD) installed on the front of a vehicle or projected onto the front window glass, or a computer generated hologram augmented reality head up display (CGH AR HUD). FIG. 6 shows an embodiment in which the electronic device 1 is a smartphone, for convenience of explanation.

The electronic device 1 may include a display area DA and a non-display area NDA outside the display area DA. The display device may implement an image through a two-dimensional array of pixels in the display area DA.

The non-display area NDA, which is an area that does not display an image, may surround the display area DA. A driver for providing electrical signals or power to display elements located in the display area DA may be arranged in the non-display area NDA. A pad that is an area to which an electronic device or a printed circuit board may be electrically connected may be arranged in the non-display area NDA.

The electronic device 1 may have different lengths in an x-axis direction and a y-axis direction. For example, as shown in FIG. 6, a length in the x-axis direction may be less than a length in the y-axis direction. In another embodiment, a length in the x-axis direction and a length of the y-axis direction may be the same. In still another embodiment, a length in the x-axis direction may be greater than a length in the y-axis direction.

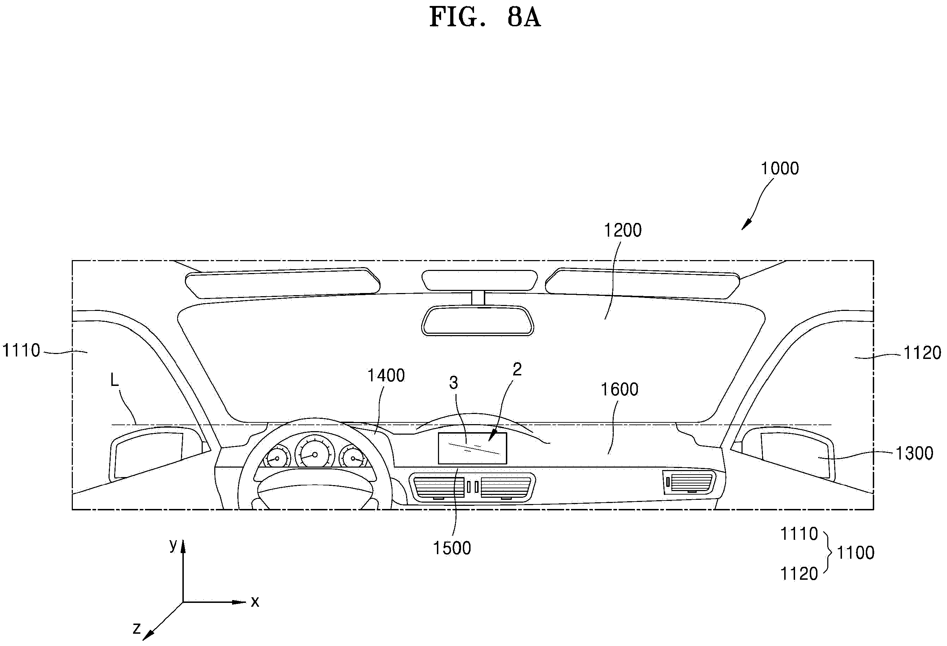

### Description of FIGS. 7 and 8A to 8C

FIG. 7 is a schematic perspective view of the exterior of a vehicle 1000 as an electronic device including a light-emitting device according to an embodiment.

FIGS. 8A to 8C are each a schematic diagram of the interior of a vehicle 1000 according to embodiments.

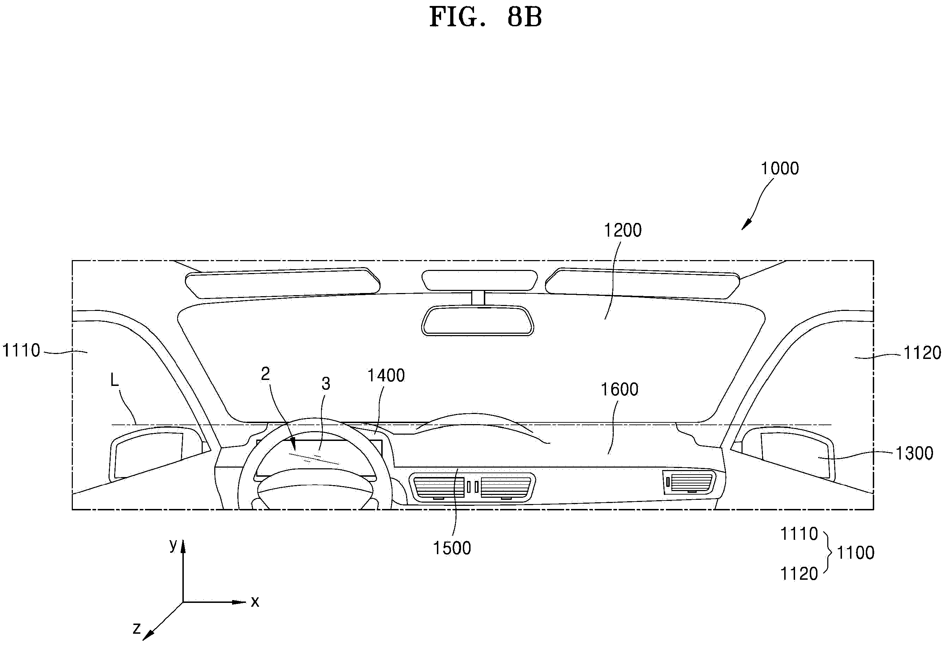

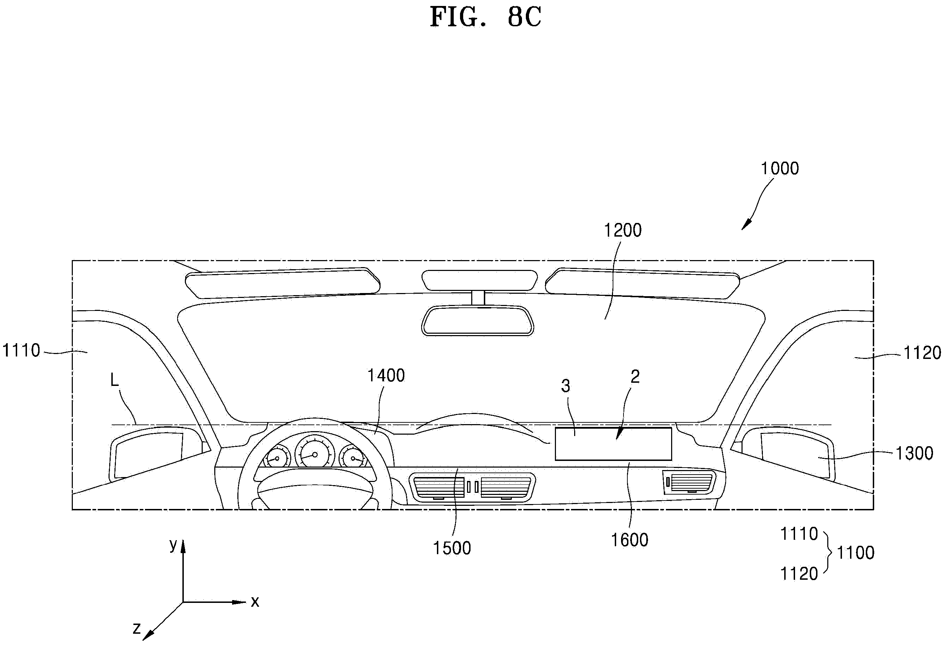

Referring to FIGS. 7, 8A, 8B, and 8C, the vehicle 1000 may refer to one or more suitable devices that move a subject to be transported, such as a person, an object, or an animal, from a departure point to a destination. Examples of the vehicle 1000 may include a vehicle that drives on a road or track, a ship that moves over a sea or river, and an airplane that flies in the air utilizing the action of air.

The vehicle 1000 may travel on a road or track. The vehicle 1000 may move in a given direction according to the rotation of at least one wheel. Examples of the vehicle 1000 may include a three-wheeled or four-wheeled vehicle, a construction machine, a two-wheeled vehicle, an engine, a bicycle, and a train that runs on a track.

The vehicle 1000 may include a body having an interior and an exterior, and a chassis that is a portion excluding the body and includes mechanical devices installed therein that are necessary for driving the vehicle. The exterior of the body may include a front panel, a bonnet, a roof panel, a rear panel, a trunk, at least one door, and a pillar provided at a boundary between doors. The chassis of the vehicle 1000 may include a power generation device, a power transfer device, a driving device, a steering device, a braking device, a suspension, a transmission, a fuel device, and front, rear, left, and right wheels.

The vehicle 1000 may include a side window glass 1100, a front window glass 1200, a side mirror 1300, a cluster 1400, a center fascia 1500, a passenger seat dashboard 1600, and a display device 2.

The side window glass 1100 and the front windows glass 1200 may be partitioned by a pillar arranged between the side window glass 1100 and the front window glass 1200.

The side window glass 1100 may be installed on the side of the vehicle 1000. In an embodiment, the side window glass 1100 may be installed in the door of the vehicle 1000. The side window glass 1100 may be provided as multiple side window glasses that may face each other. In an embodiment, the side window glass 1100 may include a first side window glass 1110 and a second side window glass 1120. In an embodiment, the first side window glass 1110 may be arranged adjacent to the cluster 1400, and the second side window glass 1120 may be arranged adjacent to the passenger seat dashboard 1600.

In an embodiment, the side window glasses 1100 may be spaced apart from each other in an x direction or in a -x direction. For example, the first side window glass 1110 and the second side window glass 1120 may be spaced apart from each other in the x direction or in the -x direction. A virtual straight line L connecting the side window glasses 1100 may extend in the x direction or in the -x direction. For example, a virtual straight line L connecting the first side window glass 1110 and the second side window glass 1120 may extend in the x direction or in the -x direction.

The front window glass 1200 may be installed on the front of the vehicle 1000. The front window glass 1200 may be located between the side window glasses 1100 facing each other.

The side mirror 1300 may provide a rear view of the vehicle 1000. The side mirror 1300 may be installed on the exterior of the body. In an embodiment, multiple side mirrors 1300 may be provided. One of the side mirrors 1300 may be located outside of the first side window glass 1110. Another one of the side mirrors 1300 may be located outside of the second side window glass 1120.

The cluster 1400 may be located at the front of a steering wheel. A tachometer, a speedometer, a coolant thermometer, a fuel gauge, a turn signal indicator, a high beam indicator, a warning light, a seatbelt warning light, an odometer, a driving record system, an automatic shifting selection lever indicator, a door open warning light, an engine oil warning light, and/or fuel shortage warning light may be located on the cluster 1400.

The center fascia 1500 may include a control panel having buttons for adjusting an audio device, an air conditioning device, and a seat heater. The center fascia 1500 may be located on a side of the cluster 1400.

The passenger seat dashboard 1600 may be spaced apart from the cluster 1400 with the center fascia 1500 therebetween. In an embodiment, the cluster 1400 may be arranged to correspond to the driver's seat, and the passenger seat dashboard 1600 may be disposed to correspond to the passenger seat. In an embodiment, the cluster 1400 may be adjacent to the first side window glass 1110, and the passenger seat dashboard 1600 may be adjacent to the second side window glass 1120.

In an embodiment, the display device 2 may include a display panel 3, and the display panel 3 may display an image. The display device 2 may be located inside the vehicle 1000. In an embodiment, the display device 2 may be located between the side window glasses 1100 facing each other. The display device 2 may be located in at least one of the cluster 1400, the center fascia 1500, and the passenger seat dashboard 1600.

The display device 2 may include an organic light-emitting display, an inorganic light-emitting display, a quantum dot display, and/or the like. Hereinafter, an organic light-emitting display including the light-emitting device according to an embodiment is utilized as an example of the display device 2. However, one or more suitable display devices as described herein may be utilized as embodiments.

Referring to FIG. 8A, the display device 2 may be arranged in the center fascia 1500. In an embodiment, the display device 2 may display navigation information. In an embodiment, the display device 2 may display information about audio settings, video settings, or vehicle settings.

Referring to FIG. 8B, the display device 2 may be arranged in the cluster 1400. The cluster 1400 may show driving information and/or the like through the display device 2. Thus, the cluster 1400 may be digitally implement driving information. The digital cluster 1400 may digitally display vehicle information and driving information as images. For example, the needle and gauge of a tachometer and one or more suitable warning lights may be displayed by a digital signal.

Referring to FIG. 8C, the display device 2 may be arranged in the passenger seat dashboard 1600. The display device 2 may be embedded in the passenger seat dashboard 1600 or may be located on the passenger seat dashboard 1600. In an embodiment, the display device 2 arranged in the passenger seat dashboard 1600 may display an image regarding information displayed on the cluster 1400 and/or information displayed on the center fascia 1500. In another embodiment, the display apparatus 2 arranged in the passenger seat dashboard 1600 may display information that is different from the information displayed on the cluster 1400 and/or different from the information displayed on the center fascia 1500.

### Manufacturing method

Respective layers included in the hole transport region, the emission layer, and respective layers included in the electron transport region may be formed in a certain region by utilizing one or more suitable methods selected from vacuum deposition, spin coating, casting, Langmuir-Blodgett (LB) deposition, ink-jet printing, laser-printing, and laser-induced thermal imaging.

When layers constituting the hole transport region, an emission layer, and layers constituting the electron transport region are formed by vacuum deposition, the deposition may be performed at a deposition temperature of about 100 °C to about 500 °C, a vacuum degree of about 1,333 x 10⁻⁶ Pa (about 10⁻⁸ torr) to about 0,1333 Pa (about 10⁻³ torr), and a deposition speed of about 0.001 nm/sec (0.01 Å/sec) to about 10 nm/sec (100 Å/sec), depending on a material to be included in a layer to be formed and the structure of a layer to be formed.

### Definitions of terms

The term "C₃-C₆₀ carbocyclic group" as utilized herein may be a cyclic group consisting of carbon atoms as the only ring-forming atoms and having 3 to 60 carbon atoms. The term "C₁-C₆₀ heterocyclic group" as utilized herein may be a cyclic group having 1 to 60 carbon atoms and further including, in addition to a carbon atom, at least one heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. For example, a C₁-C₆₀ heterocyclic group may have 3 to 61 ring-forming atoms.

The term "cyclic group" as utilized herein may be a C₃-C₆₀ carbocyclic group or a C₁-C₆₀ heterocyclic group.

The term "π electron-rich C₃-C₆₀ cyclic group" as utilized herein may be a cyclic group having 3 to 60 carbon atoms and may not include (e.g., may exclude) *-N=*' as a ring-forming moiety. The term "π electron-deficient nitrogen-containing C₁-C₆₀ cyclic group" as utilized herein may be a heterocyclic group having 1 to 60 carbon atoms and may include *-N=*' as a ring-forming moiety.

In embodiments,
the C₃-C₆₀ carbocyclic group may be a T1 group or a group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be a T2 group, a group in which at least two T2 groups are condensed with each other, or a group in which at least one T2 group and at least one T1 group are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like),
the π electron-rich C₃-C₆₀ cyclic group may be a T1 group, a group in which at least two T1 groups are condensed with each other, a T3 group, a group in which at least two T3 groups are condensed with each other, or a group in which at least one T3 group and at least one T1 group are condensed with each other (for example, a C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, and/or the like), and
the π electron-deficient nitrogen-containing C₁-C₆₀ cyclic group may be a T4 group, a group in which at least two T4 groups are condensed with each other, a group in which at least one T4 group and at least one T1 group are condensed with each other, a group in which at least one T4 group and at least one T3 group are condensed with each other, or a group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and/or the like).

The T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group.

The T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group.

The T3 group may be a furan group, a thiophene group, a 1H-pyrrole group, a silole group, or a borole group.

The T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group", "C₃-C₆₀ carbocyclic group", "C₁-C₆₀ heterocyclic group", "π electron-rich C₃-C₆₀ cyclic group", or "π electron-deficient nitrogen-containing C₁-C₆₀ cyclic group" as utilized herein may each be a group condensed to any cyclic group, a monovalent group, or a polyvalent group (for example, a divalent group, a trivalent group, a tetravalent group, and/or the like) according to the structure of a formula for which the corresponding term is utilized. For example, a "benzene group" may be a benzo group, a phenyl group, a phenylene group, and/or the like, which may be readily understand by one of ordinary skill in the art according to the structure of a formula including the "benzene group."

Examples of a monovalent C₃-C₆₀ carbocyclic group and a monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Examples of a divalent C₃-C₆₀ carbocyclic group and a divalent C₁-C₆₀ heterocyclic group include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as utilized herein may be a linear or branched aliphatic hydrocarbon monovalent group having 1 to 60 carbon atoms. Examples of a C₁-C₆₀ alkyl group may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group.

The term "C₁-C₆₀ alkylene group" as utilized herein may be a divalent group having a same structure as the C₁-C₆₀ alkyl group.

The term "C₂-C₆₀ alkenyl group" as utilized herein may be a monovalent hydrocarbon group having at least one carbon-carbon double bond in the middle or at a terminus of a C₂-C₆₀ alkyl group. Examples of a C₂-C₆₀ alkenyl group may include an ethenyl group, a propenyl group, and a butenyl group.

The term "C₂-C₆₀ alkenylene group" as utilized herein may be a divalent group having a same structure as the C₂-C₆₀ alkenyl group.

The term "C₂-C₆₀ alkynyl group" as utilized herein may be a monovalent hydrocarbon group having at least one carbon-carbon triple bond in the middle or at a terminus of a C₂-C₆₀ alkyl group. Examples of a C₂-C₆₀ alkynyl group may include an ethynyl group and a propynyl group.

The term "C₂-C₆₀ alkynylene group" as utilized herein may be a divalent group having a same structure as the C₂-C₆₀ alkynyl group.

The term "C₁-C₆₀ alkoxy group" as utilized herein may be a monovalent group represented by -O(A₁₀₁) (wherein A₁₀₁ may be a C₁-C₆₀ alkyl group). Examples of a C₁-C₆₀ alkoxy group may include a methoxy group, an ethoxy group, and an isopropyloxy group.

The term "C₅-C₁₀ cycloalkyl group" as utilized herein may be a monovalent saturated hydrocarbon cyclic group having 3 to 10 carbon atoms. Examples of a C₃-C₁₀ cycloalkyl group may include a cyclopropyl group, a cyclobutyl group, a cyclopentyl, cyclohexyl group, a cycloheptyl group, a cyclooctyl group, an adamantanyl group, a norbornanyl group (or a bicyclo[2.2.1]heptyl group), a bicyclo[1.1.1]pentyl group, a bicyclo[2.1.1]hexyl group, and a bicyclo[2.2.2]octyl group.

The term "C₃-C₁₀ cycloalkylene group" as utilized herein may be a divalent group having a same structure as the C₃-C₁₀ cycloalkyl group.

The term "C₁-C₁₀ heterocycloalkyl group" as utilized herein may be a monovalent cyclic group having 1 to 10 carbon atoms that further includes, in addition to a carbon atom, at least one heteroatom as a ring-forming atom. Examples of a C₁-C₁₀ heterocycloalkyl group may include a 1,2,3,4-oxatriazolidinyl group, a tetrahydrofuranyl group, and a tetrahydrothiophenyl group.

The term "C₁-C₁₀ heterocycloalkylene group" as utilized herein may be a divalent group having a same structure as the C₁-C₁₀ heterocycloalkyl group.

The term "C₃-C₁₀ cycloalkenyl group" as utilized herein may be a monovalent cyclic group that has 3 to 10 carbon atoms and at least one carbon-carbon double bond in its ring, and is not aromatic. Examples of a C₃-C₁₀ cycloalkenyl group may include a cyclopentenyl group, a cyclohexenyl group, and a cycloheptenyl group.

The term "C₃-C₁₀ cycloalkenylene group" as utilized herein may be a divalent group having a same structure as the C₃-C₁₀ cycloalkenyl group.

The term "C₁-C₁₀ heterocycloalkenyl group" as utilized herein may be a monovalent cyclic group having 1 to 10 carbon atoms, that further includes, in addition to a carbon atom, at least one heteroatom, as a ring-forming atom, and having at least one carbon-carbon double bond in the cyclic structure thereof. Examples of a C₁-C₁₀ heterocycloalkenyl group may include a 4,5-dihydro-1,2,3,4-oxatriazolyl group, a 2,3-dihydrofuranyl group, and a 2,3-dihydrothiophenyl group.

The term "C₁-C₁₀ heterocycloalkenylene group" as utilized herein may be a divalent group having a same structure as the C₁-C₁₀ heterocycloalkenyl group.

The term "C₆-C₆₀ aryl group" as utilized herein may be a monovalent group having a carbocyclic aromatic system of 6 to 60 carbon atoms. Examples of a C₆-C₆₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group.

The term "C₆-C₆₀ arylene group" as utilized herein may be a divalent group having a carbocyclic aromatic system of 6 to 60 carbon atoms.

When the C₆-C₆₀ aryl group and the C₆-C₆₀ arylene group each include two or more rings, the respective rings may be condensed with each other.

The term "C₁-C₆₀ heteroaryl group" as utilized herein may be a monovalent group having a heterocyclic aromatic system of 1 to 60 carbon atoms that further includes, in addition to a carbon atom, at least one heteroatom as a ring-forming atom. Examples of a C₁-C₆₀ heteroaryl group may include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group.

The term "C₁-C₆₀ heteroarylene group" as utilized herein may be a divalent group having a heterocyclic aromatic system of 1 to 60 carbon atoms that further includes, in addition to a carbon atom, at least one heteroatom as a ring-forming atom.

When the C₁-C₆₀ heteroaryl group and the C₁-C₆₀ heteroarylene group each include two or more rings, the respective rings may be condensed with each other.

The term "monovalent non-aromatic condensed polycyclic group" as utilized herein may be a monovalent group (for example, having 8 to 60 carbon atoms) having two or more rings condensed to each other, only carbon atoms as ring-forming atoms, and no aromaticity in its entire molecular structure. Examples of a monovalent non-aromatic condensed polycyclic group may include an indenyl group, a fluorenyl group, a spiro-bifluorenyl group, a benzofluorenyl group, an indenophenanthrenyl group, and an indenoanthracenyl group.

The term "divalent non-aromatic condensed polycyclic group" as utilized herein may be a divalent group having a same structure as the monovalent non-aromatic condensed polycyclic group described above.

The term "monovalent non-aromatic condensed heteropolycyclic group" as utilized herein may be a monovalent group (for example, having 1 to 60 carbon atoms) having two or more rings condensed to each other, further including, in addition to carbon atoms, at least one heteroatom, as ring-forming atoms, and having non-aromaticity in its entire molecular structure. Examples of a monovalent non-aromatic condensed heteropolycyclic group may include a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, and a benzothienodibenzothiophenyl group.

The term "divalent non-aromatic condensed heteropolycyclic group" as utilized herein may be a divalent group having a same structure as the monovalent non-aromatic condensed heteropolycyclic group described above.

The term "C₆-C₆₀ aryloxy group" as utilized herein may be a group represented by -O(A₁₀₂) (wherein A₁₀₂ may be a C₆-C₆₀ aryl group).

The term "C₆-C₆₀ arylthio group" as utilized herein may be a group represented by -S(A₁₀₃) (wherein A₁₀₃ may be a C₆-C₆₀ aryl group).

The term "C₇-C₆₀ arylalkyl group" as utilized herein may be a group represented by -(A₁₀₄)(A₁₀₅) (wherein A₁₀₄ may be a C₁-C₅₄ alkylene group and A₁₀₅ may be a C₆-C₅₉ aryl group).

The term "C₂-C₆₀ heteroarylalkyl group" as utilized herein may be a group represented by -(A₁₀₆)(A₁₀₇) (wherein A₁₀₆ may be a C₁-C₅₉ alkylene group and A₁₀₇ may be a C₁-C₅₉ heteroaryl group).

In the specification, the group "R₁₀ₐ" may be:
deuterium (-D), -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), - C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂).
In the specification, Q₁₁ to Q₁₃, Q₂₁ to Q₂₃ and Q₃₁ to Q₃₃ may each independently be:
   hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, or a C₁-C₆₀ alkoxy group; or
   a C₃-C₆₀ carbocyclic group, a C₁-C₆₀ heterocyclic group, a C₇-C₆₀ arylalkyl group, or a C₂-C₆₀ heteroarylalkyl group, each unsubstituted or substituted with deuterium, -F, a cyano group, a C₁-C₆₀ alkyl group, a C₁-C₆₀ alkoxy group, a phenyl group, a biphenyl group, or any combination thereof.

The term "heteroatom" as utilized herein may be any atom other than a carbon atom or a hydrogen atom. Examples of a heteroatom may include O, S, N, P, Si, B, Ge, Se, and any combinations thereof.

The term "third-row transition metal" as utilized herein may include hafnium (Hf), tantalum (Ta), tungsten (W), rhenium (Re), osmium (Os), iridium (Ir), platinum (Pt), gold (Au), and/or the like.

The term "Ph" as utilized herein refers to a phenyl group, the term "Me" as utilized herein refers to a methyl group, the term "Et" as utilized herein refers to an ethyl group, the terms "tert-Bu" or "Bu^{t}" as utilized herein each refer to a tert-butyl group, and the term "OMe" as utilized herein refers to a methoxy group.

The term "biphenyl group" as utilized herein may be a "phenyl group substituted with a phenyl group." For example, the "biphenyl group" may be a substituted phenyl group having a C₆-C₆₀ aryl group as a substituent.

The term "terphenyl group" as utilized herein may be a "phenyl group substituted with a biphenyl group". For example, the "terphenyl group" may be a substituted phenyl group having, as a substituent, a C₆-C₆₀ aryl group substituted with a C₆-C₆₀ aryl group.

The symbols * and *' as utilized herein, unless defined otherwise, each refer to a binding site to a neighboring atom in a corresponding formula or moiety.

In the specification, the terms "x-axis," "y-axis," and "z-axis" are not limited to three axes in a Cartesian coordinate system, and may have a broader meaning than the aforementioned three axes in a Cartesian coordinate system. For example, the x-axis, y-axis, and z-axis may describe axes that are orthogonal to each other, or may describe axes that are in different directions that are not orthogonal to each other.

### Organic Light Emitting Diode

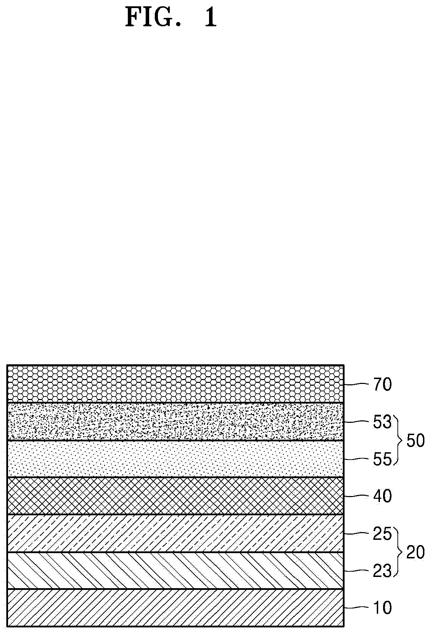

FIG. 1 is a cross-sectional view illustrating an organic light emitting diode according to an example embodiment of the present invention.

Referring to FIG. 1, an organic light emitting diode includes an anode 10, a cathode 70, a light emitting layer 40 disposed between the two electrodes, a hole conduction layer 20 disposed between the anode 10 and the light emitting layer 40, and an electron conduction layer 50 disposed between the light emitting layer 40 and the cathode 70. The hole conduction layer 20 may include a hole transport layer 25 for transporting holes and a hole injection layer 23 for facilitating injection of holes. The electron conduction layer 50 may include an electron transport layer 55 for transporting electrons and an electron injection layer 53 for facilitating injection of electrons. In some embodiments, a hole blocking layer may be disposed between the light emitting layer 40 and the electron transporting layer 55. Further, an electron blocking layer may be disposed between the light emitting layer 40 and the hole transporting layer 25. However, it is not limited thereto, and the electron transport layer 55 may serve as the hole blocking layer, or the hole transport layer 25 may serve as the electron blocking layer.

When a forward bias is applied to the organic light emitting diode, holes are injected into the light emitting layer 40 from the anode 10, and electrons are injected into the light emitting layer 40 from the cathode 70. Electrons and holes injected into the light emitting layer 40 are combined to form excitons, and light is emitted while the excitons transition to the ground state.

The light emitting layer 40 may be made of a single light emitting material, or may include a host material and a dopant material. The single light emitting material or the light emitting dopant material may be a compound represented by any one of the above-described Chemical Formulas 1 to 12 and Compounds 1 to 92, specifically, a thermally activated delayed fluorescent material. In this case, the efficiency of the organic light emitting diode can be greatly improved. The thermally activated delayed fluorescent material has a form in which an electron donating group and an electron withdrawing group are connected to benzene and the electron withdrawing group is positioned in an ortho position to the electron donating group. Thus, the difference between singlet energy and triplet energy can be reduced to less than 0.3 eV. The thermally activated delayed fluorescent material can more efficiently facilitate transition from the triplet excited state to the singlet excited state by heat (at room temperature or element operating temperature), thereby improving the quantum efficiency. In some embodiments, one or more suitable combinations of the red, green, and blue luminescent colors can be realized by the appropriate or suitable introduction of the electron donating group and the electron withdrawing group.

The host material may be selected from the group consisting of mCP (N,N-dicarbazolyl-3,5-benzene), Alq3, CBP (4,4'-N,N'-dicarbazole-biphenyl), 9,10-di(naphthalen-2-yl)anthracene, TPBI (1,3,5-tris(N-phenylbenzimidazole-2-yl)benzene), TBADN (3-tert-butyl-9,10-di(naphth-2-yl)anthracene), E3 (see the following formula), and BeBq2 (see the following formula).

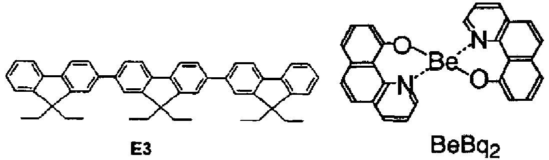

In some embodiments, the hole injection layer 23 and/or the hole transport layer 25 are layers having a HOMO level between the work function level of the anode 10 and the HOMO level of the light emitting layer 40, and has the function of enhancing the injection or transport efficiency of holes from the anode 10 to the light emitting layer 40. The electron injection layer 53 and/or the electron transport layer 55 are layers having a LUMO level between the work function level of the cathode 70 and the LUMO level of the light emitting layer 40, and has the function of enhancing the injection or transport efficiency of electrons from the cathode 70 to the light emitting layer 40.

The anode 10 may be a conductive metal oxide, a metal, a metal alloy, or a carbon material. The conductive metal oxide may be at least one selected from the group consisting of indium tin oxide (ITO), fluorine tin oxide (FTO), antimony tin oxide (ATO), SnO2, ZnO, or any combination thereof. The metal or metal alloy suitable as the anode 10 may be Au or CuI. The carbon material may be graphite, graphene, or carbon nanotubes.

The hole injecting layer 23 or the hole transporting layer 25 may include a material commonly utilized as a hole transporting material, and one layer may have a different hole transporting materials or a different hole transporting material layers. The hole transporting material may be, for example, mCP (N,N-dicarbazolyl-3,5-benzene); PEDOT:PSS (poly(3,4-ethylenedioxythiophene):polystyrenesulfonate); NPD (N,N'-di(1-naphthyl)-N,N'-diphenylbenzidine); N,N'-diphenyl-N,N'-di(3-methylphenyl)-4,4'-diaminobiphenyl (TPD); N,N'-diphenyl-N,N'-dinaphthyl-4,4'-diaminobiphenyl; N,N,N',N'-tetra-p-tolyl-4,4'-diaminobiphenyl; N,N,N',N'-tetraphenyl-4,4'-diaminobiphenyl; porphyrin compound derivatives such as copper (II) 1,10,15,20-tetraphenyl-21H,23H-porphyrin and/or the like; TAPC (1,1-bis[4-[N,N'-di(p-tolyl)amino]phenyl]cyclohexane); triarylamine derivatives such as N,N,N-tri(p-tolyl)amine and 4,4',4'-tris[N-(3-methylphenyl)-N-phenylamino] triphenylamine; carbazole derivatives such as N-phenylcarbazole and polyvinylcarbazole; phthalocyanine derivatives such as metal-free phthalocyanine and copper phthalocyanine; starburst amine derivatives; enamine stilbene derivatives; derivatives of aromatic tertiary amine and styrylamine compounds; polysilane; and/or the like. Such a hole transporting material may also serve as the electron blocking layer.

The hole blocking layer serves to prevent or reduce triplet excitons or holes from diffusing toward the cathode 70, and may be arbitrarily selected from suitable hole blocking materials. For example, oxadiazole derivatives, triazole derivatives, phenanthroline derivatives and/or the like can be utilized.

The electron transporting layer 55 may be formed of a material selected from the group consisting of TSPO1 (diphenylphosphine oxide-4-(triphenylsilyl)phenyl), tris(8-quinolinolate) aluminum (Alq3), 2,5-diarylsilole derivative (PyPySPyPy), perfluorinated compound (PF-6P), COTs (octasubstituted cyclooctatetraene), TAZ (see the following Chemical Formula), Bphen (4,7-diphenyl-1,10-phenanthroline), BCP (see the following formula), and BAIq (see the following formula).

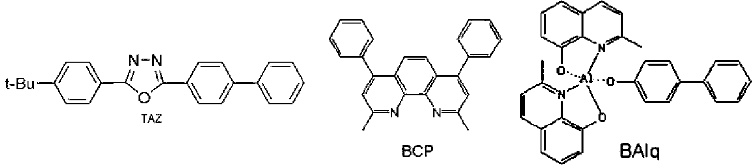

The electron injection layer 53 may be, for example, LiF, NaCl, CsF, Li2O, BaO, BaF2, or Liq (lithium quinolate).

The cathode 70 is a conductive film having a lower work function than the anode 70 and is made of a metal such as aluminum, magnesium, calcium, sodium, potassium, indium, yttrium, lithium, silver, lead, cesium, and/or the like, or a combination of two or more species among those metals.

The anode 10 and the cathode 70 may be formed utilizing a sputtering method, a vapor deposition method, or an ion beam deposition method. The hole injecting layer 23, the hole transporting layer 25, the light emitting layer 40, the hole blocking layer, the electron transporting layer 55 and the electron injecting layer 53 are, regardless of each other, formed by evaporation or coating methods such as spraying, spin coating, dipping, printing, doctor blading, or electrophoresis.

The organic light emitting diode may be disposed on a substrate, which may be disposed the anode 10 or above the cathode 70. In other words, the anode 10 may be formed on the substrate before the cathode 70, or the cathode 70 may be formed before the anode 10.

The substrate may be a light-transmissive substrate as a flat plate member, in which case the substrate may be glass; ceramics; or a polymer material such as polycarbonate (PC), polyethylene terephthalate (PET), polyethylene naphthalate (PEN), polyimide (PI), polypropylene (PP). However, the present invention is not limited to this, and the substrate may be a metal substrate capable of light reflection.

Hereinafter, example embodiments of the present invention will be described in order to facilitate understanding of the present invention. It should be understood, however, that the following examples are for the purpose of promoting understanding of the present invention and are not intended to limit the scope of the present invention.

### Examples

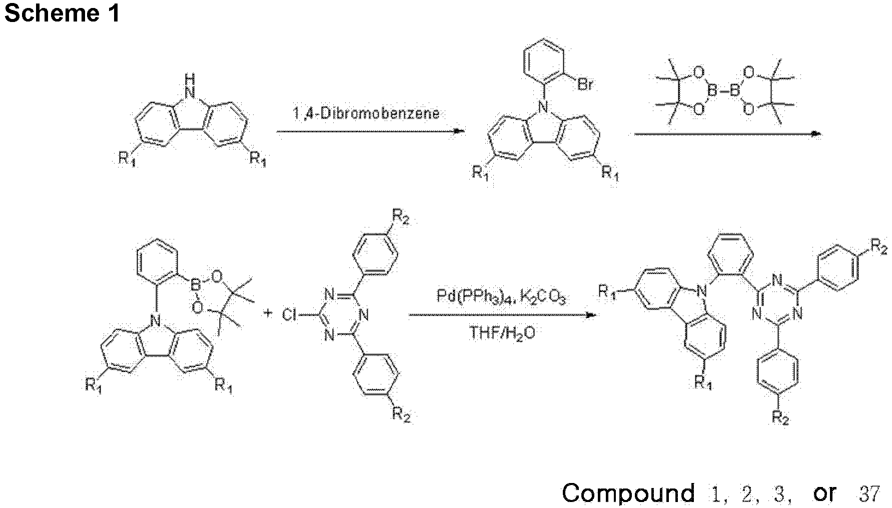

In Scheme 1, the product is Compound 1 when both of R₁ and R₂ are hydrogen, Compound 2 when R₁ is a methyl group and R₂ is hydrogen, Compound 3 when R₁ is a t-butyl group and R₂ is hydrogen, and Compound 37 when R₁ is hydrogen and R₂ is a methyl group. Compounds 1, 2, 3, and 37 are not according to the invention.

### Synthesis Example 1: Synthesis of Compound 1

Step 1 : Carbazole (10.0 g, 59.8 mmol), 1,2-dibromobenzene (21.16 g, 89.71 mmol), potassium carbonate (16.53 g, 119.61 mmol) and copper iodide (Cul) (5.69 g, 29.90 mmol) were dissolved in N,N-dimethylacetamide (250 mL) and then oxygen was removed from the solution through nitrogen bubbling. After oxygen was removed, the mixture was refluxed and stirred for 24 hours. The reaction solution was extracted with methylene chloride, and then subjected to column chromatography utilizing a methylene chloride/hexane mixed solvent as eluent to obtain 15.99 g of 9- (2-bromophenyl)carbazole. 9-(2-bromophenyl)carbazole: Mass Spec (EI) m/z 322 [(M + H)⁺]
Step 2 : 9-(2-bromophenyl)carbazole (10 g, 31.03 mmol), 4,4,4',4',5,5,5',5'-octamethyl-2,2'-bi(1,3,2-dioxaborane) (11.82 g, 46.55 mmol), potassium acetate (9.14 g, 93.11 mmol), and 1,1'-bis(diphenylphosphino) ferrocene-palladium (II) (0.76 g, 0.93 mmol) were dissolved in 1,4-dioxane (180 mL), and then oxygen in the solution was removed through nitrogen bubbling. After oxygen was removed, the mixture was refluxed and stirred for 24 hours. The reaction solution was extracted with methylene chloride and then subjected to column chromatography utilizing a methylene chloride/hexane mixed solvent as eluent to obtain 4.01 g of 9-(2-(4,4,5,5-tetramethyl-1,3,2-dioxaborolan-2-yl)phenyl) carbazole. 9-(2-(4,4,5,5-tetramethyl-1,3,2-dioxaborolan-2-yl) phenyl) carbazole : Mass Spec (EI) m/z 369 [(M + H)⁺]
Step 3 : 9-(2-(4,4,5,5-tetramethyl-1,3,2-dioxabolan-2-yl)phenyl)carbazole (3 g, 8.12 mmol), 2-chloro-4,6-diphenyl-1,3,5-triazine (2.61 g, 9.75 mmol), tetrakis (triphenylphosphine) palladium (0) (0.28 g, 0.24 mmol) were added to a mixed solvent of tetrahydrofuran (60 mL) and potassium carbonate 2M solution (20 mL), and then the mixture was refluxed for 24 hours. The reaction solution was extracted with methylene chloride, and then purified by column chromatography utilizing a methylene chloride/hexane mixed solvent as eluent, and finally 3.31 g of a pure white solid was obtained through sublimation purification.

Compound 1 : yield 86%, Mass Spec (EI) m/z 474 [(M+H)⁺]. Elemental analysis theoretical value C₃₃H₂₂N₄: C, 83.52%; H, 4.67%; N, 11.81%. Measured: C, 83.52%; H, 4.74%; N, 11.69%. ¹H **NMR** (500 MHz, CDCl₃): δ 8.57 (d, 1H), 8.03 (t, 6H), 7.85 (t, 1H), 7.78 (t, 1H), 7.73 (d, 1H), 7.48 (t, 2H), 7.34 (t, 6H), 7.24 ~ 7.19 (m, 4H).

### Evaluation Example 1

Table 1 shows the values calculated by the molecular calculation technique for Compound 1 (not according to the invention) prepared according to Synthesis Example 1, Comparative Compound A, and Comparative Compound B. Molecular calculations were performed utilizing the Gaussian 09 program through the B3LYP method according to density functional theory on a 6-31 G * basis set.

**[Table 1]**

| Sample | Structural Formula | ¹⁾B_{g} | ²⁾S₁ | ³⁾T₁ | ⁴⁾ΔE_{ST} | ⁵⁾HOMO | ⁶⁾LUMO |
|---|---|---|---|---|---|---|---|
| Compound (ortho) | 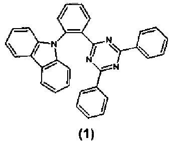 | 3.49 eV | 2.86 eV | 2.79 eV | 0.06 eV | -5.31 eV | -1.81 eV |
| Comparative Compound A (meta) | 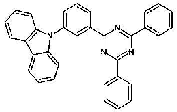 | 3.39 eV | 2.90 eV | 2.75 eV | 0.15 eV | -5.36 eV | -1.97 eV |
| Comparative Compound B (para) | 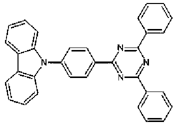 | 3.47 eV | 3.04 eV | 2.70 eV | 0.34 eV | -5.42 eV | -1.95 eV |
| 1) Bandgap, 2) Singlet Energy, 3) Triplet Energy, 4) Difference between the singlet energy and the triplet energy, 5) Highest occupied molecular orbital Energy level, 6) Lowest unoccupied molecular orbital Energy level | | | | | | | |

Referring to Table 1, Comparative Compound A or B is in a form similar to Compound 1, but carbazole as an electron donating group and diphenyltriazine as an electron withdrawing group are in meta position in Comparative Compound A and in para position in Comparative Compound B. Calculation result shows that, as the position of the carbazole and diphenyltriazine were shifted from ortho to meta, and from metal to para, the singlet energy gradually increased and the triplet energy gradually decreased. As a result, the smallest energy different was found between the singlet state and the triplet state in the ortho form. The smaller energy difference between the singlet state and the triplet state is, the easier the Reverse Intersystem Crossing (RISC) is, and the better the thermally activated delay fluorescence property can be obtained.

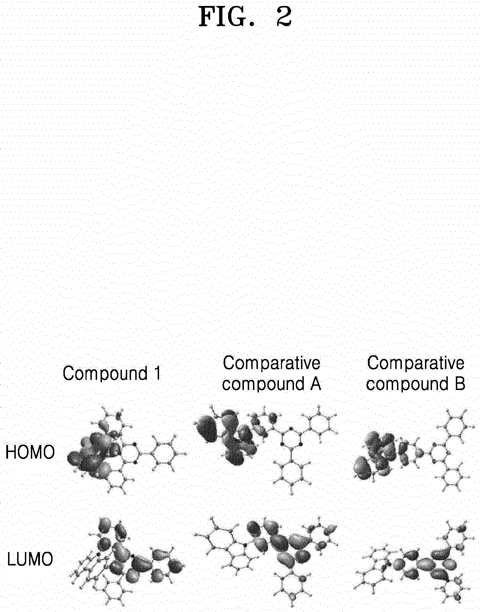

FIG. 2 shows the molecular orbital distribution of Compound 1 prepared according to Synthesis Example 1, Comparative Compound A and Comparative Compound B. The molecular orbital distribution was calculated utilizing the Gaussian 09 program through the B3LYP method according to density functional theory with a 6-31 G * basis set.
In order to show the fluorescence property effectively, the overlapping between HOMO and LUMO should exist at a certain level. As a result of calculation, HOMO and LUMO of ortho, meta and para forms are overlapped on benzene which is the central connecting unit, the fluorescence property of the ortho-form material was expected to be similar to other-form materials. These results indicate that the ortho-form material can up-convert the triplet exciton formed during electroluminescence to the singlet exciton more effectively through RISC than the meta- and para-form materials, and the singlet exciton can show effective fluorescent light emitting.

### Synthesis Example 16: Synthesis of Compound 104

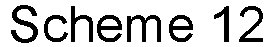

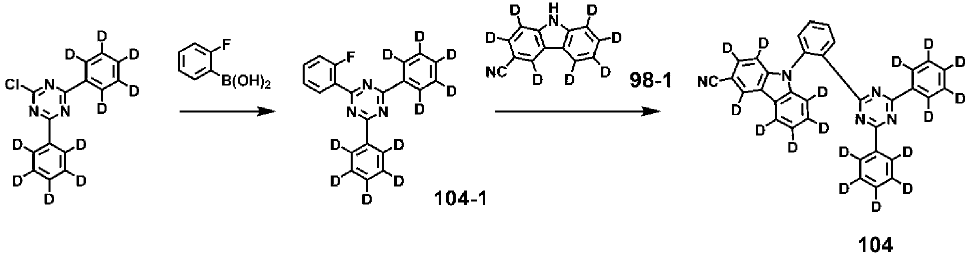

### Synthesis of Intermediate 104-1:

2-chloro-4,6-bis(phenyl-d5)-1,3,5-triazine (CAS#= 1300115-09-6) and 2-fluorophenylboronic acid (CAS#= 1993-03-9) was reacted under Pd catalyst and basic conditions to obtain Intermediate 104-1. The Intermediate 104-1 was confirmed by MS.
C₂₁H₄D₁₀FN₃ M+1: 338.21

### Synthesis of Compound 104:

4.1 g of Intermediate 104-1, 2.7 g of Intermediate 98-1, 6.4 g of K₃PO₄, and 50 mL of DMF were put into RBF (Round Bottom Flask) and stirred for 12 hours at 120 °C. After the reaction was completed, the reaction solution was extracted with ethyl acetate, the collected organic layer was dried with magnesium sulfate, and the solvent was evaporated. The residue obtained by evaporation was separated and purified by silica gel column chromatography to obtain 4.5 g (yield: 72%) of Compound 104. The Compound 104 was confirmed by LC-MS.
C₃₄H₄D₁₇N₅ M+1: 517.27

### Synthesis Example 17: Synthesis of Compound 105

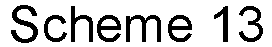

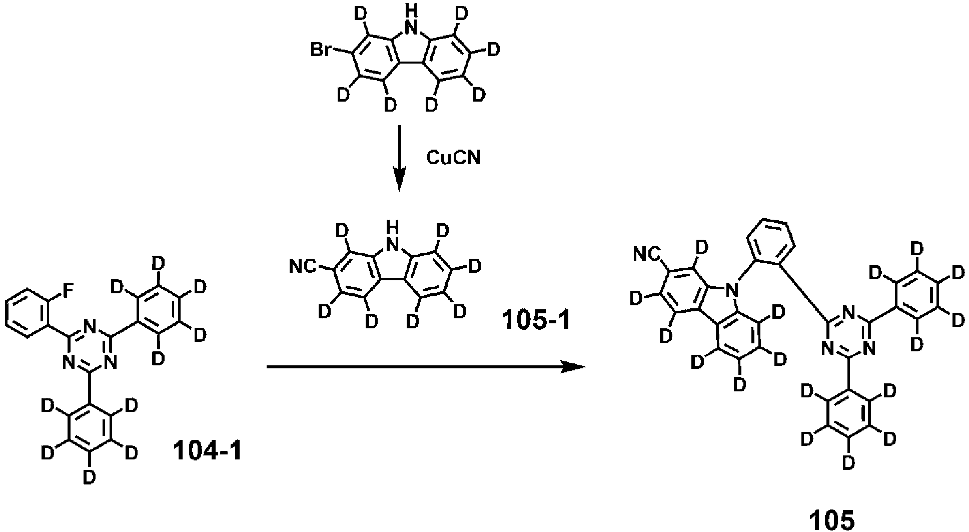

### Synthesis of Intermediate 105-1:

2-bromo-9H-carbazole-1,3,4,5,6,7,8-d7 (CAS#=2650519-97-2) was reacted with CuCN in DMF solvent to obtain Intermediate 105-1. The Intermediate 105-1 was confirmed by MS.
C₁₃HD₇N₂ M+1: 200.13

### Synthesis of Compound 105:

3.7g of Intermediate 104-1, 2.4 g of Intermediate 105-1, 5.8 g of K₃PO₄, and DMF were put in RBF and stirred for 12 hours at 120 °C. After the reaction was completed, the reaction solution was extracted with ethyl acetate, the collected organic layer was dried with magnesium sulfate, and the solvent was evaporated. The residue obtained by evaporation was separated and purified by silica gel column chromatography to obtain 2.9 g (yield: 51%) of Compound 105. The Compound 105 was confirmed by LC-MS.
C₃₄H₄D₁₇N₅ M+1: 517.28

### Synthesis Example 20: Synthesis of Compound 120

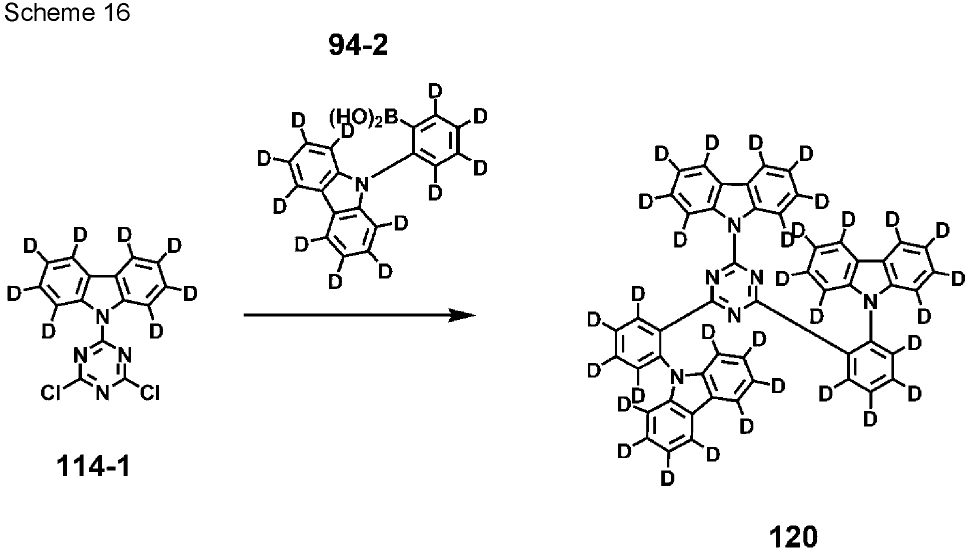

### Synthesis of Compound 120:

3.1 g of Intermediate 114-1, 7.3 g of Intermediate 94-2, 0.48 g of tetrakis (triphenylphosphine) palladium, and 3.6 g of potassium carbonate were put into a reaction vessel and dissolved in 80 mL of toluene, 20 mL of ethanol, and 20 mL of distilled water, and then the mixture was refluxed for 24 hours. After the reaction was completed, the reaction solution was extracted with ethyl acetate, the collected organic layer was dried with magnesium sulfate, and the solvent was evaporated. The residue obtained by evaporation was separated and purified by silica gel column chromatography to obtain 5.0 g (yield: 64%) of Compound 120. The Compound 120 was confirmed by LC-MS.
C₅₁D₃₂N₆ M+1: 761.49

### Synthesis Example 21: Synthesis of Compound 121

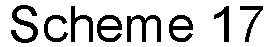

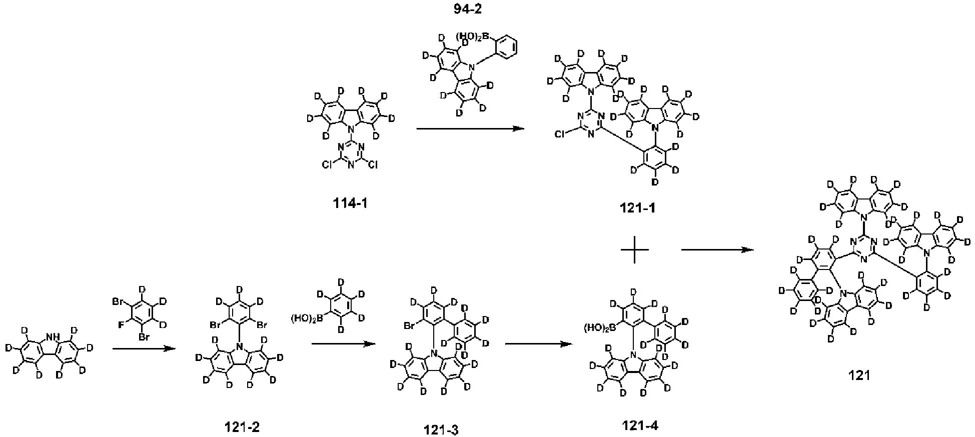

### Synthesis of Intermediate 121-1:

Intermediate 114-1 and Intermediate 94-2 was reacted under Pd catalyst and basic conditions to obtain Intermediate 121-1. The Intermediate 121-1 was confirmed by MS.
C₃₃D₂₀ClN₅ M+1: 542.29

### Synthesis of Intermediate 121-2:

9H-carbazole-1,2,3,4,5,6,7,8-d8 (CAS#=38537-24-5) and 1,3-dibromo-2-fluorobenzene-4,5,6-d3(CAS#= 2768654-61-9) was reacted under basic conditions to obtain Intermediate 121-2. The Intermediate 121-2 was confirmed by MS.
C₁₃D₁₁Br₂N M+1: 410.98

### Synthesis of Intermediate 121-3:

Intermediate 121-2 and phenyl-d5-boronic acid (CAS#= 215527-70-1) was reacted under Pd catalyst and basic conditions to obtain Intermediate 121-3. The Intermediate 121-3 was confirmed by MS.
C₂₄D₁₆BrN M+1: 414.13

### Synthesis of Intermediate 121-4:

Intermediate 121-3 was reacted with nBuLi (1.2 eq) at -78 °C. Then, the reaction solution was reacted with B(OMe)₃ (1.4 eq) to obtain Intermediate 121-4. The Intermediate 121-4 was confirmed by MS.
C₂₄H₂D₁₆BNO₂ M+1: 380.23

### Synthesis of Compound 121:

4.5 g of Intermediate 121-1, 3.8 g of Intermediate 121-4, 0.38 g of tetrakis (triphenylphosphine) palladium, and 2.9 g of potassium carbonate were put into a reaction vessel and dissolved in 80 mL of toluene, 20 mL of ethanol, and 20 mL of distilled water, and then the mixture was refluxed for 24 hours. After the reaction was completed, the reaction solution was extracted with ethyl acetate, the collected organic layer was dried with magnesium sulfate, and the solvent was evaporated. The residue obtained by evaporation was separated and purified by silica gel column chromatography to obtain 3.6 g (yield: 52%) of Compound 121. The Compound 121 was confirmed by LC-MS.
C₅₇D₃₆N₆ M+1: 841.51

### Synthesis Example 22: Synthesis of Compound 122

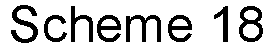

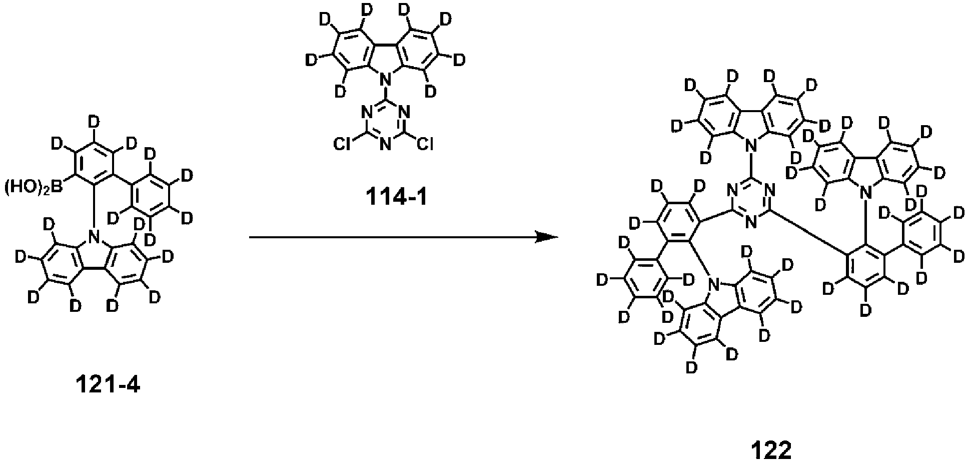

### Synthesis of Compound 122:

2.9 g of Intermediate 114-1, 7.5 g of Intermediate 121-4, 0.41 g of tetrakis (triphenylphosphine) palladium, and 3.1 g of potassium carbonate were put into a reaction vessel and dissolved in 80 mL of toluene, 20 mL of ethanol, and 20 mL of distilled water, and then the mixture was refluxed for 24 hours. After the reaction was completed, the reaction solution was extracted with ethyl acetate, the collected organic layer was dried with magnesium sulfate, and the solvent was evaporated. The residue obtained by evaporation was separated and purified by silica gel column chromatography to obtain 5.9 g (yield: 71%) of Compound 122. The Compound 122 was confirmed by LC-MS.
C₆₃D₄₀N₆ M+1: 921.57

### Evaluation Example 2

Table 2 shows the values calculated by the molecular calculation technique for Compounds prepared according to Synthesis Examples 16, 17 and 20 to 22, and Comparative Compounds C1 to C4. Molecular calculations were performed utilizing the Gaussian 09 program through the B3LYP method according to density functional theory on a 6-31 G * basis set.

**[Table 2]**

| Sample | Structural Formula | ¹⁾B_{g} | ²⁾S₁ | ³⁾T₁ | ⁴⁾ΔE_{ST} | ⁵⁾HOMO | ⁶⁾LUMO |
|---|---|---|---|---|---|---|---|
| Compound 104 | 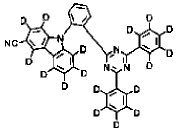 | 3.61 eV | 3.02 eV | 2.94 eV | 0.08 eV | -5.45 eV | -1.84 eV |
| Compound 105 | 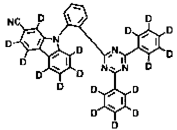 | 3.58 eV | 3.01 eV | 2.93 eV | 0.08 eV | -5.42 eV | -1.84 eV |
| Compound 120 | 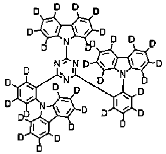 | 3.42 eV | 2.90 eV | 2.86 eV | 0.04 eV | -5.29 eV | -1.87 eV |
| Compound 121 | 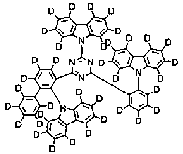 | 3.45 eV | 2.89 eV | 2.87 eV | 0.02 eV | -5.29 eV | -1.84 eV |
| Compound 122 | 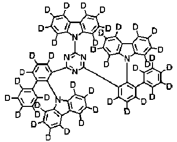 | 3.44 eV | 2.88 eV | 2.85 eV | 0.03 eV | -5.29 eV | -1.83 eV |
| Comparative Compound C1 (para) | 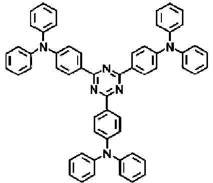 | 3.30 eV | 2.71 eV | 2.32 eV | 0.39 eV | -5.09 eV | -1.79 eV |
| Comparative Compound C2 (meta) | 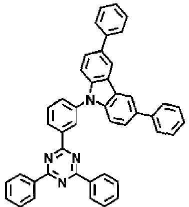 | 3.38 eV | 3.12 eV | 2.81 eV | 0.31 eV | -5.21 eV | -1.83 eV |
| Comparative Compound C3 (meta) | 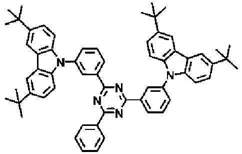 | 3.35 eV | 3.17 eV | 2.83 eV | 0.34 eV | -5.18 eV | -1.83 eV |
| Comparative Compound C4 (para) | 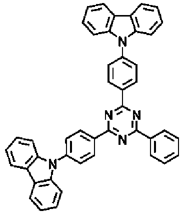 | 3.36 eV | 3.11 eV | 2.80 eV | 0.31 eV | -5.17 eV | -1.81 eV |
| 1) Bandgap, 2) Singlet Energy, 3) Triplet Energy, 4) Difference between the singlet energy and the triplet energy, 5) Highest occupied molecular orbital Energy level, 6) Lowest unoccupied molecular orbital Energy level | | | | | | | |

Referring to Table 2, Comparative Compound C1, C2, C3 or C4 is in a form similar to a compound represented by Chemical Formula 2 according to the invention, but an electron donating group and an electron withdrawing group are in meta position in Comparative Compounds C1 and C3 and in para position in Comparative Compounds C2 and C4, and Comparative Compounds C1 to C4 do not comprise deuterium. Calculation result shows that the smallest energy different was found between the singlet state and the triplet state in the ortho form. The smaller energy difference between the singlet state and the triplet state is, the easier the Reverse Intersystem Crossing (RISC) is, and the better the thermally activated delay fluorescence property can be obtained.

In order to show the fluorescence property effectively, the overlapping between HOMO and LUMO should exist at a certain level. As a result of calculation, HOMO and LUMO of ortho, meta and para forms are overlapped on benzene which is the central connecting unit, the fluorescence property of the ortho-form material was expected to be similar to other-form materials. These results indicate that the ortho-form material can up-convert the triplet exciton formed during electroluminescence to the singlet exciton more effectively through RISC than the meta- and para-form materials, and the singlet exciton can show effective fluorescent light emitting.

In a compound represented by Chemical Formula 2 according to the invention, the electron donating group and the electron withdrawing group are introduced at ortho positions of the benzene ring. In addition, in the compound represented by Chemical Formula 2 according to the invention, at least one of R₁, R₂, R₁₇ and R₁₈ comprises a deuterium, or at least one of R₂₂ to R₂₅ is a deuterium. Accordingly, the compound may have a high T₁ level, so that it may effectively block the exciton of a dopant, and the compound may have a small ΔE_{ST} value, so that energy transfer from a host to a dopant may be facilitated. Accordingly, a light-emitting device including the compound may have excellent characteristics in terms of driving voltage and maximum quantum efficiency.

### Preparation Example 11: Preparation of light-emitting device

For the anode, a corning 15 Ω/cm² (120 nm (1,200 Å)) ITO glass substrate was cut into a size of 50 mm x 50 mm x 0.5 mm, sonicated with isopropyl alcohol and pure water each for 5 minutes, and then cleaned by ultraviolet rays and ozone for 30 minutes. The resultant glass substrate was provided to a vacuum deposition apparatus.

On the glass substrate, HATCN was applied on the substrate to form a hole injection layer having a thickness of 10 nm (100 Å). Then, the first hole transport material, BCFN, was vacuum-deposited to a thickness of 60 nm (600 Å), and then, the second hole transport material, SiCzCz, which serves as a hole-transporting material, was vacuum-deposited to a thickness of 5 nm (50 Å) to form a hole transport layer.

On the hole transport layer, SiCzCz and a Compound 93 (not according to the invention) as a mixed host, and PtON-TBBI as a phosphorescent dopant were co-deposited in a weight ratio of 60:27:13 to form an emission layer having a thickness of 35 nm (350 Å).

Subsequently, on the emission layer, after depositing mSiTrz to a thickness of 5 nm (50 Å) as a first electron transport layer, and then mSiTrz and LiQ as a mixture were co-deposited in a weight ratio of 1:1 to form an electron transport layer having a thickness of 35 nm (350 Å). On the electron transport layer, LiF, an alkali metal halide, was deposited to form an electron injection layer having a thickness of 1.5 nm (15 Å), and Al was vacuum-deposited to a thickness of 8 nm (80 Å) to form a LiF/Al electrode, thereby completing the manufacture a light-emitting device.

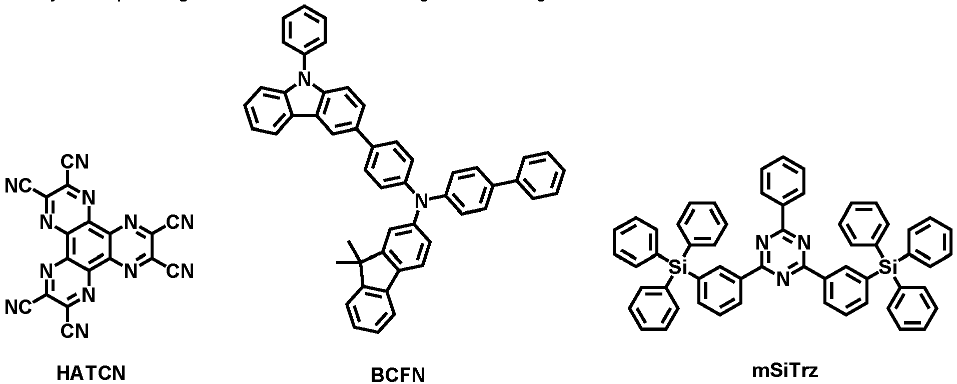

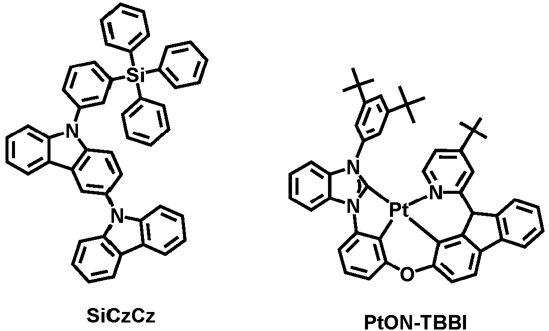

### Preparation Examples 12 to 16 and Comparative Examples 2 to 5

Light-emitting devices were manufactured in substantially the same manner as in Preparation Example 11, except that compounds shown in Table 2 were each used instead of Compound 93 in forming the emission layer.

### Evaluation Example 3

For the light-emitting devices manufactured according to Preparation Examples 12 to 16 and Comparative Examples 2 to 5, the driving voltage (V) and maximum quantum efficiency (%) at a current density of 10 mA/cm² were measured, and results thereof are shown in Table 3.

The driving voltage and current density of the light-emitting device were measured using a source meter (Keithley Instrument, 2400 series), and the maximum quantum efficiency was measured using an external quantum efficiency measuring device (Hamamatsu Photonics, C9920-2-12). In the evaluation of the maximum quantum efficiency, luminance/current density was measured using a luminance meter with wavelength sensitivity calibration, and the maximum quantum efficiency was calculated assuming a Lambertian angular luminance distribution with a perfect diffusive reflecting surface. The evaluation results of the characteristics of the organic light-emitting device are shown in the Table 3 below.

**[Table 3]**

| | Host material of emission layer | Driving voltage (V) | Current density (mA/cm ²) | Maximum quantum efficiency (%) | Luminescent color |
|---|---|---|---|---|---|
| Preparation Example 12 | Compound 104 | 4.7 | 2.3 | 19.8 | Blue |
| Preparation Example 13 | Compound 105 | 4.6 | 2.3 | 21.5 | Blue |
| Preparation Example 14 | Compound 120 | 5.2 | 2.3 | 21.5 | Blue |
| Preparation Example 15 | Compound 121 | 5.3 | 2.3 | 22.1 | Blue |
| Preparation Example 16 | Compound 122 | 5.1 | 2.3 | 22.2 | Blue |
| Comparative Example 2 | Compound C1 | 5.6 | 2.3 | 9.7 | Blue |
| Comparative Example 3 | Compound C2 | 5.8 | 2.3 | 13.2 | Blue |
| Comparative Example 4 | Compound C3 | 6.0 | 2.3 | 11.3 | Blue |
| Comparative Example 5 | Compound C4 | 6.1 | 2.3 | 8.7 | Blue |

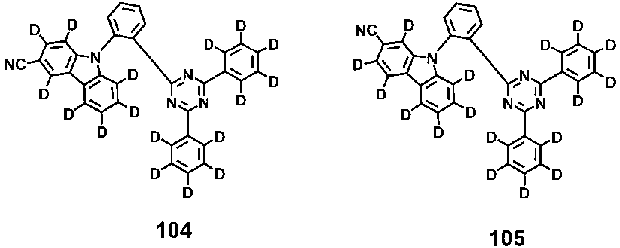

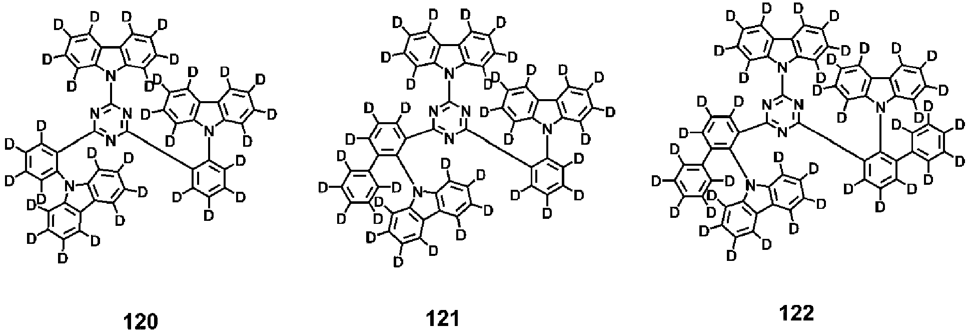

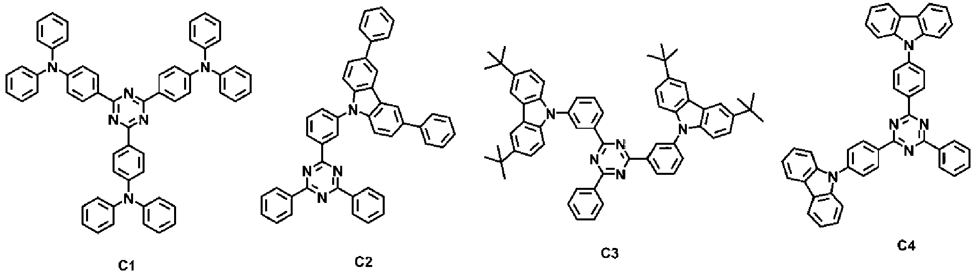

From Table 3, it was confirmed that the light-emitting devices according to Preparation Examples 12 to 16 had excellent characteristics in terms of driving voltage and maximum quantum efficiency compared to the light-emitting devices according to Comparative Examples 2 to 5.

According to the one or more embodiments, by including a compound represented by Formula 2, a light-emitting device may have excellent characteristics in terms of driving voltage and maximum quantum efficiency. In addition, a high-quality electronic apparatus and electronic equipment may be manufactured by using the light-emitting device.

The present invention is not limited to the above-described embodiments and the accompanying drawings. While the example embodiments of the present invention and their advantages have been described in more detail, it should be understood that one or more suitable changes, substitutions, and alterations may be made herein without departing from the scope of the present invention as long as the embodiments fall under the scope of the claims.

## Claims

1. A light-emitting material represented by Chemical Formula 2: 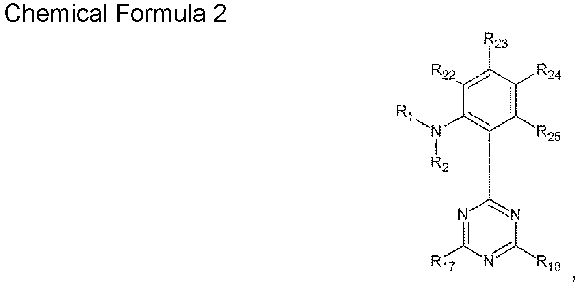 wherein in Chemical Formula 2,
R₁ and R₂ are, independently of each other, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, or a substituted or unsubstituted C3-C30 heteroaryl group; and R₁ and R₂ are optionally combined together with the nitrogen to which they are attached to form a substituted or unsubstituted heterocyclyl or a substituted or unsubstituted heteroaryl group,
R₂₂ to R₂₅ are, independently of each other, hydrogen, deuterium, a halogen group, or a substituted or unsubstituted C4-C6 aryl group
wherein the triazine group substituted with R₁₇ and R₁₈ in Chemical Formula 2 is represented by any one of Structural Formulas B1 to B13, B50 and B51: 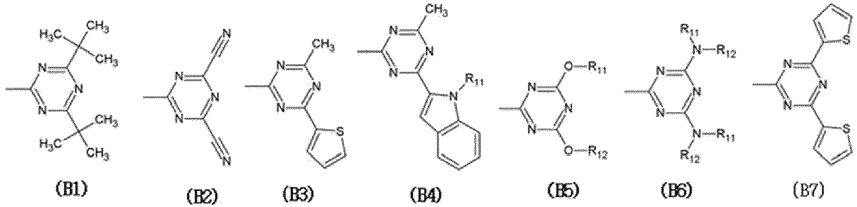 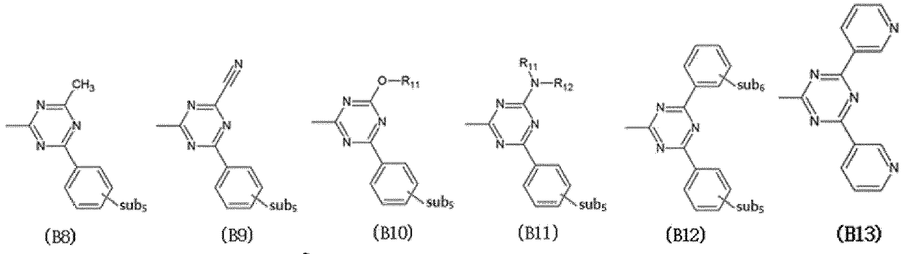 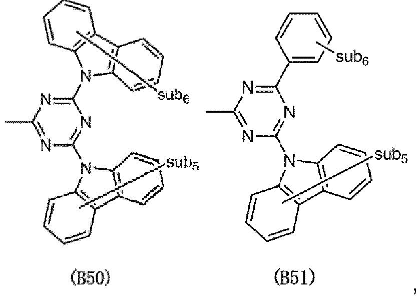
wherein in Structural Formulas B1 to B13, B50 and B51,
R₁₁ and R₁₂ are, independently of each other, hydrogen, deuterium, or a substituted or unsubstituted C1-C2 alkyl group,
Sub₅ and Sub₆ are, independently of each other, hydrogen, deuterium, a halogen group, a cyano group, a substituted or unsubstituted C1-C9 alkyl group, a substituted or unsubstituted C6-C30 aryl group, a substituted or unsubstituted C3-C30 heteroaryl group, a substituted or unsubstituted C1-C9 alkyloxy group, a substituted or unsubstituted -NRₑR_{f}, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group, and Sub₅ and Sub₆ are, independently of each other, optionally fused to a main body to which they are bonded to form a substituted or unsubstituted cyclic or a substituted or unsubstituted aryl, and
Rₑ and R_{f} are each independently selected from the group consisting of a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, and a substituted or unsubstituted C3-C30 heteroaryl group, and Rₑ and R_{f} are optionally combined together with the nitrogen to which they are attached to form a substituted or unsubstituted heterocyclyl or a substituted or unsubstituted heteroaryl,
wherein, in Structural Formula B50, Sub₅ is a cyano group, whereby Sub₅ may also exist, independently of each other, in multiple numbers, so that other Sub₅ may be as defined above,
wherein, in Structural Formula B51, R₂₂ to R₂₅ are, independently of each other, deuterium, a halogen group, or a substituted or unsubstituted C4-C6 aryl group, and
wherein at least one selected from R₁, R₂, R₁₇ and R₁₈ comprises a deuterium, or at least one selected from R₂₂ to R₂₅ is a deuterium.

2. The light-emitting material of claim 1,
wherein, in Chemical Formula 2, R₁ and R₂ are combined together with the nitrogen to which they are attached to form a substituted or unsubstituted heterocyclyl or a substituted or unsubstituted heteroaryl group.

3. The light-emitting material of claim 1,
wherein, in Chemical Formula 2, R₁ and R₂ are combined together with the nitrogen to which they are attached to form a substituted or unsubstituted heterocyclyl or a substituted or unsubstituted heteroaryl group represented by any one of Structural Formulas A1 to A6 and A9 to A19: 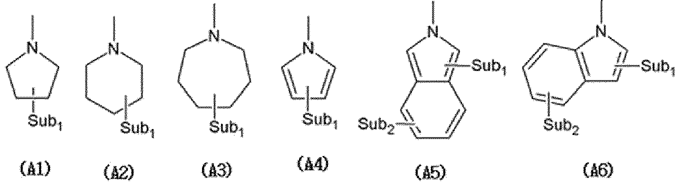 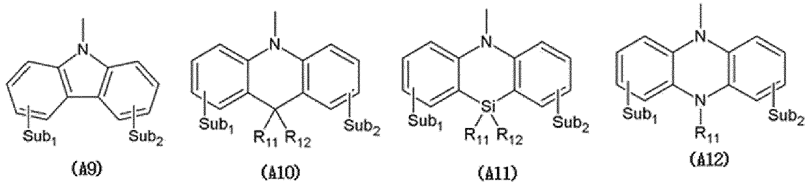 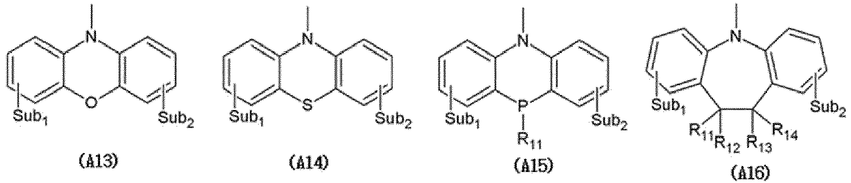 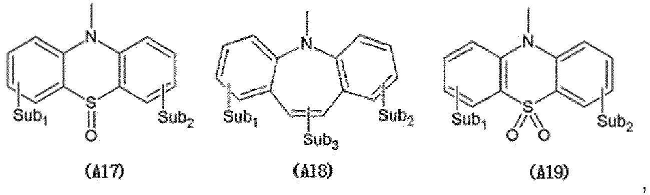
wherein, in Structural Formulas A1 to A6 and A9 to A19,
R₁₁ to R₁₄ are, independently of each other, hydrogen, deuterium, a substituted or unsubstituted C1-C2 alkyl group, or a substituted or unsubstituted C6-C30 aryl group,
Sub₁ and Sub₂ are, independently of each other, hydrogen, deuterium, a halogen group, a cyano group, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C3-C30 heteroaryl group, a substituted or unsubstituted C1-C9 alkyloxy group, a substituted or unsubstituted -NR_{c}R_{d}, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylsilyl group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group,
R_{c} and R_{d} are, independently of each other, selected from the group consisting of a substituted or unsubstituted C1-C9 alkyl group, a substituted or unsubstituted C5-C30 cycloalkyl group, a substituted or unsubstituted C6-C30 aryl group, a substituted or unsubstituted C6-C30 aralkyl group, and a substituted or unsubstituted C3-C30 heteroaryl group, and R_{c} and R_{d} are optionally combined together with the nitrogen to which they are attached to form a substituted or unsubstituted heterocyclyl or a substituted or unsubstituted heteroaryl group, and
Sub₁ and Sub₂ are, independently of each other, optionally fused to a main body to which they are bonded to form a substituted or unsubstituted cyclic or a substituted or unsubstituted aryl group.

4. The light-emitting material of claim 1,
wherein, in Chemical Formula 2, R₁ and R₂ are combined together with the nitrogen to which they are attached to form a substituted or unsubstituted heterocyclyl or a substituted or unsubstituted heteroaryl group represented by any one of Structural Formulas A1 to A6 or A9 to A45: 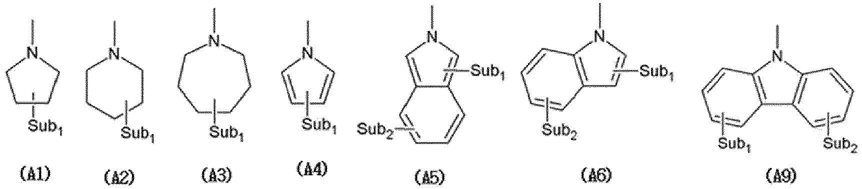 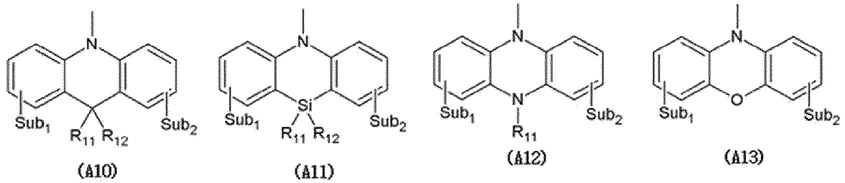 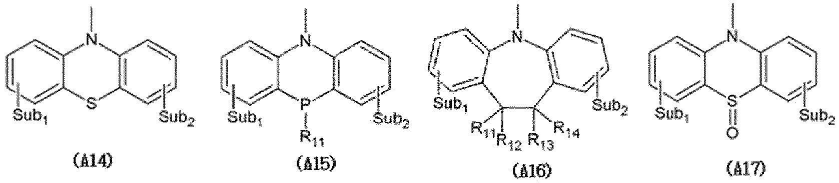 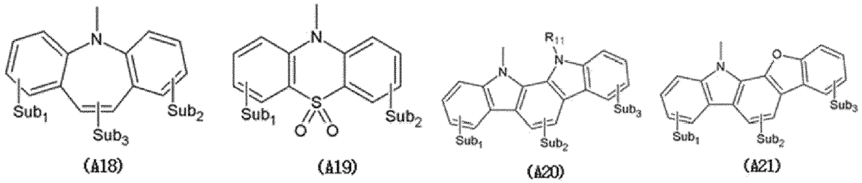 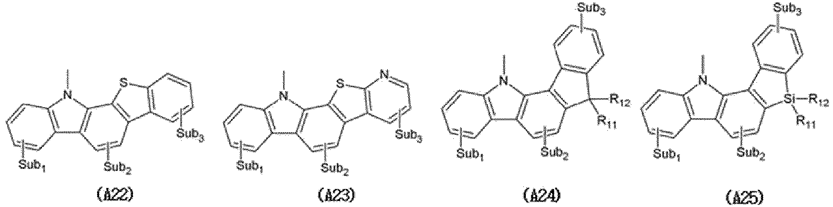 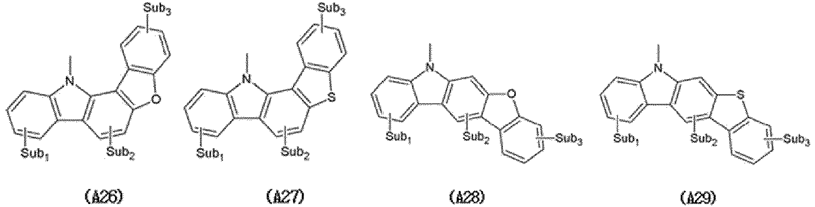 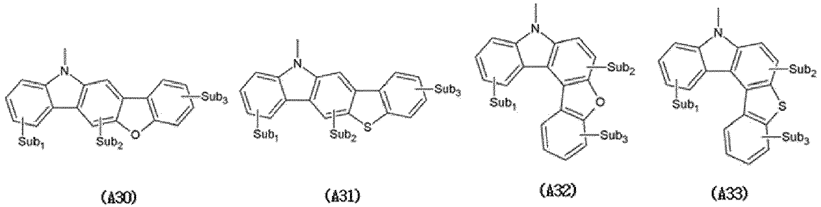 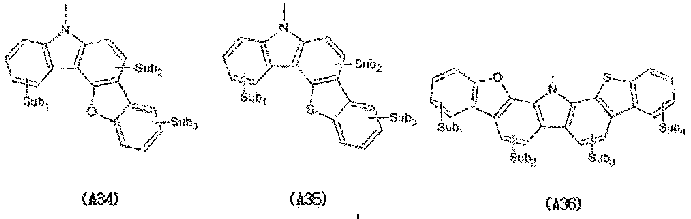 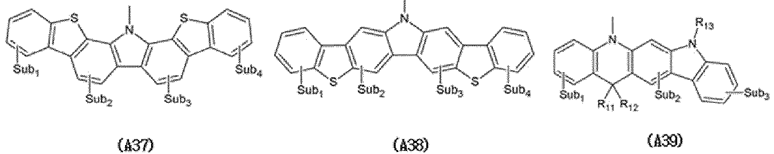 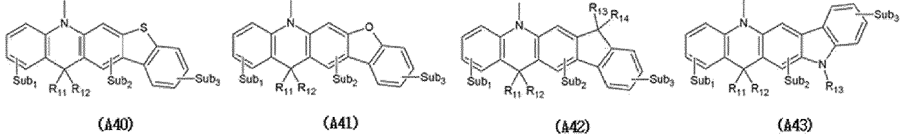 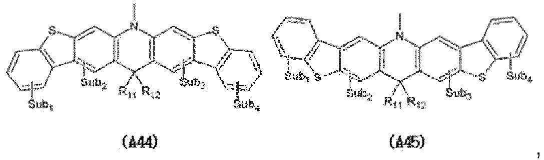
or, in Chemical Formula 2, R₁ and R₂ are selected to be represented by any one of Structural Formulas A7 or A8: 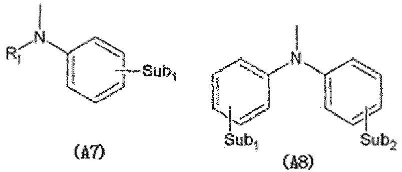
wherein in Structural Formulas A1 to A45,
R₁₁ to R₁₄ are, independently of each other, hydrogen, deuterium, a substituted or unsubstituted C1-C2 alkyl group, or a substituted or unsubstituted C6-C30 aryl group, and
Sub₁ to Sub₄ are, independently of each other, hydrogen, deuterium, a halogen group, a cyano group, a substituted or unsubstituted C1-C9 alkyl group, a substituted or unsubstituted C5-C30 cycloalkyl group, a substituted or unsubstituted C6-C30 aryl group, a substituted or unsubstituted C6-C30 alkylaryl group, a substituted or unsubstituted C6-C30 aralkyl group, a substituted or unsubstituted C3-C30 heteroaryl group, a substituted or unsubstituted C1-C9 alkyloxy group, a substituted or unsubstituted C6-C30 aryloxy group, a substituted or unsubstituted silyl group, a substituted or unsubstituted phosphine group, a substituted or unsubstituted phosphine oxide group, a substituted or unsubstituted thiol group, a substituted or unsubstituted sulfoxide group, or a substituted or unsubstituted sulfone group.

5. The light-emitting material of claim 4,
wherein, in Structural Formulas A1 to A45, Sub₁ to Sub₄ are, independently of each other, hydrogen, deuterium, a halogen group, or a cyano group.

6. The light-emitting material of at least one of claims 1 to 5,
wherein the triazine group substituted with R₁₇ and R₁₈ in Chemical Formula 2 is represented by any one of Structural Formulas B8 to B12, B50 and B51: 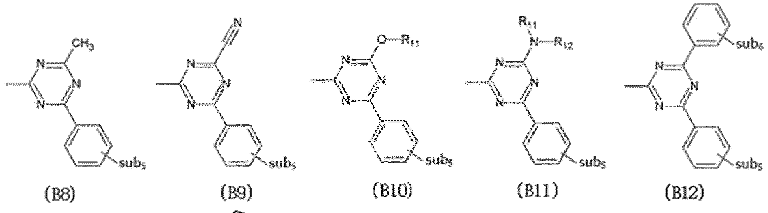 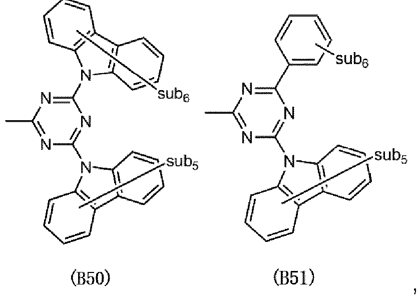
wherein in Structural Formulas B8 to B12, B50 and B51,
R₁₁ and R₁₂ are, independently of each other, hydrogen, deuterium, or a substituted or unsubstituted C1-C2 alkyl group,
Sub₅ and Sub₆ are, independently of each other, hydrogen, deuterium, a halogen group, a cyano group, a substituted or unsubstituted C1-C9 alkyl group, a substituted or unsubstituted C6-C30 aryl group, a substituted or unsubstituted C3-C30 heteroaryl group, a substituted or unsubstituted C1-C9 alkyloxy group, a substituted or unsubstituted silyl group, a substituted or unsubstituted phosphine group, a substituted or unsubstituted phosphine oxide group, a substituted or unsubstituted thiol group, a substituted or unsubstituted sulfoxide group, a substituted or unsubstituted sulfone group, a substituted or unsubstituted C5-C30 arylthio group, a substituted or unsubstituted C5-C30 aryloxy group, a substituted or unsubstituted C5-C30 arylamine group, or a substituted or unsubstituted C5-C30 aralkyl group; and Sub₅ and Sub₆ are, independently of each other, optionally fused to a main body to which they are bonded to form a substituted or unsubstituted cyclic or a substituted or unsubstituted aryl, and
wherein, in Structural Formula B50, Sub₅ is a cyano group, whereby Sub₅ may also exist, independently of each other, in multiple numbers, so that other Sub₅ may be as defined above.

7. The light-emitting material of at least one of claims 1 to 5,
wherein the triazine group substituted with R₁₇ and R₁₈ in Chemical Formula 2 is represented by any one of Structural Formulas B12 and B50 to B54: 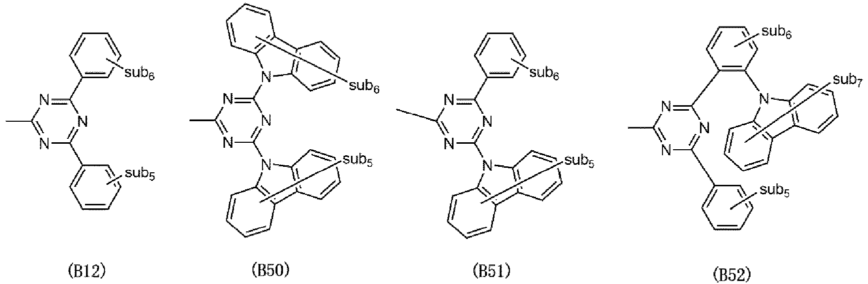 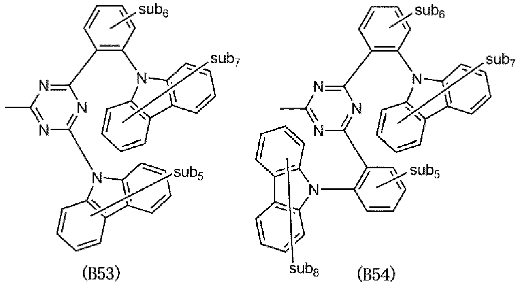
wherein in Structural Formulas B12 and B50 to B54,
Sub₅ to Sub₈ are, independently of each other, hydrogen, deuterium, a halogen group, a cyano group, a substituted or unsubstituted C6-C30 aryl group, or a substituted or unsubstituted C3-C30 heteroaryl group, and
wherein, in Structural Formula B50, Sub₅ is a cyano group, whereby Sub₅ may also exist, independently of each other, in multiple numbers, so that other Sub₅ may be as defined above.

8. The light-emitting material of at least one of claims 1 to 7,
wherein, in Chemical Formula 2, R₂₂ to R₂₅ are, independently of each other, hydrogen or deuterium, and
wherein when the triazine group substituted with R₁₇ and R₁₈ in Chemical Formula 2 is represented by Structural Formula B51, R₂₂ to R₂₅ are, independently of each other, deuterium.

9. The light-emitting material of claim 1,
wherein the light-emitting material represented by Chemical Formula 2 is represented by any one of Compounds 100, 101, 104 to 106, 108, 109, 112, 113 and 120 to 122:

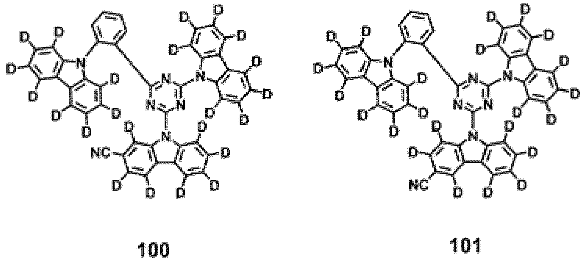

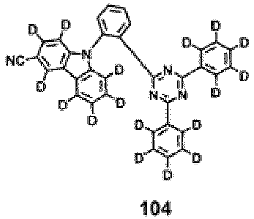

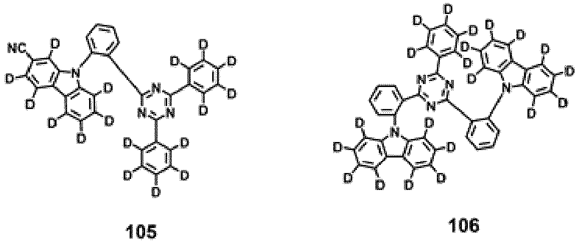

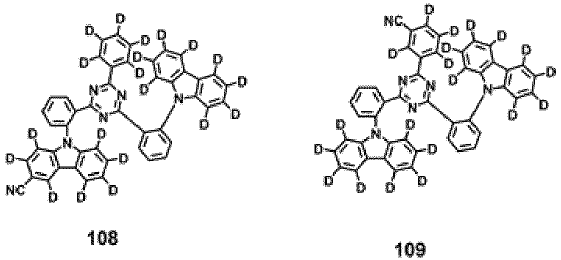

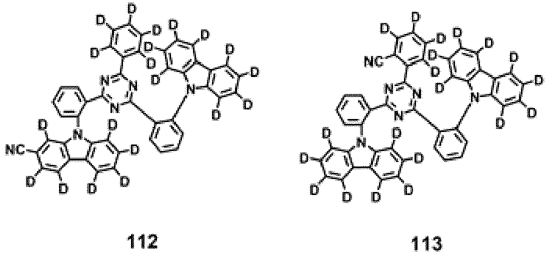

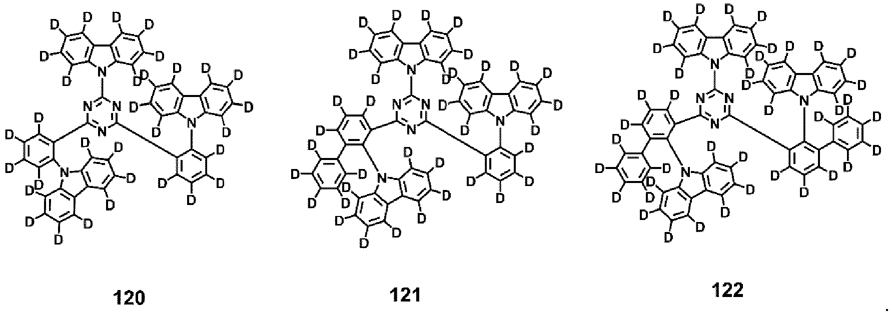

10. A light-emitting device (10) comprising:
a first electrode (110);
a second electrode (150) facing the first electrode (110);
an interlayer (130) arranged between the first electrode (110) and the second electrode (150) and comprising an emission layer; and
a light-emitting material according to at least one of claims 1 to 9.

11. The light-emitting device (10) of claim 10, wherein the emission layer comprises the light-emitting material represented by Formula 2.

12. The light-emitting device (10) of claim 10, wherein the emission layer comprises a host and a dopant, and
the host comprises the light-emitting material represented by Formula 2.

13. The light-emitting device (10) of claim 12,
wherein the host is a delayed fluorescent light emitting material.

14. The light-emitting device (10) of claim 10, further comprising:
a first capping layer arranged outside the first electrode (110); and
a second capping layer arranged outside the second electrode (150),
wherein the first capping layer or the second capping layer comprises the light-emitting material represented by Formula 2.

15. An electronic apparatus or equipment comprising the light-emitting device (10) of at least one of claims 10 to 14.

## Patentansprüche

1. Lichtemittierendes Material, das durch die chemische Formel 2 dargestellt ist: 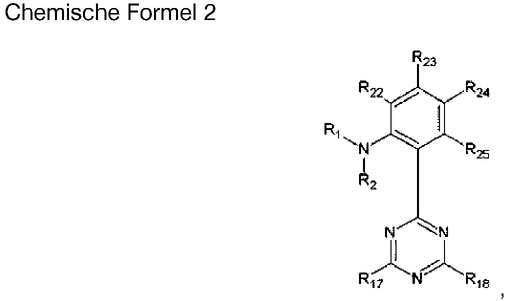 wobei in der chemischen Formel 2
R₁ und R₂ unabhängig voneinander eine substituierte oder unsubstituierte C1-C9-Alkylgruppe, eine substituierte oder unsubstituierte C5-C30-Cycloalkylgruppe, eine substituierte oder unsubstituierte C6-C30-Arylgruppe, eine substituierte oder unsubstituierte C6-C30-Alkylarylgruppe, eine substituierte oder unsubstituierte C6-C30-Aralkylgruppe oder eine substituierte oder unsubstituierte C3-C30-Heteroarylgruppe sind; und R₁ und R₂ optional zusammen mit dem Stickstoff, an den sie angelagert sind, kombiniert sind, um ein substituiertes oder unsubstituiertes Heterocyclyl oder eine substituierte oder unsubstituierte Heteroarylgruppe zu bilden,
R₂₂ bis R₂₅ unabhängig voneinander Wasserstoff, Deuterium, eine Halogengruppe oder eine substituierte oder unsubstituierte C4-C6-Arylgruppe sind,
wobei die mit R₁₇ und R₁₈ substituierte Triazingruppe in der chemischen Formel 2 durch eine beliebige der Strukturformeln B1 bis B13, B50 und B51 dargestellt ist: 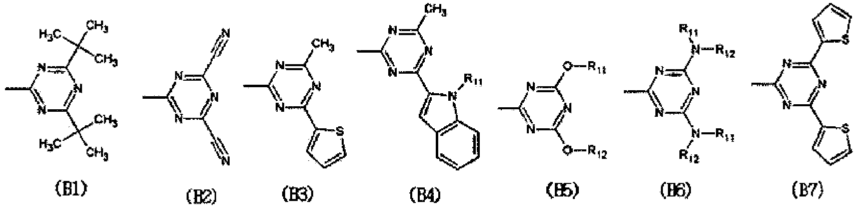 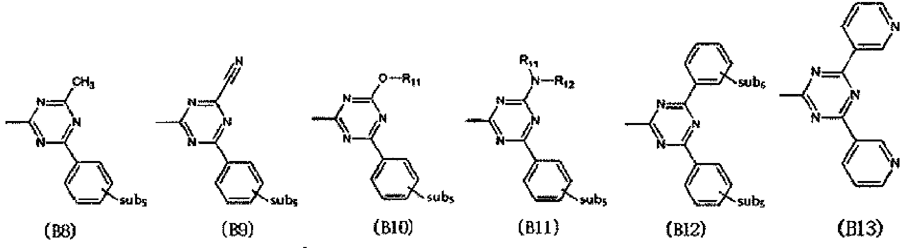 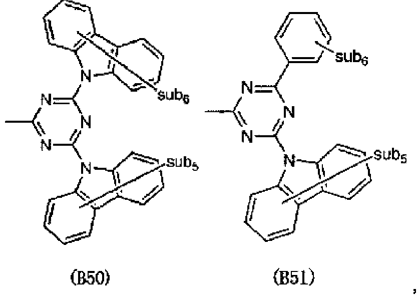
wobei in den Strukturformeln B1 bis B13, B50 und B51
R₁₁ und R₁₂ unabhängig voneinander Wasserstoff, Deuterium oder eine substituierte oder unsubstituierte C1-C2-Alkylgruppe sind,
Sub₅ und Sub₆ unabhängig voneinander Wasserstoff, Deuterium, eine Halogengruppe, eine Cyanogruppe, eine substituierte oder unsubstituierte C1-C9-Alkylgruppe, eine substituierte oder unsubstituierte C6-C30-Arylgruppe, eine substituierte oder unsubstituierte C3-C30-Heteroarylgruppe, eine substituierte oder unsubstituierte C1-C9-Alkyloxygruppe, ein substituiertes oder unsubstituiertes -NRₑR_{f}, eine substituierte oder unsubstituierte Silylgruppe, eine substituierte oder unsubstituierte Phosphingruppe, eine substituierte oder unsubstituierte Phosphinoxidgruppe, eine substituierte oder unsubstituierte Thiolgruppe, eine substituierte oder unsubstituierte Sulfoxidgruppe, eine substituierte oder unsubstituierte Sulfongruppe, eine substituierte oder unsubstituierte C5-C30-Arylthiogruppe, eine substituierte oder unsubstituierte C5-C30-Aryloxygruppe, eine substituierte oder unsubstituierte C5-C30-Arylamingruppe oder eine substituierte oder unsubstituierte C5-C30-Aralkylgruppe sind und Sub₅ und Sub₆ unabhängig voneinander optional mit einem Hauptkörper anelliert sind, an den sie gebunden sind, um ein substituiertes oder unsubstituiertes cyclisches oder ein substituiertes oder unsubstituiertes Aryl zu bilden, und
Rₑ und R_{f} jeweils unabhängig aus der Gruppe ausgewählt sind, die aus einer substituierten oder unsubstituierten C1-C9-Alkylgruppe, einer substituierten oder unsubstituierten C5-C30-Cycloalkylgruppe, einer substituierten oder unsubstituierten C6-C30-Arylgruppe, einer substituierten oder unsubstituierten C6-C30-Alkylarylgruppe, einer substituierten oder unsubstituierten C6-C30-Aralkylgruppe und einer substituierten oder unsubstituierten C3-C30-Heteroarylgruppe besteht, und Rₑ und R_{f} optional zusammen mit dem Stickstoff, an den sie angelagert sind, kombiniert sind, um ein substituiertes oder unsubstituiertes Heterocyclyl oder ein substituiertes oder unsubstituiertes Heteroaryl zu bilden,
wobei in der Strukturformel B50 Sub₅ eine Cyanogruppe ist, wodurch Sub₅ unabhängig voneinander auch in mehrfacher Anzahl vorliegen kann, sodass andere Sub₅ wie vorstehend definiert sein können,
wobei in der Strukturformel B51 R₂₂ bis R₂₅ unabhängig voneinander Deuterium, eine Halogengruppe oder eine substituierte oder unsubstituierte C4-C6-Arylgruppe sind und
wobei mindestens eines, das aus R₁, R₂, R₁₇ und R₁₈ ausgewählt ist, ein Deuterium umfasst oder mindestens eines, das aus R₂₂ bis R₂₅ ausgewählt ist, ein Deuterium ist.

2. Lichtemittierendes Material nach Anspruch 1,
wobei in der chemischen Formel 2 R₁ und R₂ zusammen mit dem Stickstoff, an den sie angelagert sind, kombiniert sind, um ein substituiertes oder unsubstituiertes Heterocyclyl oder eine substituierte oder unsubstituierte Heteroarylgruppe zu bilden.

3. Lichtemittierendes Material nach Anspruch 1,
wobei in der chemischen Formel 2 R₁ und R₂ zusammen mit dem Stickstoff, an den sie angelagert sind, kombiniert sind, um ein substituiertes oder unsubstituiertes Heterocyclyl oder eine substituierte oder unsubstituierte Heteroarylgruppe zu bilden, die durch eine beliebige der Strukturformeln A1 bis A6 und A9 bis A19 dargestellt sind: 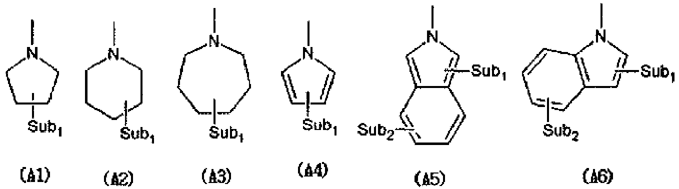 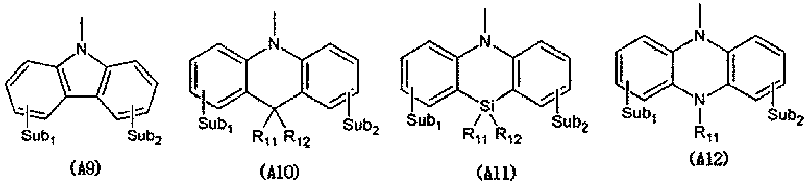 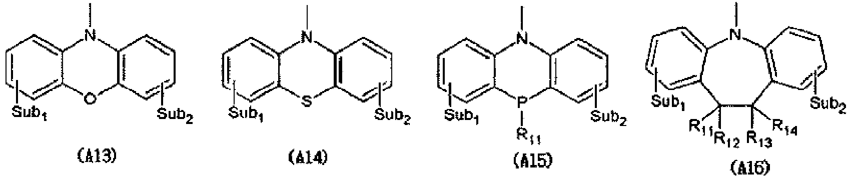 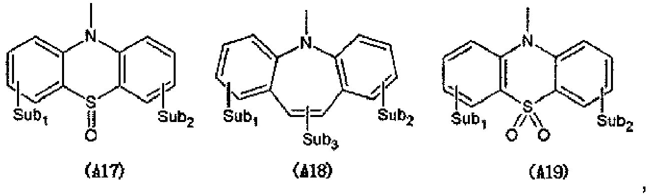
wobei in den Strukturformeln A1 bis A6 und A9 bis A19
R₁₁ bis R₁₄ unabhängig voneinander Wasserstoff, Deuterium, eine substituierte oder unsubstituierte C1-C2-Alkylgruppe oder eine substituierte oder unsubstituierte C6-C30-Arylgruppe sind,
Sub₁ und Sub₂ unabhängig voneinander Wasserstoff, Deuterium, eine Halogengruppe, eine Cyanogruppe, eine substituierte oder unsubstituierte C1-C9-Alkylgruppe, eine substituierte oder unsubstituierte C5-C30-Cycloalkylgruppe, eine substituierte oder unsubstituierte C6-C30-Arylgruppe, eine substituierte oder unsubstituierte C6-C30-Alkylarylgruppe, eine substituierte oder unsubstituierte C3-C30-Heteroarylgruppe, eine substituierte oder unsubstituierte C1-C9-Alkyloxygruppe, ein substituiertes oder unsubstituiertes -NR_{c}R_{d}, eine substituierte oder unsubstituierte Silylgruppe, eine substituierte oder unsubstituierte Phosphingruppe, eine substituierte oder unsubstituierte Phosphinoxidgruppe, eine substituierte oder unsubstituierte Thiolgruppe, eine substituierte oder unsubstituierte Sulfoxidgruppe, eine substituierte oder unsubstituierte Sulfongruppe, eine substituierte oder unsubstituierte C5-C30-Arylsilylgruppe, eine substituierte oder unsubstituierte C5-C30-Arylthiogruppe, eine substituierte oder unsubstituierte C5-C30-Aryloxygruppe, eine substituierte oder unsubstituierte C5-C30-Arylamingruppe oder eine substituierte oder unsubstituierte C5-C30-Aralkylgruppe sind, R_{c} und R_{d} unabhängig voneinander aus der Gruppe ausgewählt sind, die aus einer substituierten oder unsubstituierten C1-C9-Alkylgruppe, einer substituierten oder unsubstituierten C5-C30-Cycloalkylgruppe, einer substituierten oder unsubstituierten C6-C30-Arylgruppe, einer substituierten oder unsubstituierten C6-C30-Aralkylgruppe und einer substituierten oder unsubstituierten C3-C30-Heteroarylgruppe besteht, und R_{c} und R_{d} optional zusammen mit dem Stickstoff, an den sie angelagert sind, kombiniert sind, um ein substituiertes oder unsubstituiertes Heterocyclyl oder eine substituierte oder unsubstituierte Heteroarylgruppe zu bilden, und
Sub₁ und Sub₂ unabhängig voneinander optional mit einem Hauptkörper anelliert sind, an den sie gebunden sind, um eine substituierte oder unsubstituierte cyclische oder eine substituierte oder unsubstituierte Arylgruppe zu bilden.

4. Lichtemittierendes Material nach Anspruch 1,
wobei in der chemischen Formel 2 R₁ und R₂ zusammen mit dem Stickstoff, an den sie angelagert sind, kombiniert sind, um ein substituiertes oder unsubstituiertes Heterocyclyl oder eine substituierte oder unsubstituierte Heteroarylgruppe zu bilden, die durch eine beliebige der Strukturformeln A1 bis A6 oder A9 bis A45 dargestellt sind: 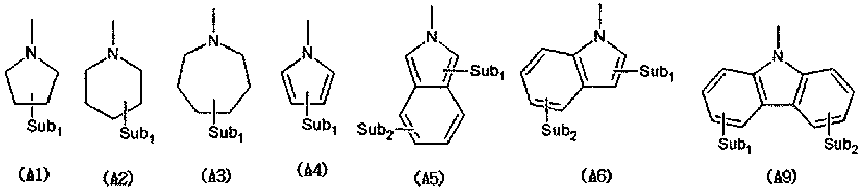 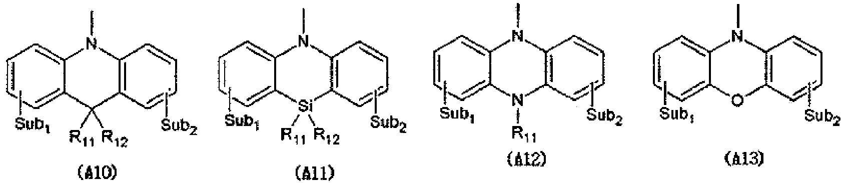 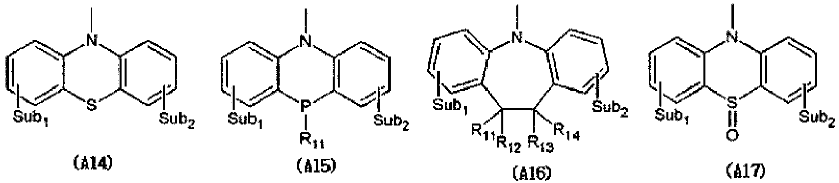 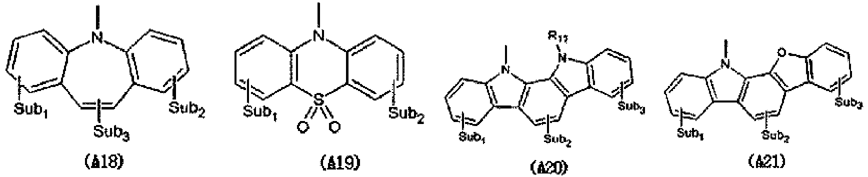 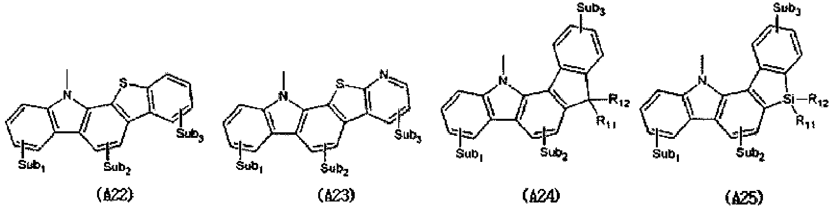 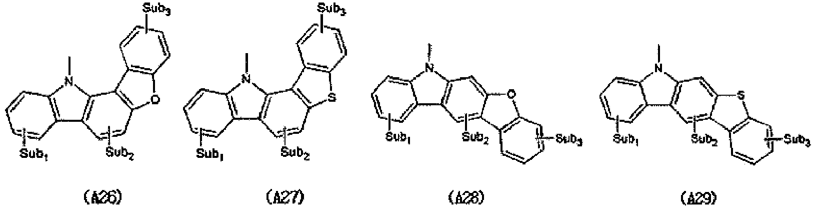 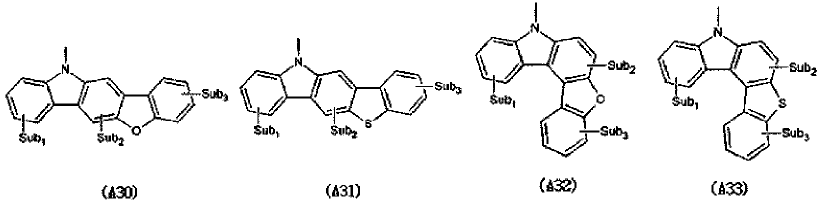 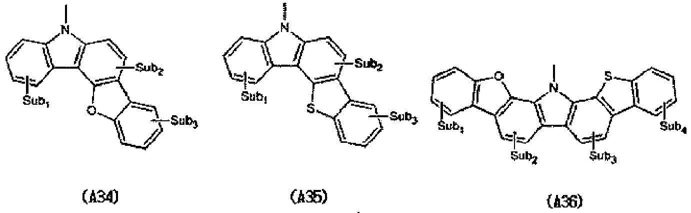 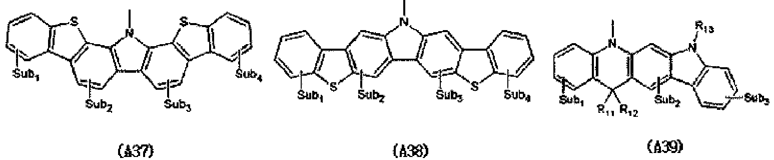 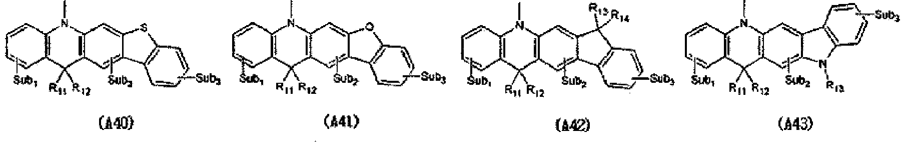 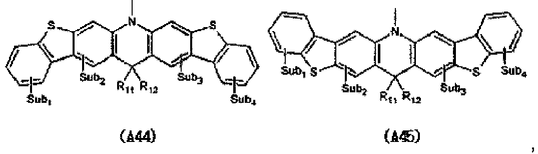
oder in der chemischen Formel 2 R₁ und R₂ so ausgewählt sind, dass sie durch eine beliebige der Strukturformeln A7 oder A8 dargestellt sind: 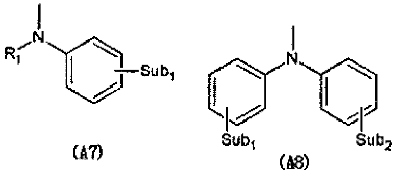
wobei in den Strukturformeln A1 bis A45
R₁₁ bis R₁₄ unabhängig voneinander Wasserstoff, Deuterium, eine substituierte oder unsubstituierte C1-C2-Alkylgruppe oder eine substituierte oder unsubstituierte C6-C30-Arylgruppe sind und
Sub, bis Sub₄ unabhängig voneinander Wasserstoff, Deuterium, eine Halogengruppe, eine Cyanogruppe, eine substituierte oder unsubstituierte C1-C9-Alkylgruppe, eine substituierte oder unsubstituierte C5-C30-Cycloalkylgruppe, eine substituierte oder unsubstituierte C6-C30-Arylgruppe, eine substituierte oder unsubstituierte C6-C30-Alkylarylgruppe, eine substituierte oder unsubstituierte C6-C30-Aralkylgruppe, eine substituierte oder unsubstituierte C3-C30-Heteroarylgruppe, eine substituierte oder unsubstituierte C1-C9-Alkyloxygruppe, eine substituierte oder unsubstituierte C6-C30-Aryloxygruppe, eine substituierte oder unsubstituierte Silylgruppe, eine substituierte oder unsubstituierte Phosphingruppe, eine substituierte oder unsubstituierte Phosphinoxidgruppe, eine substituierte oder unsubstituierte Thiolgruppe, eine substituierte oder unsubstituierte Sulfoxidgruppe oder eine substituierte oder unsubstituierte Sulfongruppe sind.

5. Lichtemittierendes Material nach Anspruch 4,
wobei in den Strukturformeln A1 bis A45 Sub₁ bis Sub₄ unabhängig voneinander Wasserstoff, Deuterium, eine Halogengruppe oder eine Cyanogruppe sind.

6. Lichtemittierendes Material nach mindestens einem der Ansprüche 1 bis 5, wobei die mit R₁₇ und R₁₈ substituierte Triazingruppe in der chemischen Formel 2 durch eine beliebige der Strukturformeln B8 bis B12, B50 und B51 dargestellt ist: 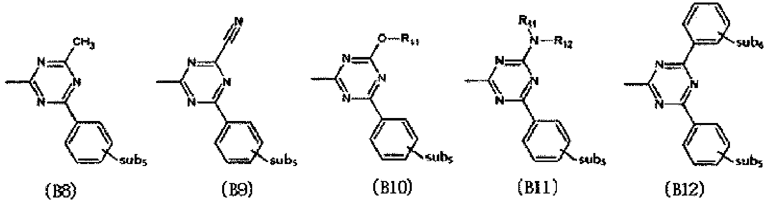 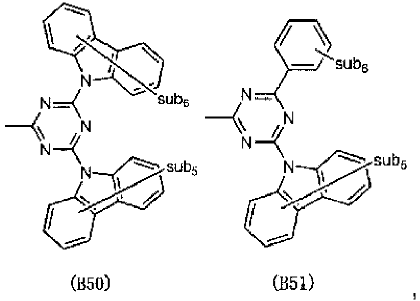
wobei in den Strukturformeln B8 bis B12, B50 und B51
R₁₁ und R₁₂ unabhängig voneinander Wasserstoff, Deuterium oder eine substituierte oder unsubstituierte C1-C2-Alkylgruppe sind,
Sub₅ und Sub₆ unabhängig voneinander Wasserstoff, Deuterium, eine Halogengruppe, eine Cyanogruppe, eine substituierte oder unsubstituierte C1-C9-Alkylgruppe, eine substituierte oder unsubstituierte C6-C30-Arylgruppe, eine substituierte oder unsubstituierte C3-C30-Heteroarylgruppe, eine substituierte oder unsubstituierte C1-C9-Alkyloxygruppe, eine substituierte oder unsubstituierte Silylgruppe, eine substituierte oder unsubstituierte Phosphingruppe, eine substituierte oder unsubstituierte Phosphinoxidgruppe, eine substituierte oder unsubstituierte Thiolgruppe, eine substituierte oder unsubstituierte Sulfoxidgruppe, eine substituierte oder unsubstituierte Sulfongruppe, eine substituierte oder unsubstituierte C5-C30-Arylthiogruppe, eine substituierte oder unsubstituierte C5-C30-Aryloxygruppe, eine substituierte oder unsubstituierte C5-C30-Arylamingruppe oder eine substituierte oder unsubstituierte C5-C30-Aralkylgruppe sind; und Sub₅ und Sub₆ unabhängig voneinander optional mit einem Hauptkörper anelliert sind, an den sie gebunden sind, um ein substituiertes oder unsubstituiertes cyclisches oder ein substituiertes oder unsubstituiertes Aryl zu bilden, und
wobei in der Strukturformel B50 Sub₅ eine Cyanogruppe ist, wodurch Sub₅ unabhängig voneinander auch in mehrfacher Anzahl vorliegen kann, sodass andere Sub₅ wie vorstehend definiert sein können.

7. Lichtemittierendes Material nach mindestens einem der Ansprüche 1 bis 5, wobei die mit R₁₇ und R₁₈ substituierte Triazingruppe in der chemischen Formel 2 durch eine beliebige der Strukturformeln B12 und B50 bis B54 dargestellt ist: 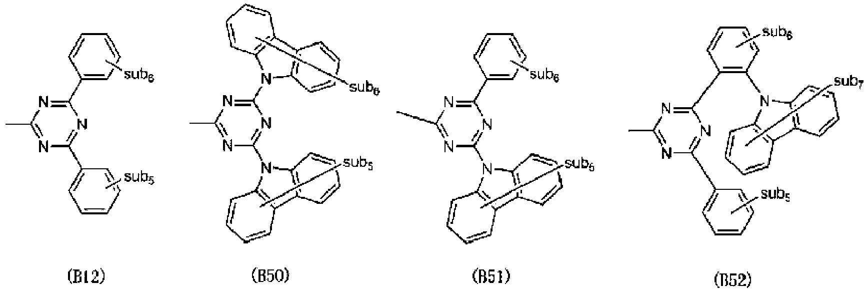 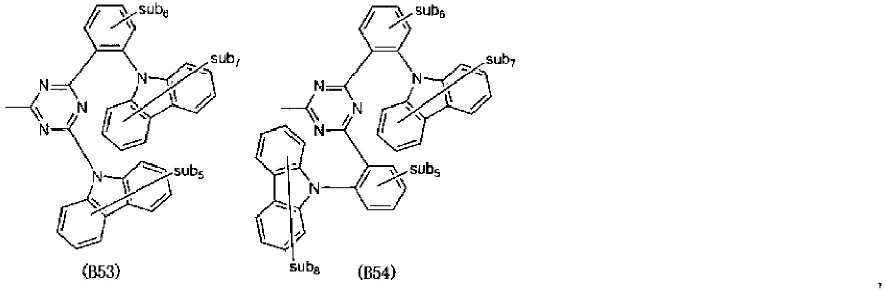
wobei in den Strukturformeln B12 und B50 bis B54
Sub₅ bis Sub₈ unabhängig voneinander Wasserstoff, Deuterium, eine Halogengruppe, eine Cyanogruppe, eine substituierte oder unsubstituierte C6-C30-Arylgruppe oder eine substituierte oder unsubstituierte C3-C30-Heteroarylgruppe sind und
wobei in der Strukturformel B50 Sub₅ eine Cyanogruppe ist, wodurch Sub₅ unabhängig voneinander auch in mehrfacher Anzahl vorliegen kann, sodass andere Sub₅ wie vorstehend definiert sein können.

8. Lichtemittierendes Material nach mindestens einem der Ansprüche 1 bis 7, wobei in der chemischen Formel 2 R₂₂ bis R₂₅ unabhängig voneinander Wasserstoff oder Deuterium sind und
wobei, wenn die mit R₁₇ und R₁₈ substituierte Triazingruppe in der chemischen Formel 2 durch die Strukturformel B51 dargestellt ist, R₂₂ bis R₂₅ unabhängig voneinander Deuterium sind.

9. Lichtemittierendes Material nach Anspruch 1,
wobei das durch die chemische Formel 2 dargestellte lichtemittierende Material durch eine beliebige der Verbindungen 100, 101, 104 bis 106, 108, 109, 112, 113 und 120 bis 122 dargestellt ist:

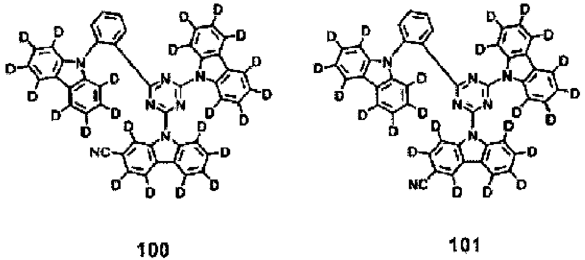

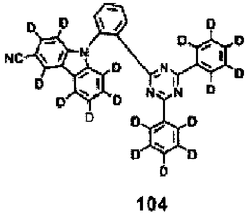

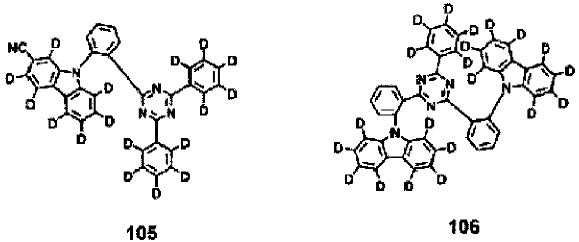

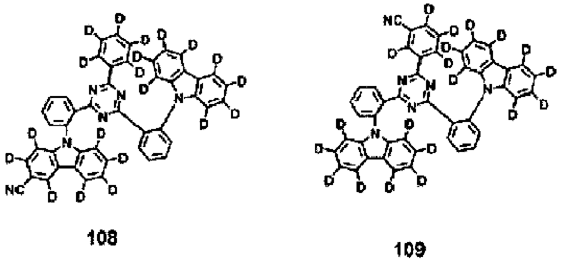

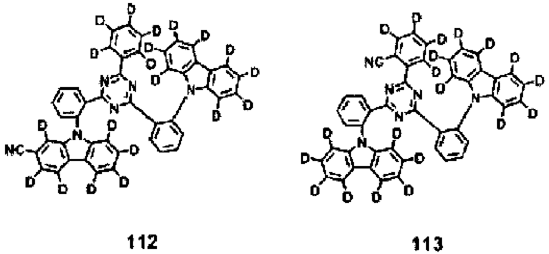

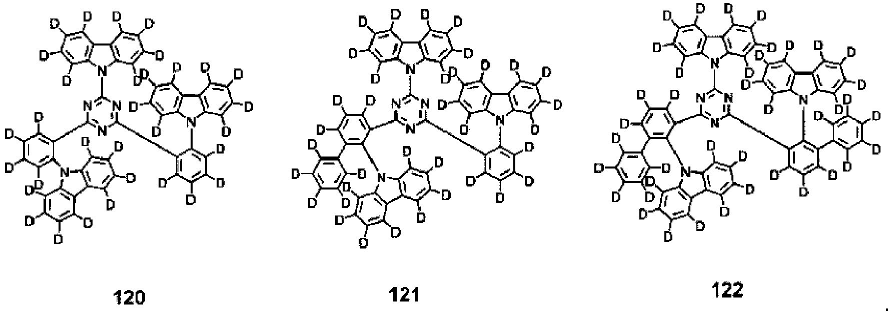

10. Lichtemittierende Vorrichtung (10), umfassend:
eine erste Elektrode (110);
eine zweite Elektrode (150), die der ersten Elektrode (110) zugewandt ist;
eine Zwischenschicht (130), die zwischen der ersten Elektrode (110) und der zweiten Elektrode (150) angeordnet ist und eine Emissionsschicht umfasst; und ein lichtemittierendes Material gemäß mindestens einem der Ansprüche 1 bis 9.

11. Lichtemittierende Vorrichtung (10) nach Anspruch 10, wobei die Emissionsschicht das durch die Formel 2 dargestellte lichtemittierende Material umfasst.

12. Lichtemittierende Vorrichtung (10) nach Anspruch 10, wobei die Emissionsschicht einen Wirt und einen Dotierstoff umfasst und
der Wirt das durch die Formel 2 dargestellte lichtemittierende Material umfasst.

13. Lichtemittierende Vorrichtung (10) nach Anspruch 12,
wobei der Wirt ein verzögert fluoreszierendes lichtemittierendes Material ist.

14. Lichtemittierende Vorrichtung (10) nach Anspruch 10, ferner umfassend:
eine erste Deckschicht, die außerhalb der ersten Elektrode (110) angeordnet ist; und
eine zweite Deckschicht, die außerhalb der zweiten Elektrode (150) angeordnet ist,
wobei die erste Deckschicht oder die zweite Deckschicht das durch die Formel 2 dargestellte lichtemittierende Material umfasst.

15. Elektronische Einrichtung oder Anlage, umfassend die lichtemittierende Vorrichtung (10) nach mindestens einem der Ansprüche 10 bis 14.

## Revendications

1. Matériau électroluminescent représenté par la formule chimique 2 :
Formule chimique 2 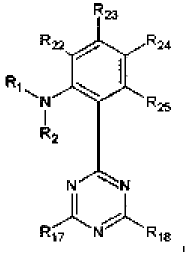
dans lequel, dans la formule chimique 2,
R₁ et R₂ sont, indépendamment l'un de l'autre, un groupe alkyle en C1 à C9 substitué ou non substitué, un groupe cycloalkyle en C5 à C30 substitué ou non substitué, un groupe aryle en C6 à C30 substitué ou non substitué, un groupe alkylaryle en C6 à C30 substitué ou non substitué, un groupe aralkyle en C6 à C30 substitué ou non substitué, ou un groupe hétéroaryle en C3 à C30 substitué ou non substitué ; et R₁ et R₂ sont facultativement combinés conjointement avec l'azote auquel ils sont attachés pour former un groupe hétérocyclyle substitué ou non substitué ou un groupe hétéroaryle substitué ou non substitué,
R₂₂ à R₂₅ sont, indépendamment les uns des autres, hydrogène, deutérium, un groupe halogéné, ou un groupe aryle en C4 à C6 substitué ou non substitué dans lequel le groupe triazine substitué par R₁₇ et R₁₈ dans la formule chimique 2 est représenté par l'une quelconque des formules développées B1 à B13, B50 et B51 : 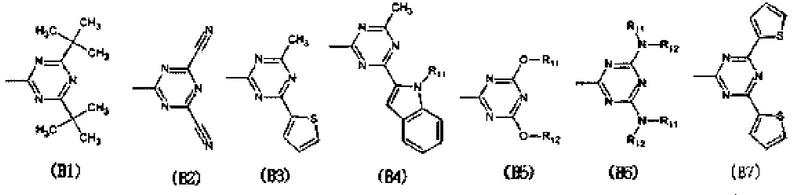 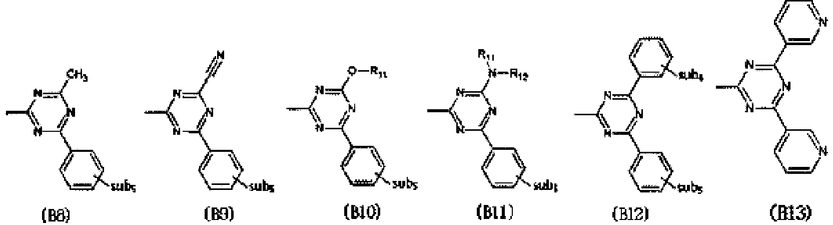 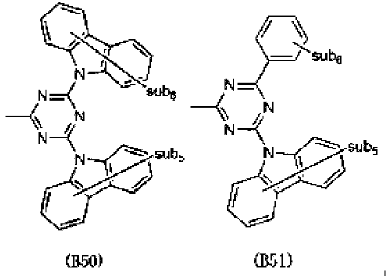
dans lequel, dans les formules développées B1 à B13, B50 et B51,
R₁₁ et R₁₂ sont, indépendamment l'un de l'autre, hydrogène, deutérium ou un groupe alkyle en C1 à C2 substitué ou non substitué,
Sub₅ et Sub₆ sont, indépendamment l'un de l'autre, hydrogène, deutérium, un groupe halogéné, un groupe cyano, un groupe alkyle en C1 à C9 substitué ou non substitué, un groupe aryle en C6 à C30 substitué ou non substitué, un groupe hétéroaryle en C3 à C30 substitué ou non substitué, un groupe alkyloxy en C1 à C9 substitué ou non substitué, un groupe -NRₑR_{f} substitué ou non substitué, un groupe silyle substitué ou non substitué, un groupe phosphine substitué ou non substitué, un groupe oxyde de phosphine substitué ou non substitué, un groupe thiol substitué ou non substitué, un groupe sulfoxyde substitué ou non substitué, un groupe sulfone substitué ou non substitué, un groupe arylthio en C5 à C30 substitué ou non substitué, un groupe aryloxy en C5 à C30 substitué ou non substitué, un groupe arylamine en C5 à C30 substitué ou non substitué, ou un groupe aralkyle en C5 à C30 substitué ou non substitué, et Sub₅ et Sub₆ sont, indépendamment l'un de l'autre, facultativement fusionnés à un corps principal auquel ils sont liés pour former un cyclique substitué ou non substitué ou un aryle substitué ou non substitué, et
Rₑ et R_{f} sont chacun choisis indépendamment dans le groupe consistant en un groupe alkyle en C1 à C9 substitué ou non substitué, un groupe cycloalkyle en C5 à C30 substitué ou non substitué, un groupe aryle en C6 à C30 substitué ou non substitué, un groupe alkylaryle en C6 à C30 substitué ou non substitué, un groupe aralkyle en C6 à C30 substitué ou non substitué, et un groupe hétéroaryle en C3 à C30 substitué ou non substitué, et Rₑ et R_{f} sont facultativement combinés conjointement avec l'azote auquel ils sont attachés pour former un hétérocyclyle substitué ou non substitué ou un hétéroaryle substitué ou non substitué,
dans lequel, dans la formule développée B50, Sub₅ est un groupe cyano, moyennant quoi Sub₅ peut également exister, indépendamment les uns des autres, en nombres multiples, de sorte que d'autres Sub₅ peuvent être tels que définis ci-dessus,
dans lequel, dans la formule développée B51, R₂₂ à R₂₅ sont, indépendamment les uns des autres, deutérium, un groupe halogéné, ou un groupe aryle en C4 à C6 substitué ou non substitué, et
dans lequel au moins un élément choisi parmi R₁, R₂, R₁₇ et R₁₈ comprend un deutérium, ou au moins un élément choisi parmi R₂₂ à R₂₅ est un deutérium.

2. Matériau électroluminescent selon la revendication 1,
dans lequel, dans la formule chimique 2, R₁ et R₂ sont combinés conjointement avec l'azote auquel ils sont attachés pour former un groupe hétérocyclyle substitué ou non substitué ou un groupe hétéroaryle substitué ou non substitué.

3. Matériau électroluminescent selon la revendication 1,
dans lequel, dans la formule chimique 2, R₁ et R₂ sont combinés conjointement avec l'azote auquel ils sont attachés pour former un groupe hétérocyclyle substitué ou non substitué ou un groupe hétéroaryle substitué ou non substitué représenté par l'une quelconque des formules développées A1 à A6 et A9 à A19 : 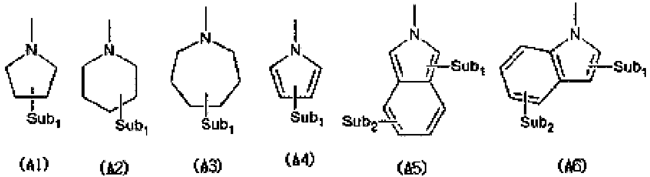 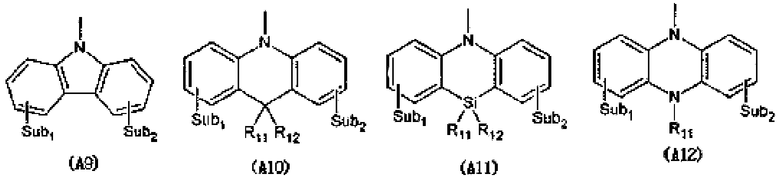 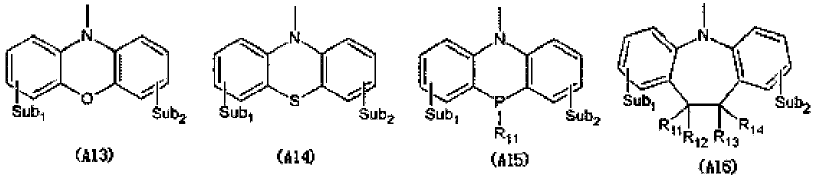 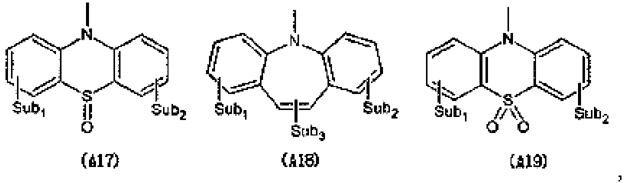
dans lequel, dans les formules développées A1 à A6 et A9 à A19,
R₁₁ à R₁₄ sont, indépendamment les uns des autres, hydrogène, deutérium, un groupe alkyle en C1 à C2 substitué ou non substitué, ou un groupe aryle en C6 à C30 substitué ou non substitué,
Sub₁ et Sub₂ sont, indépendamment l'un de l'autre, hydrogène, deutérium, un groupe halogéné, un groupe cyano, un groupe alkyle en C1 à C9 substitué ou non substitué, un groupe cycloalkyle en C5 à C30 substitué ou non substitué, un groupe aryle en C6 à C30 substitué ou non substitué, un groupe alkylaryle en C6 à C30 substitué ou non substitué, un groupe hétéroaryle en C3 à C30 substitué ou non substitué, un groupe alkyloxy en C1 à C9 substitué ou non substitué, un groupe -NR_{c}R_{d} substitué ou non substitué, un groupe silyle substitué ou non substitué, un groupe phosphine substitué ou non substitué, un groupe oxyde de phosphine substitué ou non substitué, un groupe thiol substitué ou non substitué, un groupe sulfoxyde substitué ou non substitué, un groupe sulfone substitué ou non substitué, un groupe arylsilyle en C5 à C30 substitué ou non substitué, un groupe arylthio en C5 à C30 substitué ou non substitué, un groupe aryloxy en C5 à C30 substitué ou non substitué, un groupe arylamine en C5 à C30 substitué ou non substitué, ou un groupe aralkyle en C5 à C30 substitué ou non substitué,
R_{c} et R_{d} sont, indépendamment l'un de l'autre, choisis dans le groupe consistant en un groupe alkyle en C1 à C9 substitué ou non substitué, un groupe cycloalkyle en C5 à C30 substitué ou non substitué, un groupe aryle en C6 à C30 substitué ou non substitué, un groupe aralkyle en C6 à C30 substitué ou non substitué et un groupe hétéroaryle en C3 à C30 substitué ou non substitué, et R_{c} et R_{d} sont facultativement combinés conjointement avec l'azote auquel ils sont attachés pour former un groupe hétérocyclyle substitué ou non substitué ou un groupe hétéroaryle substitué ou non substitué, et
Sub₁ et Sub₂ sont, indépendamment l'un de l'autre, facultativement fusionnés à un corps principal auquel ils sont liés pour former un cyclique substitué ou non substitué ou un groupe aryle substitué ou non substitué.

4. Matériau électroluminescent selon la revendication 1,
dans lequel, dans la formule chimique 2, R₁ et R₂ sont combinés conjointement avec l'azote auquel ils sont attachés pour former un groupe hétérocyclyle substitué ou non substitué ou un groupe hétéroaryle substitué ou non substitué représenté par l'une quelconque des formules développées A1 à A6 ou A9 à A45 : 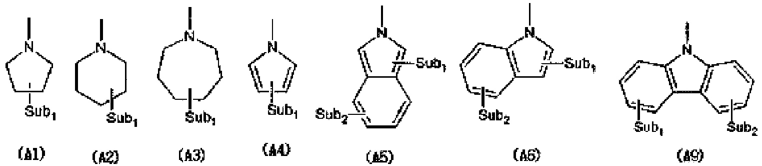 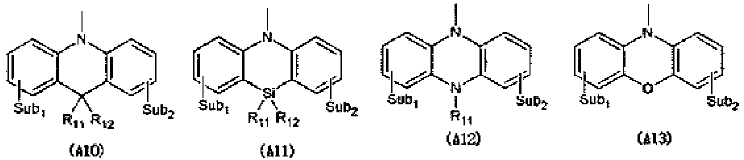 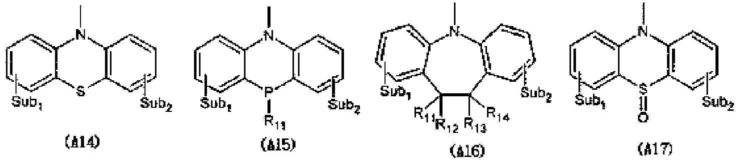 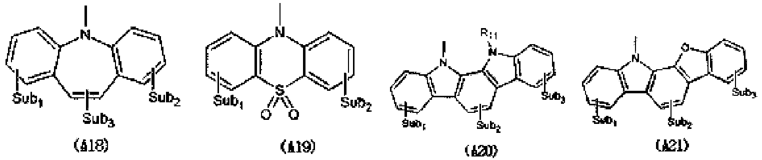 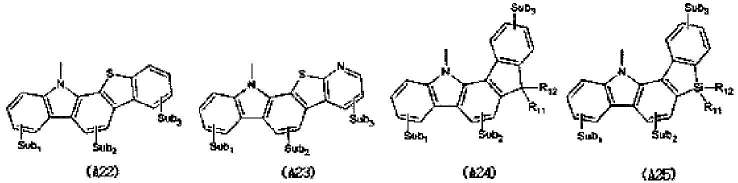 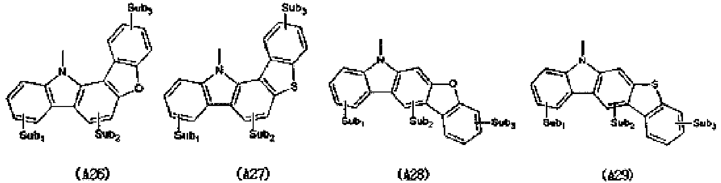 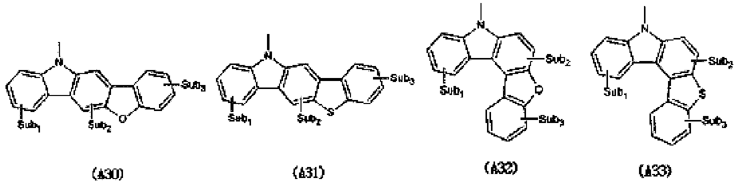 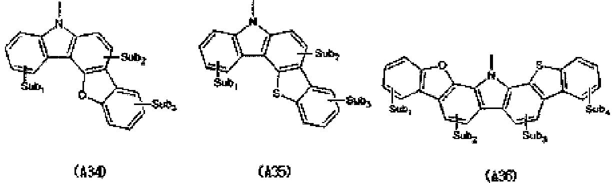 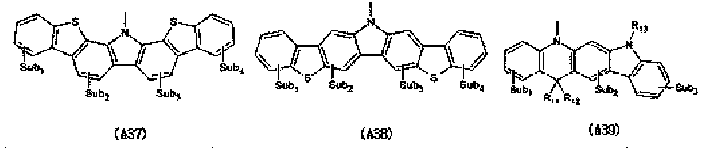 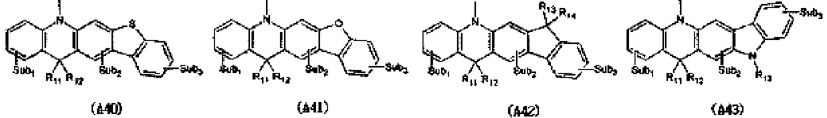 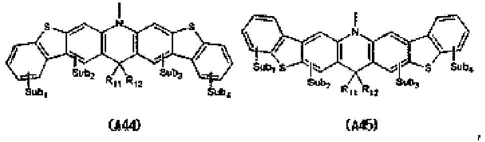
ou, dans la formule chimique 2, R₁ et R₂ sont choisis pour être représentés par l'une quelconque des formules développées A7 ou A8 : 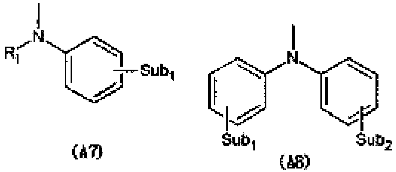
dans lequel, dans les formules développées A1 à A45,
R₁₁ à R₁₄ sont, indépendamment les uns des autres, hydrogène, deutérium, un groupe alkyle en C1 à C2 substitué ou non substitué, ou un groupe aryle en C6 à C30 substitué ou non substitué, et
Sub₁ à Sub₄ sont, indépendamment les uns des autres, hydrogène, deutérium, un groupe halogéné, un groupe cyano, un groupe alkyle en C1 à C9 substitué ou non substitué, un groupe cycloalkyle en C5 à C30 substitué ou non substitué, un groupe aryle en C6 à C30 substitué ou non substitué, un groupe alkylaryle en C6 à C30 substitué ou non substitué, un groupe aralkyle en C6 à C30 substitué ou non substitué, un groupe hétéroaryle en C3 à C30 substitué ou non substitué, un groupe alkyloxy en C1 à C9 substitué ou non substitué, un groupe aryloxy en C6 à C30 substitué ou non substitué, un groupe silyle substitué ou non substitué, un groupe phosphine substitué ou non substitué, un groupe oxyde de phosphine substitué ou non substitué, un groupe thiol substitué ou non substitué, un groupe sulfoxyde substitué ou non substitué, ou un groupe sulfone substitué ou non substitué.

5. Matériau électroluminescent selon la revendication 4,
dans lequel, dans les formules développées A1 à A45, Sub₁ à Sub₄ sont, indépendamment les uns des autres, hydrogène, deutérium, un groupe halogéné ou un groupe cyano.

6. Matériau électroluminescent selon au moins l'une des revendications 1 à 5, dans lequel le groupe triazine substitué par R₁₇ et R₁₈ dans la formule chimique 2 est représenté par l'une quelconque des formules développées B8 à B12, B50 et B51 : 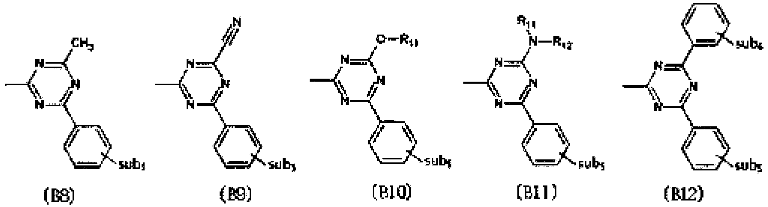 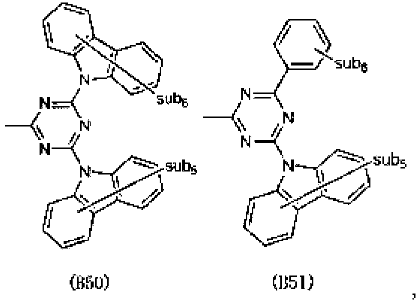
dans lequel, dans les formules développées B8 à B12, B50 et B51,
R₁₁ et R₁₂ sont, indépendamment l'un de l'autre, hydrogène, deutérium ou un groupe alkyle en C1 à C2 substitué ou non substitué,
Sub₅ et Sub₆ sont, indépendamment l'un de l'autre, hydrogène, deutérium, un groupe halogéné, un groupe cyano, un groupe alkyle en C1 à C9 substitué ou non substitué, un groupe aryle en C6 à C30 substitué ou non substitué, un groupe hétéroaryle en C3 à C30 substitué ou non substitué, un groupe alkyloxy en C1 à C9 substitué ou non substitué, un groupe silyle substitué ou non substitué, un groupe phosphine substitué ou non substitué, un groupe oxyde de phosphine substitué ou non substitué, un groupe thiol substitué ou non substitué, un groupe sulfoxyde substitué ou non substitué, un groupe sulfone substitué ou non substitué, un groupe arylthio en C5 à C30 substitué ou non substitué, un groupe aryloxy en C5 à C30 substitué ou non substitué, un groupe arylamine en C5 à C30 substitué ou non substitué, ou un groupe aralkyle en C5 à C30 substitué ou non substitué ; et Sub₅ et Sub₆ sont, indépendamment l'un de l'autre, facultativement fusionnés à un corps principal auquel ils sont liés pour former un cyclique substitué ou non substitué ou un aryle substitué ou non substitué, et
dans lequel, dans la formule développée B50, Sub₅ est un groupe cyano, moyennant quoi Sub₅ peut également exister, indépendamment les uns des autres, en nombres multiples, de sorte que les autres Sub₅ peuvent être tels que définis ci-dessus.

7. Matériau électroluminescent selon au moins l'une des revendications 1 à 5, dans lequel le groupe triazine substitué par R₁₇ et R₁₈ dans la formule chimique 2 est représenté par l'une quelconque des formules développées B12 et B50 à B54 : 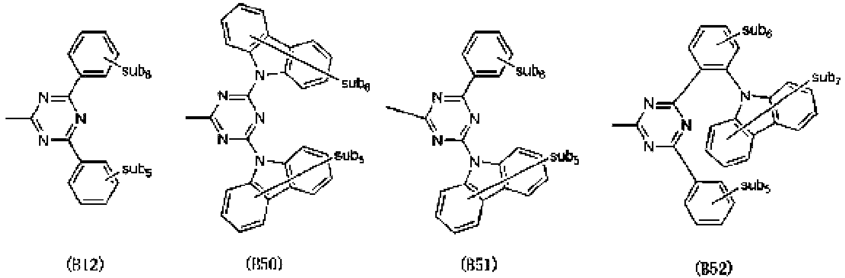 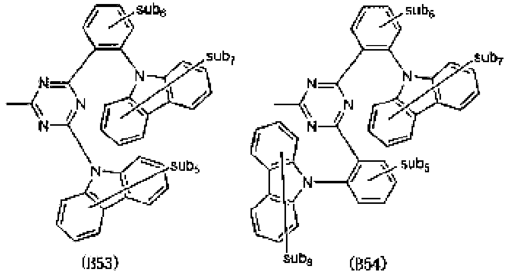
dans lequel, dans les formules développées B12 et B50 à B54,
Sub₅ à Sub₈ sont, indépendamment les uns des autres, hydrogène, deutérium, un groupe halogéné, un groupe cyano, un groupe aryle en C6 à C30 substitué ou non substitué, ou un groupe hétéroaryle en C3 à C30 substitué ou non substitué, et
dans lequel, dans la formule développée B50, Sub₅ est un groupe cyano, moyennant quoi Sub₅ peut également exister, indépendamment les uns des autres, en nombres multiples, de sorte que les autres Sub₅ peuvent être tels que définis ci-dessus.

8. Matériau électroluminescent selon au moins l'une des revendications 1 à 7,
dans lequel, dans la formule chimique 2, R₂₂ à R₂₅ sont, indépendamment les uns des autres, hydrogène ou deutérium, et
dans lequel, lorsque le groupe triazine substitué par R₁₇ et R₁₈ dans la formule chimique 2 est représenté par la formule développée B51, R₂₂ à R₂₅ sont, indépendamment les uns des autres, deutérium.

9. Matériau électroluminescent selon la revendication 1,
dans lequel le matériau électroluminescent représenté par la formule chimique 2 est représenté par l'un quelconque des composés 100, 101, 104 à 106, 108, 109, 112, 113 et 120 à 122 :

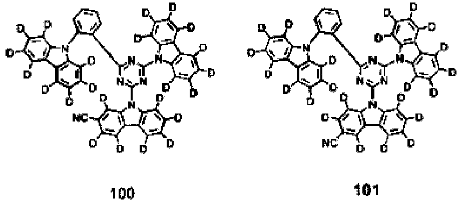

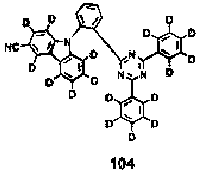

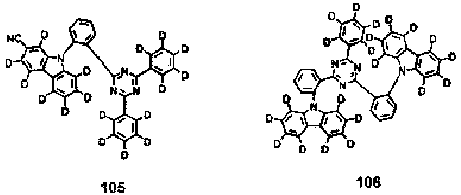

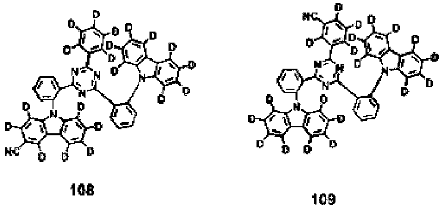

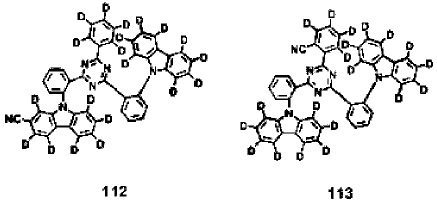

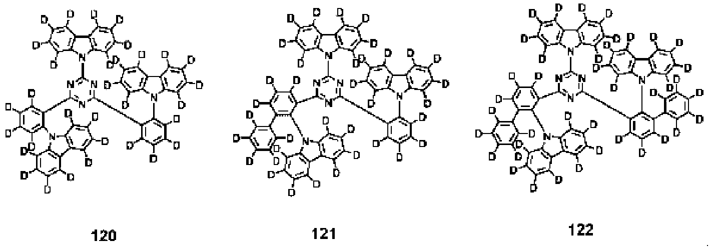

10. Dispositif électroluminescent (10) comprenant :
une première électrode (110) ;
une deuxième électrode (150) faisant face à la première électrode (110) ;
une couche intermédiaire (130) agencée entre la première électrode (110) et la deuxième électrode (150) et comprenant une couche d'émission ; et
un matériau électroluminescent selon au moins l'une des revendications 1 à 9.

11. Dispositif électroluminescent (10) selon la revendication 10, dans lequel la couche d'émission comprend le matériau électroluminescent représenté par la formule 2.

12. Dispositif électroluminescent (10) selon la revendication 10, dans lequel la couche d'émission comprend un hôte et un dopant, et
l'hôte comprend le matériau électroluminescent représenté par la formule 2.

13. Dispositif électroluminescent (10) selon la revendication 12,
dans lequel l'hôte est un matériau électroluminescent à fluorescence retardée.

14. Dispositif électroluminescent (10) selon la revendication 10, comprenant en outre :
une première couche de recouvrement agencée à l'extérieur de la première électrode (110) ; et
une deuxième couche de recouvrement agencée à l'extérieur de la deuxième électrode (150),
dans lequel la première couche de recouvrement ou la deuxième couche de recouvrement comprend le matériau électroluminescent représenté par la formule 2.

15. Appareil ou équipement électronique comprenant le dispositif électroluminescent (10) d'au moins l'une des revendications 10 à 14.
